# EUROPEAN PATENT SPECIFICATION

(11) **EP 1 303 628 B1**
(45) Date of publication and mention of the grant of the patent: **04.10.2006**
(21) Application number: 01960778.7
(22) Date of filing: 16.07.2001
(51) Int. Cl.: C12N 15/82, C12N 15/79

(54) **MOLECULAR CONTROL OF TRANSGENE SEGREGATION AND ITS ESCAPE BY A RECOVERABLE BLOCK OF FUNCTION (RBF) SYSTEM**
MOLEKULARE KONTROLLE DER TRANSGENEN SEGREGATION UND DEREN AUSBRUCH
CONTROLE MOLECULAIRE D'UNE SEGREGATION TRANSGENIQUE ET SON EVACUATION PAR UN SYSTEME DE BLOC DE FONCTION RECUPERABLE (RBF)

(30) Priority: 14.07.2000 US 617543
(43) Date of publication of application: 23.04.2003
(73) Proprietor: Unicrop Ltd., 00790 Helsinki (FI)
(72) Inventor: KUVSHINOV, Viktor, FIN-01280 Vantaa (FI); KOIVU, Kimmo, FIN-00550 Helsinki (FI); KANERVA, Anne, FIN-00550 Helsinki (FI); PEHU, Eija, Washington, DC 20016 (US)
(74) Representative: Lönnqvist, Gunnel Solveig Kristina
(86) International application number: PCT/FI2001/000670
(87) International publication number: WO 2002/006498

(56) References cited:
- WO-A1-00/37660
- WO-A1-97/30162
- WO-A2-94/03619
- US-A- 5 723 765
- VIKTOR KUVSHINOV ET AL.: 'Molecular control of transgene escape from genetically modified plants' PLANT SCIENCE vol. 160, 2001, pages 517 - 522, XP002958661
- HARUE AKASOFU ET AL.: 'Nucleotide sequence of the gene for the vigna mungo sulfhydryl-endopeptidase (SH-EP)' NUCLEIC ACIDS RESEACH vol. 18, no. 7, March 1990, page 1892, XP002958670

## Description

### The Technical Field of the Invention

The present invention is related to an improved method and complex of DNA constructs, herein called Recoverable Block of Function (RBF) systems, for obtaining an increased security level in the control of transgene segregation and transgene escape including introgression. The method and system also allows farmers to reuse their transgenic crop without risking leakage of transgenes into the environment. The increased security level is achieved by Recoverable Block of Function (RBF) systems comprising one or more blocking nucleotide constructs (BC) closely linked to a Transgene of Interest (TGI), wherein the Blocking Construct (BC) is inserted into an intron of the Transgene of Interest (TGI), if one Blocking Construct (BC) is used or between two Blocking Constructs (BC), if more than one are used, as well as one or more Recovering Constructs (RC), i.e. nucleotide constructs for recovering the blocked function under a user-controlled chemical, physical or mechanical intervention.

The present invention not only relates to the method but also to Recoverable Block of Function (RBF) systems as well as their use in the production of vectors, cells, cell-lines and/or transgenic Sexually Reproducing plant, especially plants and certain animals, such as fish, shrimps, molluscs, etc.

### The Background of the Invention

Over the past few years the safety of transgenic crop production has been the cause of great concern among the public at large and it has attracted a lot of attention in the scientific community as well. In several publications pollen from transgenic plants has been reported to have spread from the field plots. Most problematic are transgenic crop species, which have wild relatives with which they can hybridize in nature if they succeed to escape, but it is also important to prevent and control the crossing between different but related transgenic or non-transgenic crop plants or related wild-types, i.e. to keep the purity of germlines. Risk groups are found among certain crop species, for example corn, certain oil producing plants such as *Brassicae,* but also among trees, which are a specific problem due to their long life span and great production of pollen. Even if the problem at present is mainly restricted to plants, it is likely that similar problems will be encountered when animals are becoming more serious targets in the transgenic production methods. Fish, shrimps, poultry, sheep, etc. are already feasible as transgenic cattle. It is also likely that a wide range of modifications will be applied to the transgenic plants in the future, thus, intensifying the need to prevent transgene escape.

While the risks to human or animal health of a particular transgene and its product can be tested and measured, the impact of gene escape is more complex to assess. On the other hand, the potentials of improving crops by recombinant DNA techniques are so tremendous, that rather than banning the use of transgenesis, it is more productive and cost-effective to find solutions to prevent gene leakage or escape.

Due to the great impact of transgenesis and the worries raised concerning possible transgenic escape, several approaches to tackle the problem have been presented in prior art. The first approaches comprised plants with engineered sterility and the control of seed germination, also referred to as the "terminator technology" launched by Monsanto (US 5,723,765). Several reports on the application of conditional lethal factors, also called suicidal genes have been published. Mitigation genes, i.e. genes weakening selective advantages of transgenes in weeds have been suggested for use in the control of transgene escape (Gressel, J., (1999), Tibtech. 17: 361-366).

The use of engineered male or female sterility has been studied by several groups. In the male sterility (MS) method pollination is prevented, for example, by arresting mRNA synthesis. Pollination can be restored by expression of an RNase inhibitor. In said case the recovering factor (RF) is carried by the pollinating line. The two elements, MS and RF, are present in two different individual plants. Only a hybrid of these two plant individuals carries both the blocking and the recovering factors and thereby it is fertile. Consequently, the male sterility technique aims at supporting hybrid seed production. However, male sterility does not prevent escape of the transgene into the environment, because the pollinated female (MS) plants are still capable of producing hybrid seeds, which if shattering may, stay behind in the field after harvesting.

Engineered fertility control has also been achieved in transgenic *Brassica napus* by expression of the RNase gene under an anther tapetum specific tobacco promoter (De Block and Debrouwer, (1993), Planta, 189: 218-225). Expression of the RNase gene in the tapetum cells of the anther kills the pollen at early stages of development. Pollination of the male-sterile flowers with pollen of transgenic *B. napus* plants expressing the RNase gene under the same promoter recovers the male sterility (MS) trait and the hybrid plants can produce normal fertile pollen with expression of the two genes. The patent US 5,750,867 discloses the maintenance of male-sterile plants. The patent US 5,767,374 discloses a similar method for female-sterile (FM) plants in which method the ribonuclease gene is expressed in stamen cells of female parental plants and the killer gene expression in hybrid plants is restored by expression of the restorer gene coming from the pollinating parent line. The blocking gene is expressed in the female organs of the parent plant, while pollen remains fertile. The female-sterile (FM) plants are intended to be pollinated by male sterile (MS) plants for production of hybrid seeds.

The patent US 5,728,92 discloses a method based on the expression of antisense mRNA of a gene vital for anther development. The antisense molecule is expressed at the same time with a sense molecule, both of which are driven by the same promoter. Concurrent expression of the sense and antisense orientations of the gene provides a silencing mechanism, which prevents anther development. The patent US 6,013,859 discloses a method of hybridization. The blocking is provided by two consequent enzymatic reactions in the anthers or microspores. The pollinating parental line confers resistance to a selective marker (herbicide). The patent US 6,005,167 in turn describes a method, which applies antisense technique for providing male or female sterility based on blocking of the chalcone synthase expression in a developing anther or another part of the flower. Chalcone synthase is a key-enzyme in the flavonoid biosynthesis. Blocking of the expression of this gene leads to an unrecoverable blocking of fertilization.

The patent US 5,723,765 describes a method for arresting seed germination or function. The technique comprises activation of the function of the inhibited blocking gene through excision of a specific DNA sequence between the promoter and the blocking or terminator gene by specific Cre recombinase enzyme encoded by another gene placed under a Tet-repressed promoter. Seeds of transgenic plants not treated with tetracycline are capable of germinating under natural conditions. If the transgenic seeds are treated with tetracycline, the gene encoding for Cre recombinase activates and excises the DNA insertion between a Lea promoter and a toxin gene. Thereby, the block is removed and the toxin activated. The toxin does not kill the plant immediately because the expression will be initiated only during late embryogenesis with the action of the Lea promoter.

The idea disclosed in US 5,723,765 implies the inhibition of the development of seeds in the second generation. Without the mechanism of suppression, the 'killer' gene is activated during late stages of embryo development of the progeny and therefore the seeds of the next generation do not germinate. The fundamental problem with said technique is that once the plants have been tetracycline treated, i.e. the killer gene has been activated, they cannot be rescued. Furthermore, if said transgenic plant carrying the tetracycline recoverable construct escapes to the environment, it is capable of germinating, growing to maturity, flowering and reproducing sexually. In other words, the transgene escape from transgenic plants is not prevented. Said so called "terminator technology" has encountered negative public attention because it gives the seed producing companies the possibility to control the market of transgenic seed production.

The International Patent Application WO 94/03619 discloses the control of transgenic plant growth by a chemical activation or a "gene switch". The switched gene produces a repressor, which can inactivate the expression of a "disrupter gene". When a chemical induction of the repressor gene is applied, the disrupter gene is repressed. If the repressor gene is not activated, the disrupter gene destroys the cell and the functions of the plant. The repression mechanism may comprise a repressor protein, including a promoter and operator system. Alternatively, the repressor can be present as a gene encoding an inhibitor of the gene encoding the disrupter protein. The "chemical switch" described in WO 94/03619 can also be used to control the activity of a recombinase gene, which can remove DNA sequence flanked by recombinase recognition sites. The recombinase enzyme removes a part of the trait gene and blocks the function of the value-added trait.

The International Patent Application WO 00/37660 discloses methods and genetic compositions for limiting out-crossing and undesired gene flow in crop plants. WO 00/37660 discloses a genetic system comprising two DNA constructs, a repressible dominant lethal gene and repressor gene, which is located at a locus that segregates independently from the repressible dominant lethal gene. A complicate cloning and selection system for designing plants containing a repressible lethal gene and a repressor gene in different sister chromosomes is also described.

The patent US 5,498,533 discloses the regulation of potato development by expression of sense and antisense constructs of the calmodulin gene. Expression of sense-oriented calmodulin gene increases shoot and tuber growth, whereas plants carrying antisense constructs exhibit decreased shoot and tuber growth, while the expression of antisense calmodulin gene can be used as a factor blocking a physiological function.

Gressel (1999) discloses in his review (Tibtech, 17: 361-366) several applications of current and hypothetical systems based on tandem transgenetic-mitigation (TM) technology which can be used to prevent the rise of superweeds. The TM genes are positive or neutral to the crop but deleterious to the weed. The TM genes can change the morphology or physiology of the weed by providing seed dormancy, early or late seed ripening, plant dwarfing as well as other traits, which weaken the evolutionary fitness of voluntary weeds as compared to the parental strain. However, the mitigation genes can not entirely prevent transgene escape, because they are neither lethal nor even adverse for crop plants. Accordingly, transgenetic mitigation technology does not prevent the crossing between different lines of the same crops.

The effect of mitigation genes is generally achieved only after prolonged negative selection periods during which time possible silencing or mutations in the mitigating genes reduce their effect and consequently act against achieving the prevention of transgene escape. In his review article Gressel (1999) suggests the use of tandem constructs comprising two mitigation genes to decrease the possibility of the transgene escape. Tandems of mitigation genes do not solve the problem of crossing between different lines of related crop plants. Silencing or mutations in one mitigation gene reduces the negative selection pressure in the action.

Most of the systems intended for providing control of escape of transgenic plants have been described in prior art are unrecoverable. In other words, they cannot be reused once the recovery system has been applied. A further limitation in some of the above described systems is that they require vegetative propagation, because fertilization or embryo development is arrested.

Accordingly, the control situation is far from ideal in spite of the multitude of different approaches suggested in prior art. Alternative and more effective methods and systems to combat the problem are needed. When starting from the use of mitigating genes, it can be concluded that sufficient security is not achieved with mitigation genes. Lethal or sterility causing genes are required. Prior art systems applying lethal or sterility causing genes have been described and used, but sufficient security levels cannot be ensured, especially when longer selection times are required. A question not answered by prior art methods and systems, is for example the following: What happens in the frequently occurring cases when lethal, disrupter or blocking genes are inactivated? Lethal or sterility causing genes can be expected to be silenced or mutated with a frequency of about 10⁻⁶, which allows a small but acknowledgeable escape of transgenes. Furthermore, some of the prior art systems applying lethal or sterility causing genes require undesired steps, such as distribution of chemicals into the environment. This is not well seen by the users or public at large. The lacking possibility of reuse of crop can be mentioned.

The main objective of the present invention is to provide an increased security level for controlling introgression or transgene segregation and escape into the environment.

A further objective of the present invention is to allow the farmer provided with the correct instructions to reuse his crop.

Another objective of the present invention is to provide a system in which the effects of the genes having a blocking effect can be recovered without using chemicals and if chemicals must be used the recovery can be carried out under confined conditions.

### The Summary of the Invention

The increased security level for controlling transgene segregation and escape into the environment is achieved by a method and/or a system including a complex of DNA constructs comprising one or more Blocking Constructs (BCs) comprising genes capable of blocking one or more functions, which are essential for the survival and sexual reproduction of a transgenic organism. The increased security level is achieved by incorporating into the recipient organism or Sexually Reproducing plant in the form of one or more Transgenic Inserts (TIs) or DNA cassettes, one or more Blocking Constructs (BCs), which can be placed into an intron of a Transgene of Interest (TGI) or placed in close proximity with the Transgenic Insert (TI) comprising one or more Transgenes of Interest (TGIs).

The function blocked by the Blocking Construct (BC) is preferably recovered by a user-controlled intervention which is not present under natural conditions. If out-crossing of the transgenic organism happens the function which is essential for survival or sexual reproduction is blocked. The user-controlled intervention is such that it can be repeated not only once but several times and preferably the intervention does not require chemical interventions, which have to be carried out in the environment.

The increased security level as compared to prior art methods and systems is achieved specifically by using two or more Blocking Constructs (BC) placed on each side of a Transgenic Insert (TI) comprising one or more Transgenes of Interest (TGIs) or by placing the Blocking Construct (BC) in an intron of a Transgene of Interest (TGI).

Accordingly, the present invention is related to a method for increasing the security level for controlling transgene segregation and preventing the escape of transgenes into the environment by a molecular control mechanism comprising the steps of constructing one or more complexes of DNA constructs, herein called Recoverable Block of Function (RBF) systems comprising in addition to one or more Transgenes of Interest (TGIs), one or more Blocking Constructs (BCs) located in the close proximity of the Transgene of Interest (TGI), preferably in an intron of a Transgene of Interest (TGI) or if more than one Blocking Constructs (BCs) are used between said Blocking Constructs (BCs), and at least one means for recovering or a recovering tool, i.e. a user-controlled means or intervention for recovering the blocked functions. Said Blocking Construct (BC) has the capacity of blocking at least one function essential for the survival and/or sexual reproduction of a Sexually Reproducing plant.

The nucleotide sequence or gene providing the blocking effect in the Blocking Construct (BC) is either the Transgene of Interest (TGI) or it is closely linked to at least one Transgene of Interest (TGI) encoding the desired transgenic product. The recovering of the blocked functions comprises at least one user-controlled intervention optionally combined with one or more Recovering Constructs (RCs). The control takes place automatically by negative selection in the generations following the first escaped hybrid generation comprising a Transgene of Interest (TGI).

The presence of a Recovering Construct (RC) in the Recoverable Block of Function (RBF) technology of the present invention enables in the Blocking Construct (BC), the use of transgenes, which can be lethal or cause sterility in the host plant. The use of such genes makes the negative selection required for the effect to take place, absolute and shortens the negative selection time needed as compared to the negative selection in mitigation technology. The security level of Recoverable Block of Function (RBF) systems using Double Blocking Constructs (BCs) is increased because the silencing or mutation in one of the Blocking Constructs (BCs) does not decrease the effect of the second Blocking Construct (BC). Alternatively, the security level of the Recoverable Block of Function (RBF) systems of the present invention can be increased by introducing the Blocking Construct (BC) into an intron of the Transgene of Interest (TGI). This reduces the possibilities that the Blocking Construct (BC) is inactivated by crossing over or mutated without detrimental effects being caused to the Transgene of Interest (TGI), too. Therefore, the combination of the Recoverable Block of Function (RBF) concept with a Multiple, i.e. Double or Triple and/or intron-introduced Blocking Construct (BC) system provides a substantially increased security level in the control of transgene segregation and transgene escape to make the system feasible in transgenic plant and animal DNA technology in practice.

The transgenic Sexually Reproducing plant is ultimately prevented from sexual reproduction or from producing viable progeny by the Blocking Construct (BC), which under natural, non-controlled conditions arrests a function, which is essential for the survival or reproduction of said Sexually Reproducing plant. The blocked function can be recovered in order to allow normal development, proliferation, growth and sexual reproduction for agricultural, horticultural, forestal, industrial or any other feasible purposes, by applying, at least one user-controlled intervention, which at a susceptible moment in the development cycle of the Sexually Reproducing plant recovers the blocked function. Said means for recovering comprises an external, user-controlled treatment or manipulation step, including a chemical, physical or mechanical intervention, which recovers or unblocks the blocked function.

The transgene is prevented from leaking into the environment through hybridization or out-crossing of the parental transgenic Sexually Reproducing plant with its wild-type relatives or other cultivated non-transgenic or transgenic relatives, by the function of the Blocking Construct (BC) unit, which under natural conditions arrests the essential function of the transgenic Sexually Reproducing plant or any hybrids carrying said Blocking Construct (BC) as long as no user-controlled external intervention is provided. This control of segregation rapidly leads to extinction of the transgene in nature.

The present invention provides a complex of DNA constructs for preventing the segregation and escape of a transgene in a Sexually Reproducing plant into the environment. Said complex of DNA constructs, herein called the Recoverable Block of Function (RBF) system; comprises one or more Blocking Construct (BC) units containing at least one nucleotide sequence capable of blocking a molecular or physiological function which is essential for the survival and/or reproduction of a transgenic Sexually Reproducing plant.

Said nucleotide sequence gene in the Blocking Construct (BC) can alternatively be the Transgene of Interest (TGI), i.e. it can be the nucleotide sequence forming a self-controlling entity. Preferably, it is placed closely linked in immediate proximity or vicinity of the Transgene of Interest (TGI), either in the intron or between two Blocking Constructs (BCs). The Recoverable Block of Function (RBF) system may also comprise one or more optional Recovering Constructs (RCs), which are placed close to the Blocking Constructs (BCs) or in non-allelic chromosomes. The Recovering Constructs (RCs) should not be placed in a sister chromosome of the chromosome containing the Transgene of Interest (TGI) flanked by Blocking Constructs (BCs) or with Blocking Constructs (BCs) inserted in the intron of the Transgene of Interest (TGI). The Recovering Constructs (RC) are regulatable by an external, user-controlled, artificial intervention. In other words, the Recoverable Block of Function (RBF) system can function with or without the Recovering Construct (RC) as long as some external means or interventions, a user-controlled treatment for recovering, is available and can be applied.

The transgenic Sexually Reproducing plant is prevented from reproducing by withdrawing the user applied intervention, which prevents the action of the Blocking Construct (BC). The action, e.g. out-crossing, arrests and/or alters an essential function effecting the survival, development and/or sexual reproduction of a transgenic Sexually Reproducing plant.

The blocked function is recovered at a susceptible moment of the growth or developmental cycle of the Sexually Reproducing plant in order to allow normal growth and reproduction of said organism for production purposes, including agricultural, horticultural, forestal and/or industrial applications, by applying at least one externally applicable, artificial and user-controlled intervention.

If a transgenic Sexually Reproducing plant is prone to interbreeding or out-crossing with its cultured of wild-type relatives, the transgene segregation is controlled and/or the leakage of said transgene into nature is prevented by the fact that in nature no intervention or treatment is provided that would unblock the negative effects of the Blocking Construct (BC). Accordingly, under natural conditions at least one essential molecular or physiological function of the transgenic Sexually Reproducing plant is arrested which function prevents sexual reproduction.

In the method of the present invention, the function effecting the survival of the host Sexually Reproducing plant or its developmental and/or reproductive cycle is characterized by arresting the development, altering the phenotype or morphology of the transgenic Sexually Reproducing plant in an organ-specific, spatiotemporal or constitutive manner, in such a way that survival or sexual reproduction is prevented.

A suitable moment for removing or recovering the blocking effect by applying an external, artificial and user-controlled intervention step, or treatment is a susceptible moment or stage in the developmental cycle of the Sexually Reproducing plant, at which moment, an organ-specific, spatiotemporal or constitutive morphological change prevents the Sexually Reproducible plant from free hybridization or crossing and thereby from sexual reproduction under natural conditions. Such susceptible moments are for example early or late seed germination, flowering, incapability to form inflorescence, flowers or fruits and/or formation of dwarf phenotype, especially when the Blocking Construct (BC) comprises a nucleotide sequence, the expression of which blocks an essential molecular or physiological function or causes a morphological change preventing free hybridization and/or reproduction. The blocking occurs at the level of DNA, mRNA, protein or metabolite and results in death, phenotypical or physiological alteration, such as early or late seed germination, lack of growth and/or vegetation, incapability to form flowers, inflorescence, flowers or fruits, formation of dwarf phenotype, etc.

The Blocking Construct (BC) can be placed in the intron of the nucleotide sequence of the Transgene of Interest (TGI) or two or more Blocking Constructs (BCs) are placed on each side of a Transgenic Insert (TI) comprising one or more Transgenes of Interest (TGIs). If the two Blocking Constructs (BCs) are the same or different and recoverable by the same or different recovering mechanisms, they are placed at both sides of the nucleotide sequence of interest (TGI) resulting in a Multiple, i.e. Double or Triple Recoverable Block of Function (M-RBF, D-RBF or T-RBF) system. In contrast, the Recovering Construct (RC) should be placed in the same plant, but preferably in different non-allelic chromosomes resulting in a Segregating Recoverable Block of Function (S-RBF) system.

In Multiple Recoverable Block of Function (M-RBF) systems a first Recovering Construct (RC₁) is placed close to a second Blocking Construct (BC₂) in a first Transgenic Insert (TI). Placed in a second non-allelic chromosome a second Recovering Construct-first Blocking Construct (RC₂-BC₁) complex controls the Transgenic Insert (TI) and the first Transgenic Insert (TI) controls the second Transgenic Insert (TI), respectively. The Transgene-of Interest-Blocking-Constructs (BC₁-TGI-BC₂) complex controls another Transgene-of-Interest-Blocking-Construct (TGI-BC) complex with increased reliability and negative selection of Transgene of Interest (TGI).

Also contemplated in the present invention are cloning vectors, i.e. DNA cassettes and cells or cell-lines for convenient preparation of transgenic plants or transgenic non-human animals harboring one or more Transgenic Insert (TI) controlled by the Recoverable Block of Function (RBF) systems of the present invention. A novel polycloning site shown in SEQ ID NO :4: is also presented for convenient insertion of Transgenes of Interest (TGIs) and other Blocking and Recovering Constructs (BCs and RCs). Also described are synthetic nucleotide sequences, which are adapted for insertion either into introns of Transgenes of Interest (TGIs) or for flanking the Transgenes of Interest (TGIs) and for insertion into model plants.

The characteristic features of the present invention are defined in more detail in the claims.

### The Detailed Description of the Drawings

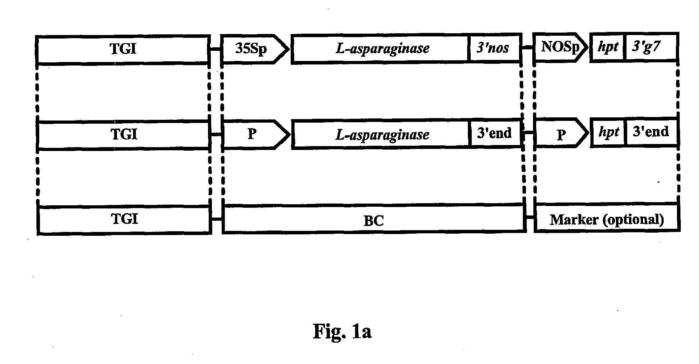

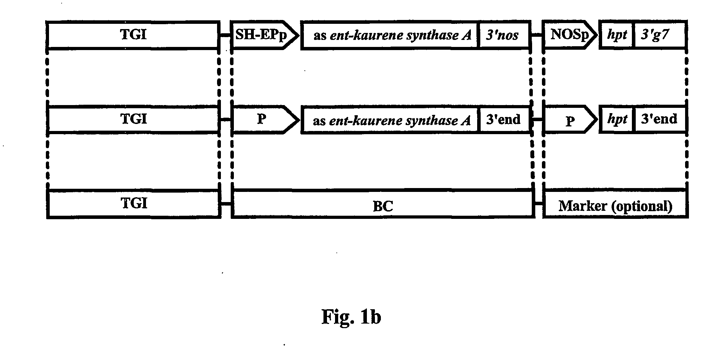

**Figure 1** is a schematic presentation of molecular constructs used in the Simple Recoverable Block of Function (RBF) systems. The constructs are presented in detail (upper row) to show the principles of placing the genes and on a more general level (middle and lower rows). Boxes of promoters presented with arrowheads show the direction of the gene sequences. Abbreviations: p, promoter; 3'end, 3'end of the gene or polyadenylation signal site; as, antisense.
**Figure 1a** presents the construct described in Example 1
**Figure 1b** presents the construct described in Example 2.

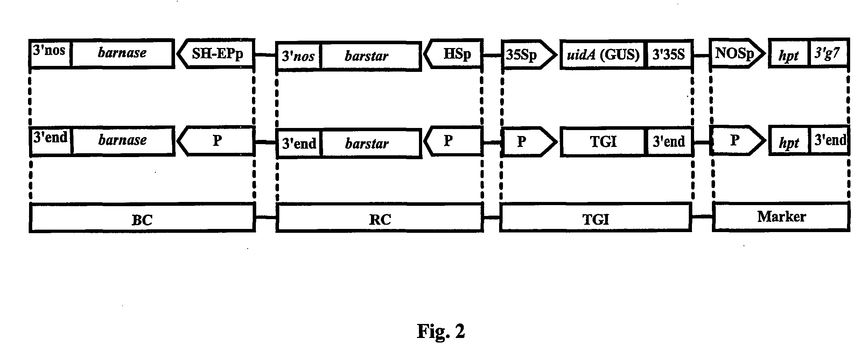

**Figure 2** presents the molecular constructs used in the Recoverable Block of Function (RBF) system and described in Example 3. The constructs are presented in detail (upper row) to show the principles of placing the genes and on a more general level (middle and lower rows). Boxes of promoters presented with arrowheads show the direction of the gene sequences. Abbreviations: p, promoter; 3'end, 3'end of the gene or polyadenylation signal site.

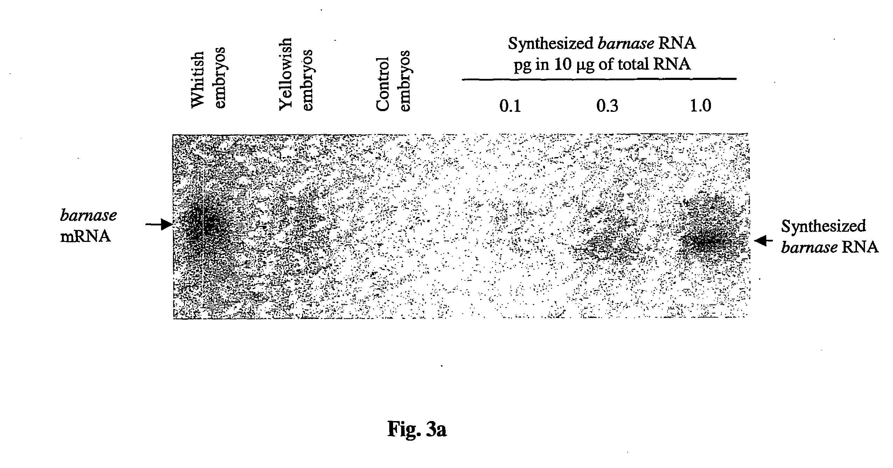

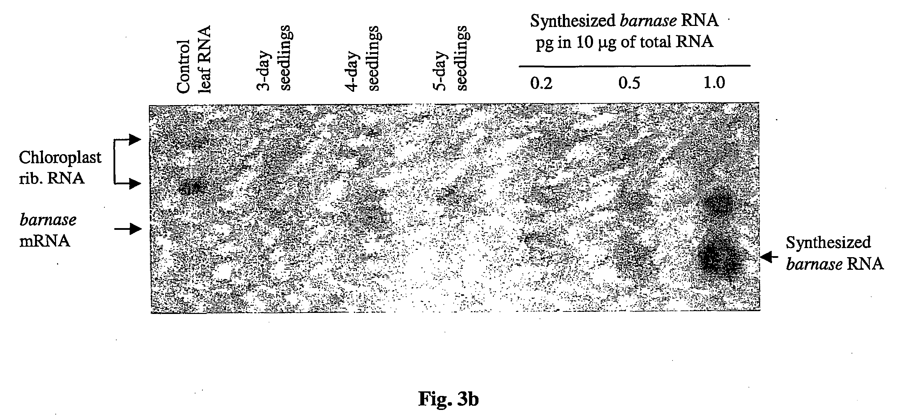

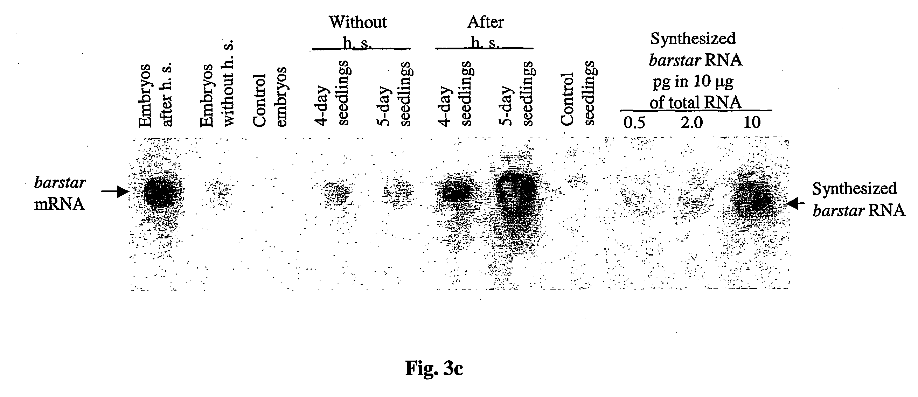

**Figure 3**. Northern blot analysis of barnase and barstar expression in transgenic tobacco during embryo maturation, seed germination and with/without heat shock treatment. Barnase and barstar genes are used as blocking and recovering genes, respectively, in the Recoverable Block of Function (RBF) system.
**Figure 3a** shows the expression of barnase mRNA in tobacco embryos at middle stage (whitish embryos) and late stage (yellowish embryos) of embryogenesis. 10 mg of total RNA was loaded in each lane. Total RNA from non-transgenic embryos and 0.1, 0.3 and 1.0 pg of synthesized barnase RNA mixed with 10 mg of total RNA from non-transformed tobacco embryos were used as controls. Barnase mRNA expression was at the middle stage of embryogenesis 0.1 pg/mg of total RNA and at the late stage of embryogenesis 0.03 pg/mg of total RNA.
**Figure 3b** shows the expression of barnase mRNA in tobacco seedlings. 10 mg of total RNA was loaded in each lane. Total RNA from non-transgenic embryos and 0.2, 0.5 and 1.0 pg of synthesized barnase RNA mixed with 10 mg of total RNA from non-transformed tobacco embryos were used as controls. The expression of barnase mRNA started on the third day of germination, achieved a maximum 0.04 pg /mg of total RNA on the fourth day and faded on the fifth day of germination. Two non-specific bands of chloroplast ribosomal RNA were also detected on the blot.
**Figure 3c** shows the expression of barstar mRNA after heat shock (h.s.) and without heat shock. 10 mg of total RNA was loaded in each lane. 0.5, 2.0 and 10 pg of synthesized barstar RNA mixed with 10 mg of RNA from non-transgenic control tobacco embryos were used as positive control. Heat shock treatment activated barnase mRNA expression up to 1 - 3 pg/mg of total RNA in both the embryogenesis and germination stages of the development. Barstar mRNA expression under HSp was leaky without temperature treatment, at the level of 0.05 - 0.2 pg/mg of total RNA.

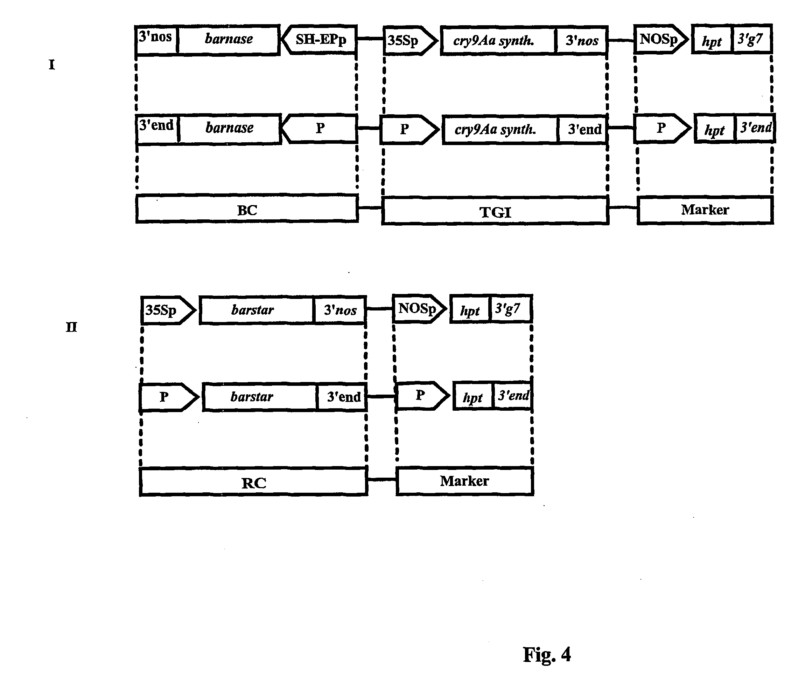

**Figure 4** presents the molecular constructs used in the Recoverable Block of Function (RBF) system and described in Example 4. The constructs are presented in detail (upper row) to show the principles of placing the genes and on a more general level (middle and lower rows). Boxes of promoters presented with arrowheads show the direction of the gene sequences. Abbreviations: p, promoter; 3'end, 3'end of the gene or polyadenylation signal site. The constructs I and II are inserts in different non-allelic chromosomes and are shown separately.

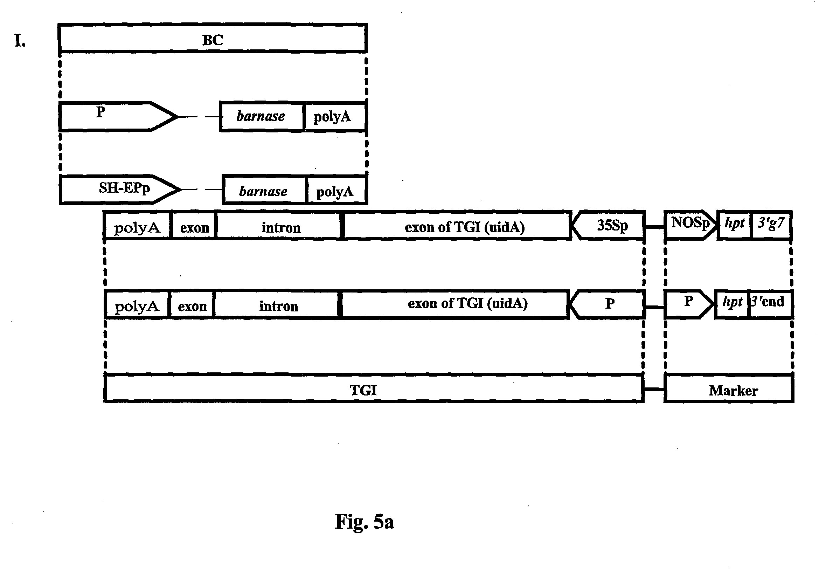

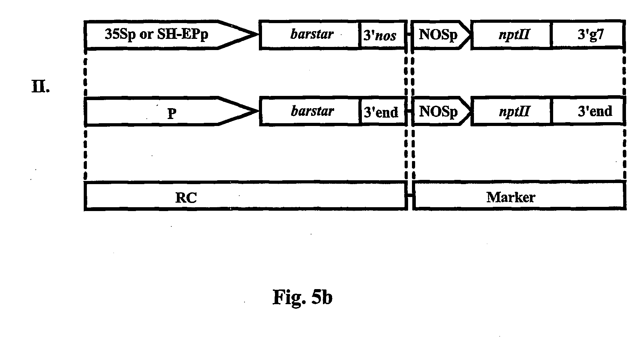

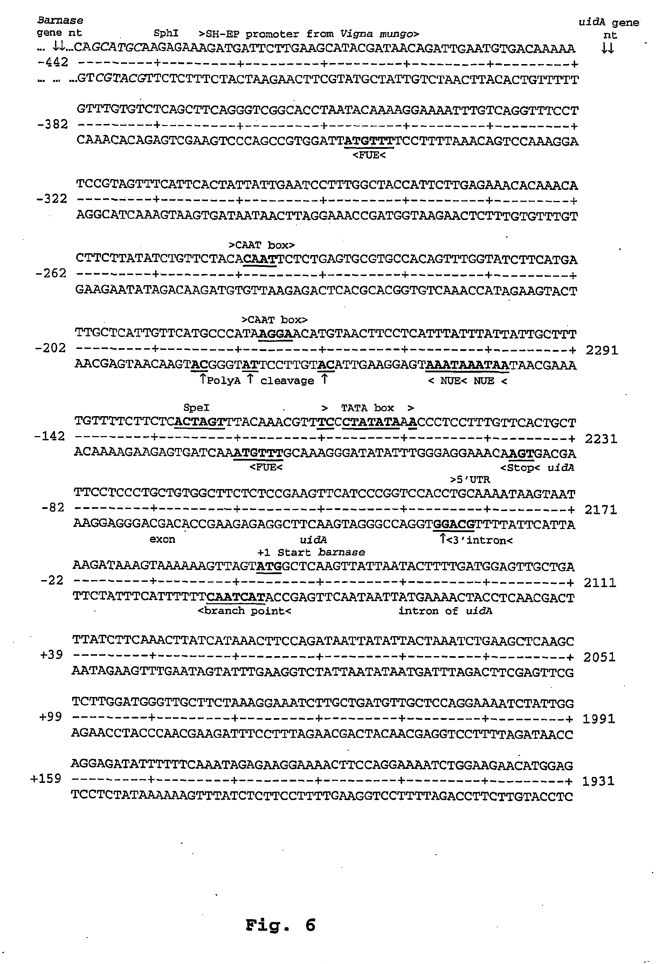

**Figure 5** presents the molecular constructs used in the Recoverable Block of Function (RBF) system and described in Example 5. The constructs are presented in detail (upper row) to show the principles of placing the genes and on a more general level (middle and lower rows). Boxes of promoters presented with arrowheads show the direction of the gene sequences. I and II are inserts in different non-allelic chromosomes and are shown separately. Abbreviations: p, promoter; 3'end, 3'end of the gene or polyadenylation signal site.
**Figure 5a** shows the construct in chromosome I in detail and on a more general level.
**Figure 5b** shows the construct in chromosome II in detail and on a more general level.

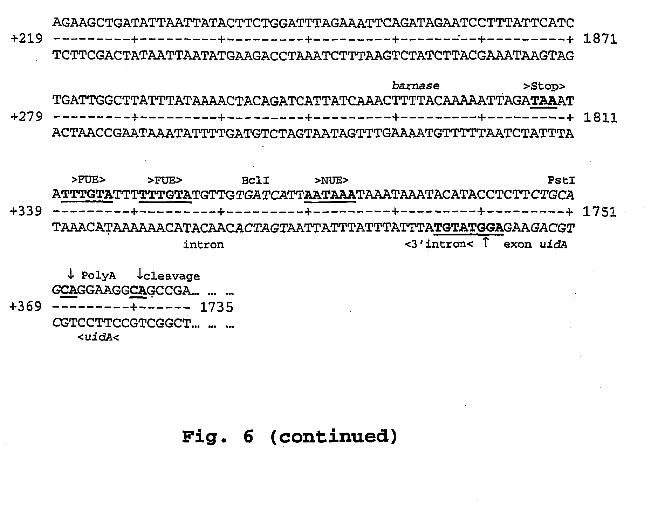

**Figure 6** presents the sequence of the barnase gene placed inside the intron of the uidA gene: The barnase gene construct is shown downstream and contains the SH-EP promoter (about last three hundred nucleotides are shown) coding sequence and polyadenylation signal sites. The uidA gene construct is placed upstream beginning from the nucleotide 1735 of the coding sequence. The uidA gene construct shows exon-intron-exon-polyadenylation site sequences. Abbreviations: nt nucleotides; FUE far upstream element of polyadenylation site; NUE near upstream element of polyadenylation site; SphI, SpeI, PstI, BclI, restriction sites; > and <, directions of the signal sites, CAAT and TATA, signal boxes of the promoter.

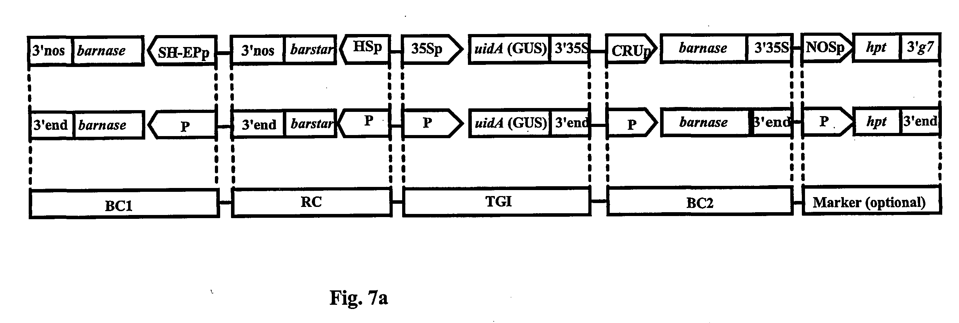

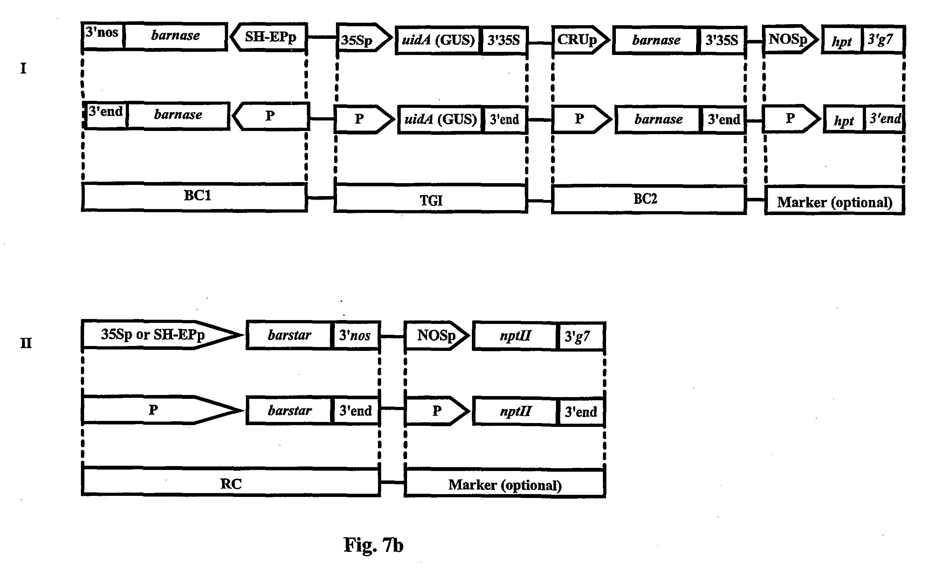

**Figure 7** presents the molecular constructs used in the Recoverable Block of Function (RBF) system and described in Example 6. The constructs are presented in detail (upper row) to show the principles of placing the genes and on a more general level (middle and lower rows). Boxes of promoters presented with arrowheads show the direction of the gene sequences. I and II are inserts in different non-allelic chromosomes and are shown separately. Abbreviations: p, promoter; 3'end, 3'end of the gene or polyadenylation signal site.
**Figure 7a** shows the one-insert construct of double inducing Recoverable Block of Function (RBF) system.
**Figure 7b** shows the two-insert construct of double segregating Recoverable Block of Function (RBF) system.

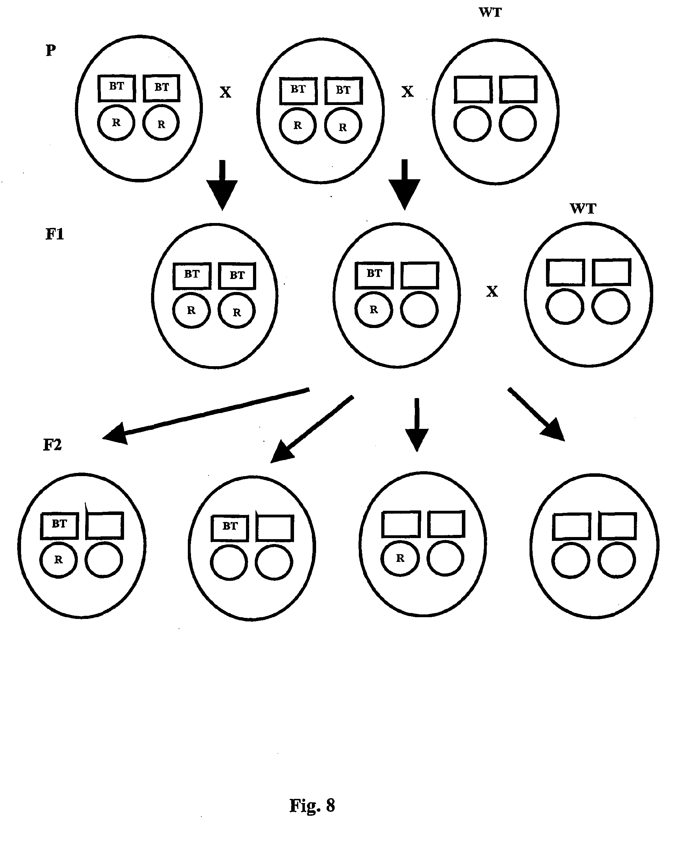

**Figure 8** is a schematic drawing showing the hybridization of the transgenic plants carrying Transgenic Inserts (TIs) of a Segregating Recoverable Block of Function (S-RBF) system (described in Examples 4, 5 and 6 and presented in Figures 4, 5 and 7b). Chromosomes carrying Blocking Construct (B) linked to the gene of interest (T) as well as Recovering Construct (R) placed in a different non-allelic chromosome are shown. Wild type (WT) chromosomes are shown without markings BT or R. The parental plants (P) included in hybridization are shown in the first line. The condition of genes involved in the Recoverable Block of Function (RBF) does not change in the result of intraline crossing. Intraline F1 progeny has the homozygous genotype for Recoverable Block of Function (RBF) and the gene of interest. External regulation of the Recoverable Block of Function (RBF) implies to support homozygous condition of the transgenes through intraline hybridization. In the case of outside hybridization, the first F1 hybrid progeny genotypes are heterozygous for all transgene constructs. The plants remain alive. The segregating Recoverable Block of Function (RBF) starts to act from F2 hybrid progeny in the case of outside hybridization. In F2 hybrids, only half of the plants carry Blocking Construct (B) linked to the transgene of interest (T). Half of them will be unable to reproduce because of absence of Recovering Construct (R). Therefore, beginning from the second outside hybrid progeny, 50% negative selection eliminates the transgene of interest from natural populations. In the case of segregating Recoverable Block of Function (RBF), Recovering Construct (R) can freely segregate in the genomes of wild population plants.

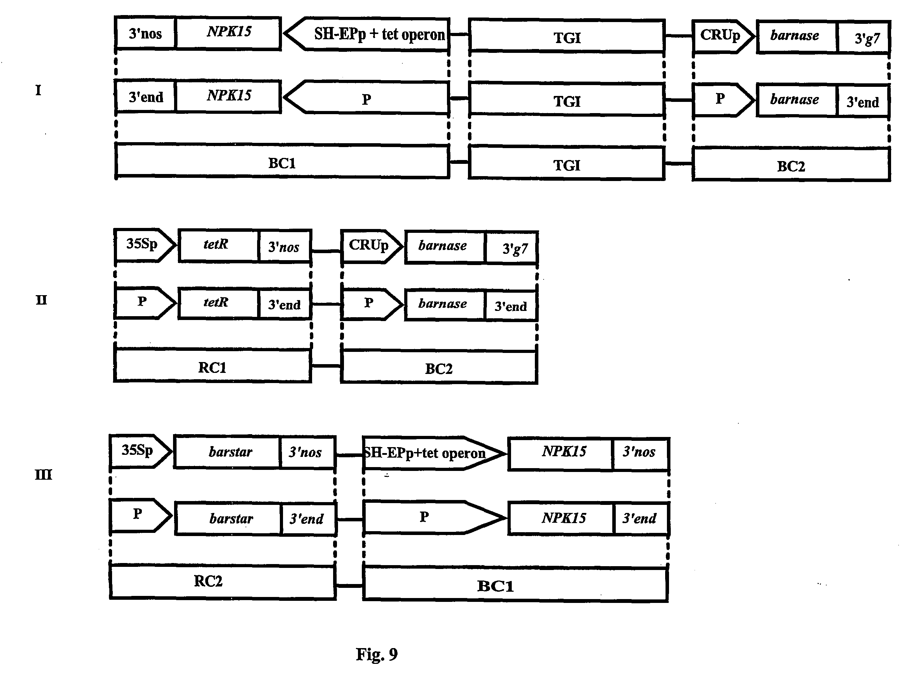

**Figure 9** presents the molecular constructs used in the Recoverable Block of Function (RBF) system and described in Example 7. The constructs are presented in detail (upper row) to show the principles of placing the genes and on a more general level (middle and lower rows). Boxes of promoters presented with arrowheads show the direction of the gene sequences. I, II and III are inserts in different non-allelic chromosomes and are shown separately. Abbreviations: p, promoter; 3'end, 3'end of the gene or polyadenylation signal site; BC1, first Blocking Construct; BC2, second Blocking Construct; RC1, first Recovering Construct; RC2, second Recovering Construct.

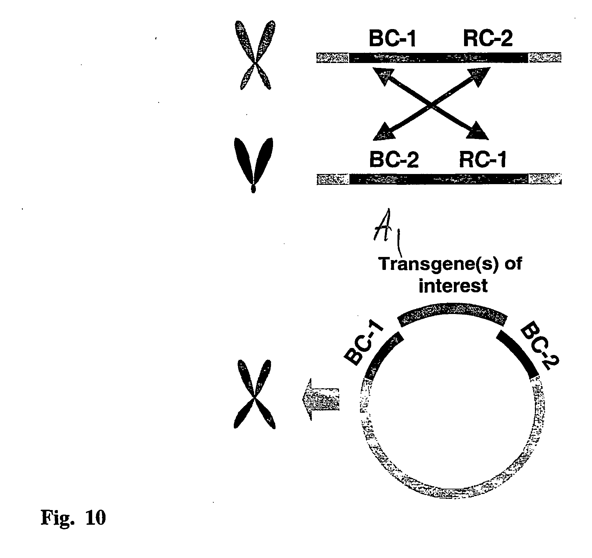

**Figure 10** presents Triple Recoverable Block of Function (T-RBF) system. A ready-made recipient transgenic Sexually Reproducing plants carries three Transgenic Inserts (TIs) or DNA cassettes each located in separate non-allelic chromosomes in the recipient Sexually Reproducing plant line. In a first Transgenic Insert (TI-I) a first Blocking Construct (BC-1) and a second Recovering Construct (RC-2) and in a second Transgenic Insert (TI-II) a second Blocking Construct (BC-2) and first Recovering Construct (RC-1). Said first and second Transgenic Insert (TI-I and TI-II) are placed in two different non-allelic chromosomes. The transgene(s) of interest (TGIs) are cloned, into the polycloning site between two Blocking Constructs (BC-1 and BC-2) proving a third Transgenic Insert III, which id transformed into a third non-allelic chromosome of the recipient Sexually Reproducing Multicellular Organism (SRMO) line. The arrows and arrowheads indicate interactions between the Blocking and Recovering Constructs (BC & RC). The first Recovering Construct (RC-1) controls the Blocking Construct (BC-1) in the first and third Transgenic Insert (TI-I and TI-III) and the second Recovering Construct (RC-II) controls the second Blocking Construct (BC-II) in the second Transgenic Insert (TG-II) and in the third Transgenic Insert (TI-III). If the resulting Sexually Reproducing plant hybridizes with wild relatives, the chromosomes will separate according to the Mendelian law and the frequency of 7/8 in each generation.

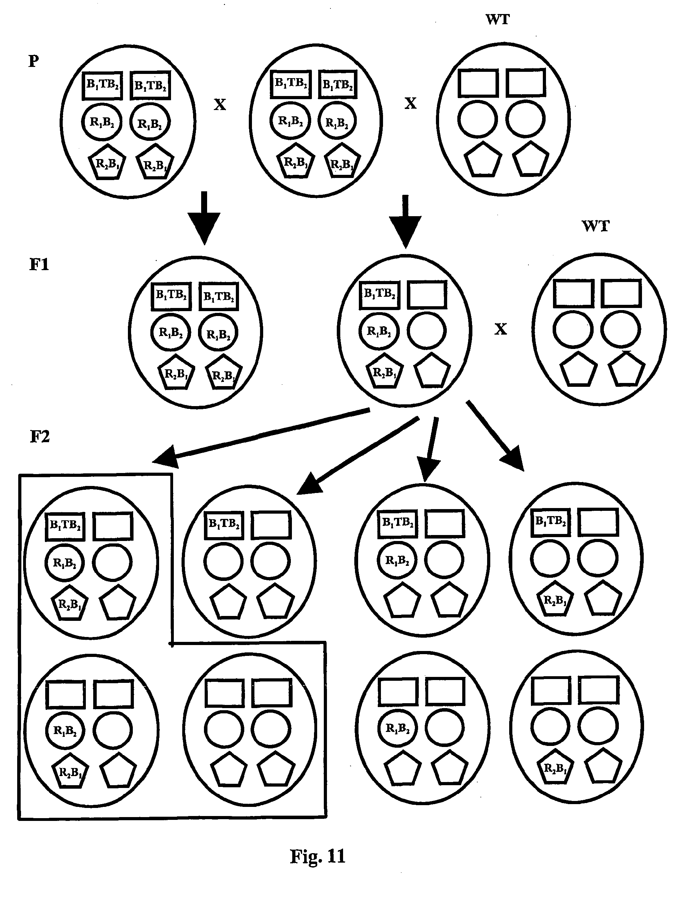

**Figure 11** is a schematic drawing showing the hybridization of transgenic plants carrying the Transgenic Inserts (TIs) of a Triple Recoverable Block of Function (T-RBF) system. The constructs of the first Recoverable Block of Function (RBF) are depicted as B1 and R1 for Blocking and Recovering Constructs, respectively. B2 and R2 constructs belong to the second Recoverable Block of Function (RBF). The Blocking and Recovering Constructs are placed in different non-allelic chromosomes in opposite order and linked in pairs R1 +B2 and R2+B1. The transgene of interest (T) is placed in the third non-allelic chromosome. It is situated between the first (B1) and the second (B2) Blocking Construct. The intraline crossing supports homozygous genotype of both Recoverable Block of Function (RBF) in progenies. F1 outside hybrid progeny carry heterozygous genotype for all Recoverable Block of Function (RBF) constructs and the gene of interest. The plants of the progeny are able to reproduce. Segregation of the constructs in F2 outside hybrid progeny leads to strong negative selection of the Sexually Reproducing plant carrying the gene of interest (T). Only one from eight hybrid genotypes is able to sexually reproduce and carries the transgene of interest (T). None of the transgenic constructs can freely hybridize with wild relatives. F2 progeny drawn in the box is capable to sexually reproduce.

### The Detailed Description of the Invention

### Terms Used in the Invention

In the present invention most of the terms used have the same meaning as they generally have in the fields of recombinant DNA techniques, molecular biology and in plant and animal breeding. Some terms are, however, used in a somewhat different way and are explained in more detail below.

The term "Recoverable Block of Function (RBF) system" means a conceptual molecular system or molecular control mechanism including one or more DNA constructs or Transgenic Inserts (TIs) which comprise one or more Blocking Constructs (BCs) having a nucleotide or DNA sequence capable of blocking a function which is essential for the survival or reproduction of the transgenic Sexually Reproducing Multicellular Organism (SRMO) as well as user-controlled means for recovering the blocked function, i.e. a recovering tool. The Recoverable Block of Function (RBF) system performs the control of segregation and prevents leakage to the environment of the Transgene(s) of Interest (TGI_{S}) in the Sexually Reproducing plant, which include both plants and non-human animals to which the Recoverable Block of Function (RBF) system is applicable. The Transgenic Insert (TI) of the Recoverable Block of Function (RBF) system is introduced into the Sexually Reproducing plant together with the Transgenes of Interest (TGIs) by *per se* known transformation processes.

It is to be noted that some of the Recoverable Block of Function (RBF) systems defined below are not novel as such without including the novel feature of the present invention, which is the location of the Blocking Constructs (BCs) either in the intron of the Transgene of Interest (TGI) or two Blocking Constructs (BCs) on each side of a Transgene of Interest (TGI) or in a Multiple Recoverable Block of Function (M-RBF) system.

The term "Delayed Recoverable Block of Function (D-RBF) system" or "Segregating Recoverable Block of Function (S-RBF) system" means a kind of Recoverable Block of Function (RBF) system, wherein the Recovering Construct (RC) or constructs are placed in the same individual organisms in different non-allelic chromosomes apart from the Blocking Construct (BC) which is closely linked to the Transgene(s) of Interest (TGIs), i.e. a complex TGI-BC-construct. Therefore, segregation takes place independently of the complex TGI-BC-construct. This means that the blocking factor does not start to act until the second heterozygous hybrid generation (F₂), when the Blocking and Recovering Constructs (BCs and RCs) segregate to different generative cells as described below.

The Recoverable Block of Function (RBF) model systems can be divided in the following types according to the mechanism of action and the arrangement or structure of the constructs.

If the Recoverable Block of Function (RBF) system comprises solely of a Blocking Construct (BC) and its action is compensated by externally applied recovering, the system is a Simple Recoverable Block of Function (RBF) system. If the Recovering Construct (RC) is activated in response to an outside stimulus and releases a function arrested by the Blocking Construct (BC) expressed under a different promoter, the model is a General or Full herein called Inducible Recoverable Block of Function (I-RBF) system. If the Recovering Construct (RC) is placed in a different non-allelic chromosome, the model is a Segregating Recoverable Block of Function (S-RBF) system. The Reversed Segregating Recoverable Block of Function (RS-RBF) system comprises two different Transgenic Inserts (TIs) of the Recoverable Block of Function (RBF) system. These are situated in different non-allelic chromosomes opposite to each other so that the Blocking Construct (BC) of the first Recoverable Block of Function (RBF) is linked to a Recovering Construct (RC) of the second Recoverable Block of Function (RBF) system and the Blocking Construct (BC) of the second Recoverable Block of Function (RBF) system is linked to the Recovering Construct (RC) of the first Recoverable Block of Function (RBF) system.

The term "Externally Compensated or Simple Recoverable Block of Function (RBF)" means a Recoverable Block of Function (RBF) system in which consists solely of the Blocking Construct (BC). The Blocking Construct (BC) can be constitutively active as described in Example 1, development specifically as described in Example 2 or organ specifically. The recovering tool comprises an external compensation of the required metabolite, for example an amino acid as described in Example 1, hormone described in Example 2 or some other metabolite. There is no Recovering Construct (RC).

The term "Inducible Recoverable Block of Function (I-RBF)" means a Recoverable Block of Function (RBF) system which consists of both a Blocking Construct (BC) and a Recovering Construct (RC). Expression of the Blocking Construct (BC) can be constitutive or organ or development specific as described in Example 3. The Recovering Construct (RC) can be responsive to an external physical stimulus as shown in Example 3 or a chemical stimulus as described in WO 94/03619. The physically Inducible Recoverable Block of Function (I-RBF) of the present invention differs from the chemically Inducible Recoverable Block of Function (I-RBF) described in WO 94/03619 also by the fact that the Blocking Construct (BC) is placed in the intron of the Transgene of Interest (TGI).

The term "Recovering Construct (RC)" can be placed in the same chromosome as the Blocking Construct (BC) forming an "Inducible Recoverable Block of Function (I-RBF) system". The Recovering Construct (RC) can be placed in the same chromosome as or in a chromosome which is allelic with the Transgene of Interest (TGI) and/or Blocking Construct (BC) as described in WO 00/37660, or it can be placed in a different, non-allelic chromosome resulting in a Segregating Recoverable Block of Function (S-RBF) system.

The term "Delayed Recoverable Block of Function (RBF)" or "Segregating Recoverable Block of Function (S-RBF)" means a Recoverable Block of Function (RBF) system comprising a Blocking Construct (BC) and a Recovering Construct (RC) which are located in different non-allelic chromosomes in the same individual organism. The recovering process or intervention in this system implies intraline crossing to support homozygous conditions of the Recoverable Block of Function (RBF) genes and to prevent separation of the Blocking and Recovering Constructs (RC&BC)

The term "Reversed Delayed Recoverable Block of Function System (RD-RBF)" or "Reversed Segregating Recoverable Block of Function System (RS-RBF)" means that an additional Blocking Construct (BC) is linked to the first Recovering Construct (RC₁) in the first Transgenic Insert (TI) and this additional Blocking Construct (BC) is recovered with a second Recovering Construct (RC₂) linked to the Transgene of Interest (TGI) and the first Blocking Construct (BC₁) in a second Transgenic Insert (TI). The Reversed Segregating Recoverable Block of Function System (RS-RBF) controls the release of both the Blocking and the Recovering Constructs (BC & RC) into the environment.

A Segregating Recoverable Block of Function (S-RBF) system does not work in the first generation of outline hybridization because it will be present in heterozygous condition marked as BbRr (where 'b' and 'r' are recessive alleles which do not contain a Blocking Construct (BC) or a Recovering Construct (RC), respectively). Accordingly, both of the constructs act as in a homozygous parental line. The Segregating Recoverable Block of Function (S-RBF) system starts to act from the second out-crossing generation, when all the Bbrr hybrids will die or have an altered feature because of the lack of the Recovering Construct (RC). The Recoverable Block of Function (RBF) system implies 50 % negative selection of the Transgene of Interest (TGI) linked to the Blocking Construct (BC) in each hybrid generation after the first hybrid progeny. The Segregating Recoverable Block of Function (S-RBF) system implies recognition of the fact that Recovering Construct (RC) may be released to the environment.

The term "Blocking Construct (BC)" means a DNA or nucleotide sequence, which is vitally important or essential for the survival and/or sexual reproduction of the transgenic host organism. The Blocking Construct (BC) can simultaneously be a Transgene of Interest (TGI) forming a self supporting entity. The Blocking Constructs (BCs) are preferably others than the Transgenes of Interest (TGIs) but they should be closely linked to the Transgenes of Interest (TGIs). The blocking DNA or nucleotide sequence in the Blocking Construct (BC) is responsible for blocking a particular, molecular or physiological function in the host organism. It comprises a nucleotide (DNA or RNA) sequence e.g. a gene, the action of which leads to extinction or sterility of the host organism. Accordingly, the Sexually Reproducing plant becomes incapable of sexual reproduction under unmanipulated, natural conditions if they escape. Said nucleotide sequences of the Blocking Constructs (BCs) together with the means for recovering, i.e. the recovering tool controls the transfer of Transgenes of Interest (TGIs) to the following generations and accordingly the spread of the gene(s) to the surrounding natural or cultured populations. The Blocking Construct (BC) is preferably linked to the Transgene of Interest (TGI), in the extreme case it is placed in the intron of the Transgene of Interest (TGI) flanking it. A prerequisite is that at least one, preferably, two Blocking Constructs (BCs) are situated in the same Transgenic Insert (TI) as the Transgene of Interest (TGI). All the constructs should be present in the same individual. The Blocking Construct (BC) should express in the host organism either constitutively or in an organ-specific, developmental or spatiotemporal manner. The term "Blocking Construct" is synonymous to the terms "repressible lethal gene", "disrupter gene" or "terminator gene", which have been used in prior art publications WO 94/03619, WO 00/37660 or US 5,723,765.

The term "Reversed Delayed Recoverable Block of Function (RD-RBF)" or "Reversed Segregating Recoverable Block of Function (RS-RBF)" means a system that controls the release of both the Blocking Construct (BC) (linked to the Transgene of Interest (TGI)) and the Recovering Construct (RC). The segregation of a Recoverable Block of Function (RBF) system is shown schematically in Figure 11. The Recovering Construct (RC) contains another blocking gene, which controls the release of the Recovering Construct (RC). The blocking gene action is recovered by a second Recovering Construct (RC), which is linked to a first Blocking Construct (BC) and the Transgene of Interest (TGI). In Figure 11, the Transgenic Insert of the Recoverable Block of Function (RBF) system, in the alleles, are for the convenience marked as T for the Transgene of Interest, B1 for the first Blocking Construct acting in combination with R1 which is the first Recovering Construct and B2 for the second (different from the first) Blocking Construct acting in combination with R2, which is the second recovering construct. The allelic chromosomes B1B1TTR2R2 are situated in one pair of the allelic (sister) chromosomes and R1R1B2B2 are situated in another pair of allelic chromosomes. The first out-hybrid will carry the genotypes B1b1TtR2r2 and R1r1B2b2, respectively. Thus, starting from the second out-hybrid generation the B1 Blocking Construct will control the release of the Transgene of Interest T, and the B2 Blocking Construct will control the release of the R1 Recovering Construct. Therefore, the Reversed Segregating Recoverable. Block of Function (RS-RBF) system controls the release of all the transgenic constructs from the plant. The external control or artificial treatment comprises a user-controlled interline crossing in order to support the homozygous condition of transgenic plants (in a similar way as in an ordinary Segregating Recoverable Block of Function (S-RBF) system. The second Recovering or Blocking Construct can be fused with the first Blocking or Recovering Construct in the same gene sequences as follows: B1 fused with R2 and B2 fused with R1. Certain types of Segregating and Reversed Recoverable Block of Function (RBF) are described in WO 00/376600. However, a Blocking Construct (BC) inserted in an intron of the Transgene of Interest (TGI) and Multiple including Double or Triple Recoverable Block of Function (RBF) are not disclosed.

The term "Intraline Crossing" means crossing between two homozygous organisms which crossing provides a homozygous genotype and in other words supports homozygous conditions and is an environmentally safe intervention provided by the present invention. The term "Introgression" means the flow of genes that bypass reproductive barriers and move between populations via the mating of fertile hybrids with parent populations.

As described above the aim of the present invention is to increase the reliability of known Recoverable Block of Function (RBF) systems. The goal is achieved by development of concepts of Multiple, including Double and Triple Recoverable Block of Function (RBF) systems as well as an intron-introduced Blocking Construct (BC) systems.

The term "Increased Level of Security" means that a security level in which the frequency of inactivation of the Transgene of Interest (TGI) is lower than 10⁻⁶, preferably it is lower than 10⁻⁸, more preferably it is lower than 10⁻¹⁰ and most preferably it is 10⁻¹² in cases where the inactivation of the blocking gene is 10⁻⁶. Double Recoverable Block of Function (RBF) decreases the frequency to 10⁻¹².

Double Recoverable Block of Function (D-RBF) with an inducible Recovering Construct (RC) has a 100 % negative selection if no inactivation is occurring. It is to be expected that the Sexually Reproducing Multicellular Organism (SRMO) looses the Transgene of Interest (TGI) closely bound to the Blocking Construction with a rate that is higher than the rate generally mentioned in prior art.

The term "Double Recoverable Block of Function (D-RBF)" means a Recoverable Block of Function (RBF) system that consists of two Blocking Constructs (BCs). Preferably two different Blocking Constructs (BCs) are expressed under the control of different promoters. The blocking genes are linked to the Transgene of Interest (TGI) from both sides. The Blocking Construct (BC) can even be inserted inside the gene in a suitable intron (Figure 5). The possibility that said Recoverable Block of Function (RBF) action is destroyed due to a crossing-over, deletion of DNA, promoter silencing mechanism or other similar events is thereby decreased.

Structurally Double Blocking Constructs (BCs) are linked to a Transgene of Interest (TGI) preferably from both sides of the gene. The Blocking Constructs (BCs) can consist of the same or different genes driven under the same or different promoters. A preferable arrangement of the complex DNA constructs is obtained when two different blocking genes are driven under different promoters and placed in distal positions of the Transgenic Insert (TI) comprising one or more Transgenes of Interest (TGIs). One Recovering Construct (RC) is sufficient, if the Blocking Constructs (BCs) contain the same functional gene (Example 6). Double Recoverable Block of Function (D-RBF) can contain two different Recovering Constructs (RCs), if the Blocking Constructs (BCs) contain different functional genes. The Recovering Constructs (RCs) can be placed in the same or different Transgenic Insert (TI). Thus, Double Recoverable Block of Function (D-RBF) can be based on the Inducible or Segregating Recoverable Block of Function (RBF). Inducible Double Recoverable Block of Function (D-RBF) with single Recovering Construct (RC) is described in Example 6.

The term "Multiple Recoverable Block of Function (M-RBF) system" or specifically Triple Recoverable Block of Function (RBF) is at present the most developed Multiple Recoverable Block of Function (RBF) system. The Triple Recoverable Block of Function (RBF) combines all the best traits of the Segregating, Double and Reversed Recoverable Block of Function (RBF) systems. It confers stronger negative selection than the Segregating Recoverable Block of Function (RBF). Triple Recoverable Block of Function (RBF) does not require special treatments besides intraline crossing which supports homozygous conditions like in Segregating Recoverable Block of Function (S-RBF) systems. It prevents any transgenic constructs from flowing into environment, because the protection is double. The Triple Recoverable Block of Function (RBF) system is quite easy to construct because different Recoverable Block of Function (RBF) genes are placed in three different Transgenic Inserts (TIs) in different non-allelic chromosomes. For the convenience the segregation is described as follows.

The transgene of interest (T) is placed between two different blocking constructs (B1 and B2) in the same manner as in Double Recoverable Block of Function (D-RBF) resulting in a system marked B1TB2. Two different recovering constructs (R1 and R2) are placed separately in two different non-allelic (non-sister) chromosomes. Additional blocking constructs are linked to the recovering constructs in the following order: R1+B2 and R2+B1. The inserts are cloned in the recipient plant line in homozygous condition in the non-allelic (non-sister) chromosomes. Additionally, a Reversed Recoverable Block of Function (RBF) containing no Transgene of Interest (TGI) is incorporated into a recipient Sexually Reproducing plant line and a Double-blocking Sexually Reproducing plant transformation vector carries a polycloning site placed between said two Blocking Constructs (BCs). This vector can be used for cloning different transgenes of interest into the recipient Sexually Reproducing plant line in third non-allelic chromosome (Figure 10).

The mechanism of the Triple Recoverable Block of Function (RBF) acts in the same manner as Segregating Recoverable Block of Function (S-RBF). It begins to work from the second hybrid progeny. Plants in the first hybrid progeny containing a construct of the Transgene of Interest (TGI) and being capable of sexual reduction are only 12,5 % from whole hybrid progeny or 25 % from hybrids carrying the Transgene of Interest (TGI). Schematically hybridization of the plants is shown in Figure 11.

A preferred embodiment of the Multiple Recoverable Block of Function (M-RBF) system is the "Triple Recoverable Block of Function (RBF) system", which is described in Example 7, but even more complex Recoverable Block of Function (RBF) systems can be constructed by one skilled in the art when following the general principles laid down in the disclosure of the present invention.

The term "means for recovering" or "recovering tool" means a method for recovering the Sexually Reproducing plant host from the detrimental consequences of the action of the Blocking Constructs (BCs). The recovering tool may comprise one or more interventions or treatments. Said user controlled manipulation steps lead to direct external compensation of the blocked function, which can recover the deficiency of a particular function. The recovering tool can also be an outside stimulus activating one or more Recovering Constructs (RCs) which have been inserted into the genome of the Sexually Reproducing plant. Thus, the recovering tool comprises at least one external user-controlled intervention combined with one or more optional "Recovering Constructs (RCs)".

The term "Recovering Construct (RC)" is synonymous with the term "repressor gene", which has been used in prior art publications and means a DNA construct or nucleotide (DNA or RNA) sequence, which recovers, unblocks or releases the function blocked by the Blocking Construct (BC). The Recovering Construct (RC) is introduced into the genome of the Sexually Reproducible plant, separately or together with the Blocking Construct (BC) and Transgene of Interest (TGI) or Transgenes of Interest (TGIs). The action of the Recovering Construct (RC) is always user-controlled and externally regulated. The Recovering Construct (RC) does not act, in other words it lacks function during the life of the Sexually Reproducible plant under non-treated, unmanipulated, natural conditions.

The term "user-controlled intervention" means that an external control of the function of the "recovering tool" can be provided by an outside stimulus on a responsive promoter. In case the Recovering Block of Function (RBF) system herein called the "Segregating Recoverable Block of Function (S-RBF) system" is used, the external regulation implies intraline crossing of the transgenic Sexually Reproducible plants to support the homozygous condition of the Recoverable Block of Function (RBF) system.

Preferred plants are flowering plants, which according to taxonomic classification include both angiosperms and gymnosperms, especially crop plants, such as cereals. Especially Sexually Reproducing plants are organisms which can interbreed or cross with other related wild-type or cultivated both transgenic and non-transgenic organisms.

The term "Transgene (gene or nucleotide sequence) of Interest or Transgene of Interest (TGI)" means a DNA or nucleotide sequence, including RNA sequences, which encode a desired gene product, i.e. a protein or an enzyme or other substances, which may include metabolites, hormones, toxins, antibiotics, etc., and which are obtainable as end-products by the action of the direct gene products. Said Transgenes of Interest (TGIs) are introduced into the genome of the Sexually Reproducing plant using available genetic transformation and/or transfer systems.

The DNA sequences or Transgenes of Interest (TGIs) usually encode products or molecules useful in agriculture, horticulture, forestry and/or other industrial applications. Alternatively, the gene products have some other feasible applications. Generally, the DNA sequence is foreign or heterologous to the Sexually Reproducing plant, but sometimes homologous nucleic acid or DNA sequences can be used and inserted e.g. as multiple copies, e.g. as tandem inserts, in order to obtain optional amounts of the desired product. In other words, the DNA sequence can consist of one gene or nucleotide sequence or multiples thereof. Alternatively, several different genes can be introduced as multicopies or tandem inserts in order to facilitate expression of complicated metabolic pathways. In a particular embodiment of the present invention the Blocking Construct (BC) itself serves as the Transgene of Interest (TGI).

The term "Blocking" is a molecular control mechanism, e.g. a nucleotide sequence the expression of which blocks, arrests or inhibits a function essential for the survival, growth, development and/or sexual reproduction and is capable of arresting the development of the molecular machinery of the host organism at the level of DNA, mRNA, protein or metabolite. If the molecular machinery is applied at a susceptible or vulnerable moment in the developmental cycle, it results in death, phenotype alteration, early or late seed germination or flowering, incapability to form inflorescence, flowers or fruitation, formation of dwarf phenotype or some other morphological changes, which prevent free hybridization or interbreeding of transgenic Sexually Reproducing plants under natural conditions.

The term "natural conditions" means the growing conditions, which are the usual ambient parameters, including temperature, humidity, irradiation, chemical composition of soils during growth or production of the Sexually Reproducing plants; including plant or animal in agriculture, horticulture, forestry as well as in nature.

The term "escape of a transgene into the environment" means prevention of transgene leakage into nature through hybridization or crossing of the parental transgenic organism with its wild-type or cultured non-transgenic relatives. In other words, undesired transfer of a gene or genes through sexual crossing is avoided and release of the transgene is prohibited. The transgenic organism is prevented from growing, reproducing sexually or developing viable progeny. This is achieved by allowing the Blocking Construct (BC) to function under normal, natural, unmanipulated, treatment- or intervention-free conditions. Said natural, unmanipulated conditions block, arrests, destroys or destructs a function which is essential for the survival or reproduction of the Sexually Reproducing plant. By segregation, said essential function of the Sexually Reproducing plant leads to the extinction of the transgene in nature.

"Recovering the blocked function" means unblocking the blocked function or recovering the blocked function in order to allow normal growth and sexual reproduction of the Sexually Reproducing plant for agricultural, horticultural, forestal, industrial or any other production purposes. The removal of the blocked function is obtainable by preferably artificial, user-controlled or user-regulated means applied from the outside. Said externally applicable means comprise manipulation steps, i.e. treatments or interventions, which are applied *per se* or act via a Recovering Construct (RC).

The terms "external treatment" and "external manipulation" mean some artificial interventions or actions, which are different from those occuring naturally in the sites of crop growth or in the nature. Preferably they are used under confined conditions.

### THE GENERAL DESCRIPTION OF THE INVENTION

The present invention is related to molecular biology and provides a method and system for improved controlling of transgene segregation and spread into a natural population or into another transgenic or non-transgenic crop population in the environment. A complex of DNA constructs, herein called a Recoverable Block of Function (RBF) system in contrast to the unrecoverable blocking systems known from prior art, is the key issue of the present invention. The block comprises a nucleotide sequence capable of blocking certain vitally important, physiological or developmental functions of Sexually Reproducing plant, including plants and/or animals. The blocked function leads either to the extinction of the escaped transgene, i.e. the block is essential for the survival of the transgene. Alternatively, it leads to a significant change in the physiology of the plant or animal, resulting in the inability of the plant or animal to reproduce. The blocked function of the plant or animal can be molecular, biochemical or physiological in nature. Recovering of the blocked function comprises an external, artificial user-controlled intervention comprising a chemical, physical or mechanical treatment, which is performed with or without a DNA construct enabling the transgenic plant or animal to overcome the block of the function. One of the applications of the technology is to prevent the transgene from escaping through hybridization under natural conditions. The invention operates in the fields of molecular biology, transgenic technology, plant biochemistry and physiology, and also in specific fields of DNA synthesis and antisense technology.

Accordingly, the present invention is related to a molecular control system with an increased security level for the control of transgene expression or inadvertent escape of transgenes into the environment. The molecular control system acts through a Recoverable Block of Function (RBF) system claimed in the present invention, which implies the idea of linking Transgenes of Interest (TGI), into a vector or DNA cassette comprising one or more DNA construct(s) capable of blocking a particular function in a plant or an animal. The block leads to the extinction or death of the plant or animal or arrests the development or alters the phenotype or physiology of the plant/animal under natural conditions in such a way that sexual reproduction is prevented. The block of function is recoverable by the action of a recovering tool, which comprises an external user-controlled treatment and optionally includes at least one DNA construct encoding for a mRNA, a protein or an enzymatic product inducible by an external chemical or physical stimulus or expressing constitutively. Therefore, every Sexually Reproducing plant including the parental line or any subsequent hybrid thereof carrying the Transgene of Interest (TGI) linked to a Recoverable Block of Function (RBF) system will die or remain sterile under natural conditions when no external user-controlled intervention is applied, which occurs for example in case of inadvertent escape or out-crossing. Plants or animals provided with a Recoverable Block of Function (RBF) system can be repaired, i.e. the block can be removed, by a particular external procedure at a certain stage of the development.

The Recoverable Block of Function (RBF) system is not only a particular molecular construct but in a broader sense a tool, which operates with three conceptual constructs: The Transgene of Interest (TGI), Blocking Constructs (BCs) and Recovering Constructs (RCs) and a user-controlled intervention.

The Transgene of Interest (TGI) is a DNA sequence, which is controlled with a Recoverable Block of Function (RBF) system. It confers to the host organism one or more traits useful in agriculture, industrial production, forestry or horticulture. The Blocking Constructs (BCs) and Recovering constructs (RCs) belong to the Recoverable Block of Function (RBF) system and serve to control the Transgene of Interest (TGI) in transgenic Sexually Reproducing plants.

The Blocking Construct (BC) blocks a particular physiological or molecular function and prevents hybridization or out-crossing of host plants or host animals carrying at least one Transgene of Interest (TGI) linked to the Blocking Construct (BC).

The Blocking Construct (BC) comprises a DNA construct linked to the Transgene of Interest (TGI) and encodes a factor capable of blocking a certain molecular or physiological function. This block leads to the death or alteration of the phenotype or physiology of the transgenic Sexually Reproducing plant resulting in incapacity of sexual reproduction. The Blocking Construct (BC) can act through DNA-recombinase, antisense mRNA, ribonuclease, toxin, hormone production, enzymatic action or some other molecular mechanisms. The block of a particular function may also include any alteration in the molecular machinery leading to some phenotypic or physiological changes preventing reproduction of the Sexually Reproducing plant under natural conditions. It may for example be early or late seed germination or flowering, incapability to form inflorescence or fruits, formation of dwarf phenotype or some other morphological change.

Preferred examples of molecular mechanisms acting in Blocking Constructs (BCs) are the expression of:
- an enzyme with unusual organ- or development-specificity;
- an antisense mRNA of an enzyme important in development or functioning of the Sexually Reproducing plant;
- of a toxin;
- an enzyme producing a toxic metabolite; or
- an enzyme recombining DNA, RNA or protein molecules in a way that is undesirable for Sexually Reproducing plant.

The nucleic acid sequence present in the Blocking Construct (BC) should preferably express an enzyme, which is characterized by destroying or overproducing a chemical substance, such as a metabolite with unusual organ- or development-specificity. Such enzymes are for example L-asparaginase or L-glutaminase. The Blocking Construct (BC) may also comprise a nucleic acid sequence, encoding an antisense mRNA of an enzyme, which is essential for the development and/or functioning of the host organism, nucleotide sequence encoding a nuclease or nucleic acid recombinase. A preferred example is a nucleotide sequence encoding ent-kaurene synthase A, which is important in development or fruitation (fruit formation) of the host plant.

The Blocking Construct (BC) may comprise a nucleotide sequence encoding a protease or a toxin, e.g. the NPK15 protein kinase or an enzyme producing a toxic substance or a metabolite e.g. an antibiotic. Alternatively, the Blocking Construct (BC) may comprise a nucleic acid sequence encoding a nuclease or nucleic acid recombinase. The nucleotide sequences used in the Blocking Constructs (BCs) are preferably synthetic and adapted to host preference or to intron preference using the intron of a suitable nucleotide sequence or gene encoding the desired product in a host organism. A preferred synthetic sequence in some embodiments of the present invention is the synthetic nucleotide sequence of *barnase* comprising SEQ ID NO:1:. It is adapted to host, in this case plant preference, whereas SEQ ID NO:3: is adapted to a plant gene intron preference.

Several useful constructs and/or techniques applicable for the Recoverable Block of Function (RBF) can also be developed based on the information reviewed below.

Antisense technique may be used to block a key function of the plant by expressing antisense mRNA of the particular enzyme responsible for the key function. Antisense mRNA may block the expression of a particular gene or genes at a certain stage of development. For example the blocking of the expression of ent-kaurene synthetase A of *Arabidopsis* leads to a dwarf phenotype of the plant (Sun and Kamiya, 1994, Plant Cell, 6: 1509-1518). Ent-kaurene is a key molecule in the biosynthetic pathway of gibberellins, which are important plant hormones. *Ent-kaurene synthase A* is encoded by a single copy gene and can be blocked with antisense technique. If the antisense mRNA molecule is expressed at the germination stage, the lack of gibberellin can be compensated with an external application of GA3 (Example 2).

Another example is antisense mRNA block of expression of *glutamine synthetase* in germinating seeds. The glutamine synthetase is a critical enzyme in all of the amino acid conversions in germinating seeds (Temple, et al., 1993, Mol. Gen. Genet. 236: 315-325).

Blocking the development of a Sexually Reproducing plant can be achieved by expression of some enzymes originating from bacteria; e.g. , L-asparaginase (Filpula, et al. , 1988, Nucl. Acid Res. 16: 10385) or L-glutaminase (Ramakrishnan and Joseph, 1996, Canadian J. Microbiol. 42: 316-325), which can block the nitrogen metabolism in a plant cell. The block of the biosynthesis of glutamine can be compensated with the external application of this amino acid (Example 1). The same technology can be applied in a range of other cases. Examples of applicable technologies are the blocking the synthesis of plant hormones, including blocking of auxin, cytokinin or ABA synthesis, blocking of production of amino acid(s) or blocking of metabolic pathways.

Several different types of unrecoverable blocks of function have been described by different groups in different patent applications and publications intended for providing control of escape of transgenic plants.

Many of the molecular mechanisms described earlier are applicable in the present invention. For example unrecoverable block of embryo development can be achieved in *Brassica napus* by expression of the modified exotoxin A of *Pseudomonas aeruginosa* under napin promoter (Koning et al. 1992; Plant Mol. Biol.,18: 247-258). Pollen sterility can be achieved using diphtheria toxin A chain expression under lat52 promoter. Toxin expression can be applied to achieve cell ablation in developing pollen (Twell, 1995, Protoplasma, 187: 144-154). The patent US 5,498,533 discloses the regulation of potato development by expression of sense and antisense constructs of the *calmodulin* gene. Expression of sense-oriented *calmodulin* gene increased shoot and tuber growth, whereas plants carrying antisense constructs exhibit decreased shoot and tuber growth. Accordingly, the expression of antisense *calmodulin* gene can be used as a factor blocking a physiological function.

There is a number of toxin or lethal genes, which are capable of blocking certain functions in the plant. Several nucleases can destroy the mRNA synthesis machinery. The Barnase and Ribonuclease A enzymes have been subjects of intensive studies. Barnase originating from *Bacillus amyloliquefaciens* (Hartley, 1989, Trends Biochem. Sci. 14: 450-454) is the most known lethal gene since Mariani, et al. (1990, Nature 347: 737-741) used it for engineering fertility control in transgenic plants. The superior trait of the Barnase system is its specific inhibitor Barstar, which effectively blocks the RNase action in plants (Mariani, et al., 1992, Nature, 357: 384-387). Thus, the lethal block of a function in plants achieved by the expression of *barnase* can be recovered by the expression of the *barstar* gene (WO 94/03619, WO 00/37660).

Several catalytic or cytolytic lethal proteins can block certain functions. Ribosomal inhibitor protein (RIP) from saporin 6 RIP gene (Barthelemy, et al. 1993, J. Biol. Chem. 268: 6541-6548; GenBank ID SOSAPG, accession No. X15655) of Saponaria officinalis directly interferes with the expression of proteins in a plant cell, without being toxic to other organisms. Widely reviewed in the art is the *Diphtheria* toxin A, which is another effective block. It has been used several times as a conditional lethal marker gene in plants (Czako and An, 1991, Plant Physiol. (Bethesda) 95:687-692; Nillson, et al., 1998, Plant J. 15: 799-804) because it leads to cell ablation (Van Der Geest, et al., 1996, Plant Physiol. (Rockville) 109: 1151-1158). Removal of the RIP or *Diphtheria* toxin A action is possible by an antisense technique; and there is also a report on effective inhibition of the *Diphtheria* toxin by mansonone-D (Madhusoodana, et al., 1998, J. Cell. Physiol. 176: 40-49.). Mansonone- D, a sesquiterpenoid orthonaphthoquinone inhibited the cytotoxicity of ricin, Modeccin, *Pseudomonas* toxin and *Diphtheria* toxin, can putatively be used for recovering the block effected by the expression of the toxins in plants. It was shown that the *Pseudomonas aeruginosa* exotoxin A can specifically arrest tobacco embryo development (Koning, et al. 1992, Plant Mol. Biol. 18: 247-258).

There are still further examples of genes potentially useful in the art. The patent US 6,022,720 discloses the Bax protein that regulates programmed mammalian cell death. Harpin, the hrpN gene product of *Erwinia amylophlora* elicits HR in tobacco and is blocked by treatment with K52a, a protein kinase inhibitor (Popham, et al. 1995, Physiol. Mol. Plant Pathology, 47:39-50). NPK15, a tobacco protein-serine/threonine kinase acts as a suicide gene and blocks the proliferation of the host cells (Ito, et al. 1994, Mol. Gen. Genet. 245: 1-10). Genes from T-DNA of *Agrobacteria* responsible for overproduction of cytokinin or auxin have also been used for alteration of plant phenotype, i.e. *ipt* (Redig, et al., 1997, Physiol. Plantarum, 99: 89-96) or *aux1* and *aux2* gene (Beclin, et al. 1993, Transgenic Res. 2: 48-55; Hamza, et al. 1993, Theor. Appl. Gen. 86: 657-664). The genes mentioned above are used as lethal markers or suicidal genes, but recovering the blocks of function has not been described.

In the present invention, several of the genes may be used for preparing Transgenic Inserts (TIs) for assembling the Recoverable Block of Function (RBF) systems, when recovering of the block is carried out by an antisense technique or expression of the Blocking Construct (BC) gene under a promoter repressible with the protein encoded by the recovering gene. These techniques are all available to one skilled in the art. The nucleotide sequences prepared for use in the Blocking Constructs (BCs) are preferably synthetic and adapted to host preference, e.g. plant preference and/or for intron preference of a suitable, sufficiently large intron present in the in a Transgene of Interest (TGI) encoding the desired product

The Recovering Construct (RC), which is optional depending upon the Recoverable Block of Function (RBF) system used in the present invention, recovers the blocked function in case an external treatment is applied and does not work under natural conditions.

The Recovering Construct (RC) comprises a nucleotide sequence providing an activating or a silencing mechanism of the expression of the gene or nucleotide sequence in the Recovering Construct (RC). The nucleotide sequences are capable of expressing e.g. an antisense RNA of the blocking nucleotide sequence. The nucleotide sequence of the Recovering Construct (RC) can for example be an antisense RNA, which is substantially similar to the NPK15 gene, or a nucleotide sequence capable of expressing a protein which can inactivate the protein expressed by the Blocking Construct (BC). In a typical example, the inactivating protein is a Barstar protein, which by binding to the Barnase nuclease inactivates the same.

The Recovering Construct (RC) may comprise a nucleotide sequence capable of expressing an enzyme producing a metabolite capable of compensating the deficiency resulted by the action of the Blocking Construct (BC). Alternatively, the Recovering Construct (RC) may comprise a nucleotide sequence capable of expressing an enzyme, which converts a toxic substance or metabolite produced by the Blocking Construct (BC) into a non-hazardous product. Such an example, is provided by the nucleotide sequence encoding a phosphate transferase capable of converting an antibiotic substance, such as hygromycin.

In addition to the optional Recovering Construct (RC) the means for recovering or the recovering tool comprises a user-controlled intervention, using for example an external compensation with a chemical substance, for example a metabolite, repression of the promoter of the blocking construct capable of silencing the blocking gene mRNA expression by antisense RNA technique, expression of a protein capable of inactivating the Blocking Construct (BC) protein by specific binding, proteolysis or other protein-protein interactions, expression of an enzyme capable of converting a toxic substance into a non-hazardous one, expression of the substance or metabolite capable of compensating or inactivating or interacting with a blocking toxic substance, metabolite, as well as some other molecular mechanisms, which may be externally regulated.

The recovering factor or means removing the block of function can be an external user-controlled intervention, including chemical, physical or mechanical means. Useful chemical means are for example amino acids, hormones, salicylic acid, tetracycline, etc. Physical means or factors include temperature, light, osmosis, gravitation, humidity. The mechanical means include for example interline breeding or support of homozygous conditions.

The recovering factor or the means with an unblocking function applicable under user-control may include externally, regulated transgenic sequences, such as receptors, antisense RNAs, boundaries, repressors or proteolytic proteins, including both enzymes or their products. The external control can be a stimulus activating a responsive promoter or an interline crossing to support homozygous condition of the Transgenes of Interest (TGIs) in case of Segregating Recoverable Block of Function (S-RBF) system.

Recovering of the blocked function can be performed in a variety of ways and depends on the particular model of the Recoverable Block of Function (RBF) system. For convenience the tools or means are divided based on the type of recovering in several groups as follows: external compensation of the blocked function, recovering by the repression of the action of a Blocking Construct (BC) at the DNA level, recovering with antisense RNA technique, recovering on the protein-protein interaction level and on the enzymatic or an other metabolite level.

The external user-controlled means preferably include the following interventions:
- inducing externally a responsive recovering gene/protein;
- expressing constitutively the nucleotide sequence of the recovering construct.
- applying externally a substance or metabolite to compensate deficiency affected by Blocking Construct (BC);
- supporting homozygous condition of Recovering construct (RC), Blocking Construct (BC) and Transgene of Interest (TGI) using intraline crossing;
- inducing Recovering Construct (RC) by applying chemical activating inducible promoter of Recovering Construct (RC); and/or
- inducing Recovering Construct (RC) by applying physical factor (heat, cold, osmotic etc.) activating inducible promoter of Recovering Construct (RC).

The means for recovering the block include applying externally a substance, metabolite or hormone to compensate deficiency affected by Blocking Construct (BC). Such substances are for example gibberellins which can be given externally to plants or antisense mRNA of ent-kaurene synthase A. Expression of a repressor peptide or a protein binding the operon sequence, which controls the expression of the blocking nucleotide sequence can also be used as means for recovering the blocked function. Examples of useful repressors and operators are the Tn10 *tet* repressor protein and the tet-operator DNA sequence. The expression of antisense RNA of the blocking nucleotide sequence can be used for activating a silencing mechanism of the Blocking Construct (BC) expression. The expression comprises for example the expression of antisense RNA of the *NPKl5* gene. The expression of a protein inactivating the protein expressed by a Blocking Construct (BC) is further applicable as a means for recovering the blocked function. The protein inactivating the protein expressed by the Blocking Construct (BC) is for example the Barstar protein which inactivates the Barnase nuclease by binding thereto. The expression of an enzyme producing a metabolite capable of compensating the deficiency caused by the action of the Blocking Construct (BC) is a useful means for recovering. The expression of an enzyme capable of converting a toxic substance or a metabolite produced by the Blocking Construct (BC) into a non-hazardous product is a possible means for recovering. A useful example is provided by an enzyme, which converts the properties of an antibiotic. As an example a phosphate transferase of the antibiotic hygromycin may be mentioned.

External compensation of the blocked function may be used in the case of Recoverable Block of Function (RBF) leading to the lack of a substance, metabolite or hormone. Lack of the substance or molecule can be compensated with external addition of the same or an analogous molecule e.g. with blocking of ent-kaurene synthetase expression in germinating seeds (Example 2). Expression of L-asparaginase or L-glutaminase leads to lack of asparagine/glutamine (Temple, et al., 1993, Mol. Gen. Genet. 236: 315-325) but can be compensated with external addition of glutamine during the germination of seeds (Example 1).

Recovering by repression of action of the Blocking Construct (BC) at the DNA level can be applied to any blocking gene. The repression of the expression is effected with a particular DNA sequence having a boundary repressor element. A repressor element can be a protein, RNA or DNA. It can be any DNA sequence, the expression of which prevents the Blocking Construct (BC) expression. The repressor molecule should be expressed from the Recovering Construct (RC) placed under the control of an inducible promoter. For example, the known Tn10 tet repressor protein (Gatz and Quail, 1988, Proc. Natl. Acad. Sci. USA, 85: 1394-1397; Gatz, et al., 1991, Mol. Gen. Genet. 227: 229-237) can serve as a recovering element in the case the tet operon DNA sequence responsible for binding to the repressor protein is situated inside the promoter of the Blocking Construct (BC) (Example 7). The Recovering Construct (RC) expressing the repressor protein can be integrated in another non-allelic chromosome and serve as a Segregating Recoverable Block of Function (S-RBF) system (Example 7).

Recovering with antisense RNA technique applies to most of the reported conditional lethal marker genes acting as a Recoverable Block of Function (RBF) system recoverable by antisense technique. Expressed minus strand mRNA of the blocking gene launches a silencing mechanism, which saves the host Sexually Reproducing plant from the block of a certain function.

If the toxin protein has an inhibitor protein or enzymatically produced inhibitor metabolite, the recovering is provided at the protein level. The inhibitor can be a protein binding to the toxin or inactivating it proteolytically. The most known example of this interaction is Barnase-Barstar mechanism (Examples 3-7). The inhibitor, according to this invention, may be some enzymatically produced molecule being capable of inactivating the toxin impact. For example, if the blocking factor is overproduction of a substance or metabolite, recovering can be an analogues molecule, which is able to block the enzyme overproducing the substance or metabolite.

If the toxin is a substance or metabolite produced by the enzyme encoded with the blocking gene, the inhibitor can be an enzyme inactivating the toxic substance. For instance, the blocking could be the expression of an antibiotic, while the inhibition of its action recovering the block could be achieved, for example, by the action of a specific phosphate transferase of this antibiotic.

The means for recovering the blocked function are provided by external application of a substance in order to compensate a deficiency affected by blocking construct. A preferred embodiment is providing gibberellins to DNA constructs expressing antisense mRNA of *ent-kaurene synthase A* or by expression of repressor peptide/protein binding the operon sequence, which controls the blocking gene expression are for example a Tn10 tet repressor protein and tet-operator DNA sequence.

The interrelation of the three conceptual constructs of the present invention, i.e. the Transgene of interest (TGI), the Blocking construct (BC) and the Recovering construct (RC) is very important in order to provide the increased security level of control, which is the main objective of the present invention.

The Blocking Construct (BC) and the Transgene of Interest (TGI) should be closely linked together. Because the Blocking Construct (BC) controls the escape of the Transgene of Interest (TGI), the constructs should be linked together as close as possible to minimize or totally eliminate the possibility of crossing-over. The construct should be situated, at least, in the same Transgenic Insert (TI) in the same individual organism, preferably in the intron of the Transgene of Interest (TGI). If more than one Blocking Constructs (BCs) is used, they should be placed immediately on each side of the Transgenic Insert (TI) comprising Transgenes of Interest (TGIs) placed between two Blocking constructs (BC). They should be extremely as closely linked as possible.

In order to minimize the possibility of separation of the Blocking Construct (BC) and the Transgene of Interest (TGI), the blocking gene can be inserted in the intron of the Transgene of Interest (TGI). This has been described in Example 5. In this case, large mutations of the Blocking Construct (BC) are almost impossible without simultaneously destroying the Transgene of Interest (TGI). Insertion of the *barnase* gene (Blocking Construct (BC)) into the intron of the Transgene of Interest (TGI) (*uidA* or GUS) in opposite orientation is shown in Figures 5 and 6. The promoter of the Blocking Construct (BC) coincides with the polyadenylation signal of the Transgene of Interest (TGI). The second exon of *uidA* gene coincides with the 3' UTR of *barnase.* The coding sequence of *barnase* is located in the intron of the *uidA* gene. Polyadenylation signals of *barnase* coincide, partially, with the intron and, partially, with the exon of the *uidA* gene. The intron-inserted Blocking Construct (BC) serves as means for increasing security of molecular control of transgene escape.

The DNA construct may comprise for example a synthetic nucleotide sequence adapted to be inserted into the Blocking Construct (BC) inserted into intron of the Transgene of Interest (TGI). The intron of the Transgene of Interest (TGI) is for example adapted for the nucleotide sequence of the Blocking Construct (BC), which is placed into the intron in opposite orientation to the nucleotide sequence of interest. An example of this is provided when inserting a synthetic nucleotide sequence substantially identical to the *barnase* gene sequence and having the SEQ ID NO:3:. A preferred example of a complex DNA construct used in the present invention is a synthetic sequence of *barnase* and *barstar* genes which are adapted for plant expression, SEQ ID NO:1: and SEQ ID NO :2:, respectively. Said complex DNA construct is further characterized by having a synthetic polycloning site, shown in SEQ ID NO :4: designed with an open reading frame (ORF) adapted for β-galactosidase expression in a pUC19 cloning vector.

In the preferred embodiments of the present invention, the Double Recoverable Block of Function (D-RBF) and Triple Recoverable Block of Function (T-RBF), the Transgenic Insert (TI) comprising one or more Transgenes of Interest (TGIs) are placed between two Blocking Constructs (BCs) provided with a polycloning site for convenient introduction of different Transgenes of Interest (TGIs) for insertion into the appropriate recipient cells.

The Blocking Construct (BC) or the factor causing the blocking may comprise any nucleotide sequence encoding for a receptor, antisense RNA, nuclease, toxin, enzyme or other product. The blocking nucleotide sequence may in unique situations when the blocking gene has a industrially useful property be the same as the Transgene of Interest (TGI) forming a self-supporting entity. Usually the Blocking Construct (BC) and Transgenes of Interest (TGIs) are not the same.

The Recovering Construct (RC) is preferably placed in the same Sexually Reproducing plant and not in a different individual organism as in some prior art embodiments.

The Recovering Construct (RC) and the Blocking Construct (BC) can be located close together or in trans-position, even in different chromosomes depending on type of the Recoverable Block of Function (RBF). When the recovering of the Recoverable Block of Function (RBF) comprises external compensation of the lack of a particular metabolite, there is no need for a Recovering Construct (RC). In a Segregating Recoverable Block of Function (RBF) system, the Recovering Construct (RC) should be in a different non-allelic chromosome from the one carrying the Blocking Construct (BC).

Expression of the Blocking Construct (BC) can be either constitutive or temporal with organ or development specificity. Expression of the Recovering Construct (RC) can be responsive to an outside stimulus or be constitutively expressed as in a Segregating Recoverable Block of Function (S-RBF) system.

Constitutive expression of the Blocking Construct (BC) leads to the highest control of the transgene expression. The recovering mechanism is dependent on external treatment or stimulus. If the block involves the lack of a substance, metabolite or hormone, recovering of such a constitutively expressed block can be done by external compensation of the deficiency of the compound (Example 1). If the recovering comprises the expression of a product under an inducible promoter, the induction of the promoter also should be continuous. It means that an artificial recovering factor should be applied during the entire life cycle of the transgenic plant. Immediately after stopping the treatment, the plant will die because of the action of the Blocking Construct (BC).

Expression of the blocking factor can be constitutive or spatiotemporal, i.e. organ or development specific. A constitutive expression of the recovering factor may be used in a Segregating Recoverable Block of Function (S-RBF) system. Expression of the recovering factor may also be inducible by a certain stimulus depending on the mechanism of the particular Recoverable Block of Function (RBF) system used.

The Blocking Construct (BC) may express in the host organism constitutively, or in an organ-, development-specific or spatiotemporal manner, and the recovering occurs by external compensation of the blocking affected deficiency or by outside induction of a responsive recovering gene/protein or a constitutive or temporal expression of the recovering gene.

Constitutive, as well as development- or organ-specific expression of both the Blocking Construct (BC) and the Recovering Construct (RC) located in different non-allelic chromosomes, results in a Segregating Recoverable Block of Function (S-RBF), comprising the most convenient control of the transgene expression (Examples 5-7). The most obvious way to construct this control is by multiple, i.e. double or triple transformation. First, the constitutively expressing Recovering Construct (RC) is transformed to the Sexually Reproducing plant. Secondly, the construct containing the Transgene of interest (TGI) linked to the gene blocking a certain function of the plant is transformed in another non-allelic chromosome.

Transformants or their hybrids with non-transgenic plants are verified to have a single copy of the gene and that the constructs are on different chromosomes. Homozygotes for both of the constructs are grown in the field. If the transgenic Sexually Reproducing plant hybridizes with any other Sexually Reproducing plant, the first hybrid progeny will survive, because all of the plants will be heterozygous for both of the genes of the Recoverable Block of Function (RBF).

Beginning from the second hybrid generation, every second hybrid plant carrying the blocking gene will die, because the recovering and the blocking genes of Recoverable Block of Function (RBF) will segregate in different cells during meiosis. Thus, recovering of Segregating Recoverable Block of Function (S-RBF) comprises the support of interline crossing of the Sexually Reproducing plant (WO 00/37660).

The Blocking Construct (BC) starts to work only in the second hybrid progeny, and leads to 50 % negative selection of the transgene construct.

Promoters applicable for constitutive expression are 35S from CaMV (Condit, et al., 1983, J. Mol. Appl. Gen. (USA), 2: 301-314; Zaitlin, et al., 1985, Biotechnology in plant science: relevance to agriculture in the eighties. Orlando, Fla. (USA). Academic press, 227-235; Williamson, et al., 1989, Plant Physiol., 90: 1570-1576), NOS (Depicker, et al., 1982, J. Mol. Appl. Gen. (USA), 1: 561-573), OCS (De Greve, et al., 1985 J. Mol. Appl. Gen. (USA), 1: 499-511) and others.

Promoters for organ- or development-specific expression can be obtained from a very wide group. For Segregating Recoverable Block of Function (S-RBF), it does not matter, if the promoter is "leaky" or not. It is important that both the Blocking and the Recovering Constructs (BCs and RCs) are expressed under the same kind of a promoter resulting in the expression of the constructs concurrently in the same tissues of the host plant.

Organ, development and induction specific expression of the Recoverable Block of Function (RBF) genes leads to temporal control of critical stages in the life cycle of the transgenic plants and an external stimulus is needed to recover the block of a particular function. The developmental stages for temporal expression can be seed germination, flowering, embryo or fruit maturation, inflorescence formation, stem or root elongation and so on. Expression of the Blocking Construct (BC) only at a particular stage of transgenic plant development makes the recovering procedure shorter and easier than in the case of constitutive expression (Example 3).

While expression of the blocking gene should be regulated by an organ- or development-specific promoter, expression of the recovering gene should be regulated by an inducible promoter, especially if the recovering is not based on external compensation of the lack of a particular metabolite. Thus, a crop plant carrying temporally expressed Recoverable Block of Function (RBF) needs artificial recovering only at the particular stage of development or in a particular organ. Without recovering, the plant will die or have an altered phenotype during that particular stage of development under natural conditions.

An important trait of the organ or development specific promoter is its high specificity excluding Segregating Recoverable Block of Function (S-RBF). In other words, the promoter should be correctly expressing and not a "leaky" promoter, because the Blocking Construct can kill or disturb plant development if expressed at undesirable stages.

Constitutive repression of the Blocking Construct (BC) under natural conditions is a possible way to organize the Recoverable Block of Function (RBF). If the Blocking Construct (BC) is expressed at a particular stage of Sexually Reproducing plant development under a specific promoter, the Recovering Construct (RC) is under the control of a repressible promoter, e.g., modified 35Sp of CaMV or NOSp with Tn10 tet repressor (Gatz and Quail, 1988, Proc. Natl. Acad. Sci. USA, 85: 1394-1397; Gatz, et al., 1991, Mol. Gen. Genet. 227: 229-237). The promoter is repressed through the life cycle with a protein expressed from a third gene situated under the constitutive promoter (Example 4). Promoter of the recovering element can be launched only when an outside stimulus (chemical or physical) removes the repressor element. In the case of the *tet* repressor, the binding protein changes its own conformation and activates the expression of the repressed construct in the presence of tetracycline. The recovering, in this case, is tetracycline treatment of the transgenic plant during expression of the Blocking Construct (BC). The other possible scenario is expression of all the three genes under the same organ-specific promoter. In this case, the Tn10 *tet* operon should be introduced in the promoter of the recovering gene. There are also other candidate repressible promoters (Lanzer, et al., 1988, Proc. Natl. Acad. Sci. USA, 85: 8973-8977).

In order to develop or construct a Recoverable Block of Function (RBF) system with temporal expression, organ or development specific promoters should be explored for possible expression of the Blocking Construct (BC). Several promoters show high seed germination specificity in expression. Unfortunately each promoter has also some additional 'star' expression at other stages of development. SH-EP or EP-C1 cysteine endopeptidases specifically degrade storage proteins. They are proteins highly expressed in germinated seeds. Additional expression can be observed in senescent tissues: cotyledons, pods and even in stems. These genes have large promoter regions of about 1200-1700 bp. EP-C 1 cysteine endopeptidase has been cloned from *Phaseolus vulgaris* (Ogushi et al., 1992, Plant Mol. Biol. 19: 705-706) and expressed in a deletion analysis series in transgenic tobacco seedlings (Yamauchi et al., 1996, Plant Mol. Biol. 30: 321-329). Expression in matured pods makes the use of this promoter problematic for expression of the Blocking Construct (BC) in germinating seeds.

SH-EP promoter of sulfhydril endopeptidase (1676 bp.) has been cloned from *Vigna mungo* (Akasofu et al., 1990, Nucl. Acid Res. 18: 1892) and investigated as SH-EP-cysteine endopeptidase (Yamauchi et al., 1996, Plant Mol. Biol. 30: 321-329). It expresses exclusively in the germinating seedlings. Only low additional expression has been detected in matured pods. Expression starts on the second day after germination is onset. The peak of the expression in germinating seeds occurs on the third day at the mRNA level and on the fourth day on protein level. Enzymatic activity continues until fifth to sixth day and then fades. A cloned gene construct with its own promoter produces in transgenic tobacco even more protein than in *Vigna mungo.* Action of the promoter in the tobacco embryo was also found. Therefore, according to the present invention, this promoter can be used especially in a Double Recoverable Block of Function (D-RBF) system during seed development and seed germination.

LEA promoters express at late embryo development stages (Hughes and Galau, 1989; Galau, et al., 1992, Plant Physiol. 99: 783-788; Devic, et al., 1996, Plant J. 9: 205-2015). LEA promoters are not "leaky" and are highly specific to late embryogenesis and consequently useful promoters for temporal expression of RBF.

A promoter of the gene encoding for caffeic acid O-methyltransferase (COMT) enzyme expresses a protein in an extremely specific manner in the stem tissue of a perennial ryegrass *Lolium perenne* (McAlister, et al., 1998, Australian J. Plant Physiol. 25: 225-235). Expression of a Blocking Construct (BC) under this kind of promoter can prevent inflorescence stem formation and, thus, propagation of the grass plants under natural conditions.

A 620 bp promoter fragment from *MT1-A* (metallothionein-like) gene is sufficient to direct expression in transformed cotton roots (Hudspeth, et al., 1996, Plant Mol. Biol. 31: 701-705). According to the present invention, it can be used for blocking root development.

Examples of useful promoters are malate synthase (MS) and isocitrate liase (ICL) promoters which are active during germination and in seedlings are suppressed by sucrose and activated by GA3. Additional expression is known to occur in senescing organs and in germinating pollen. Spatial and temporal expression under promoters of cucumber (Reynolds and Smith, 1995, Plant Mol. Biol. 27: 487-497), oilseed rape (Zhang et al., 1994, Plant Physiol. 104: 875-864) and tomato (Janssen, 1995, Plant Physiol. 108: 1339) have been thoroughly studied in transgenic tobacco and oilseed rape. Malate synthase (MS) has a wider expression spectrum but is less ideal for germination specific expression (Sarah et al., 1996, Mol. Gen. Genet. 250: 153-161). The isocitrate liase (ICL) and malate synthase (MS) promoters are active in pollen and are useful in Double Recoverable Block of Function (D-RBF) systems. MS and ICL promoters of *B. napus* have an average 1 % activity in pollen in comparison with expression in seedlings (Zhang et al., 1994, Plant Physiol. 104: 857-864). These promoters express weakly in senescing organs such as cotyledons (Graham et al., 1992, Plant Cell 4: 349-357). They can be activated in the dark and suppressed by sucrose, glucose and other sugars (Graham et al., 1994, Plant Cell 6: 761-772). Expression of isocitrate liase (ICL) in mature seeds can achieve significant expression levels (- 5 -10 %) in seedlings (Turley and Trelease, 1990, Plant Mol. Biol. 14: 137-146). MS and ICL promoters have been thoroughly investigated as cis-acting elements (Sarah et al. 1996, Mol. Gen. Genet. 250: 153-161).

A 17 bp fragment responsive to gibberellin from a promoter of catepsin B -like protein of wheat (Cejudo et al., 1992, Plant Mol. Biol. 20: 849-856) has been reported. AMY (high P1) -alpha-amylase is active in the endosperm during germination. All the known amylase promoters are also active in different organs of *Phaseolus vulgaris* and *Vigna mungo* plants (Minamikava et al., 1992, Plant Cell Physiol. 33: 253-258). There is a known GA3 induced P1 amylase from barley (Rahmatullah et al., 1989, Plant mol. Biol. 12: 119-121). Beta-1,3 glucanase is active in the endosperm during germination. This is an antifungal protein induced by GA4. It is active also in leaves and other organs, and is wound inducible (Vogeli-Lange et al., 1994, Plant J. 5: 273-278). There is a number of other candidate promoters for temporal expression and the number increases every year.

Expression of the gene responsible for recovering the action of the Blocking Construct (BC) requires promoters responsive to outside stimulus. The outside stimulus can be chemical or physical. Chemical stimulus can be any molecule capable of regulating the activity of a particular promoter. Physical stimulus can be temperature, osmosis, light, gravitation or something else.

The following two types of promoters serve as examples of responses to outside stimulus: salicylate and heat shock inducible promoters. Salicylate inducible promoters are involved in virus or other pathogen and stress responses. Promoter of the pathogenesis-related PAla protein gene has been investigated in 5' deletion experiments in transgenic tobacco (Ohshima, et al., 1990, Plant Cell 2: 95-106). In another deletion experiment several regulatory elements have been found in a 902 bp PR-1a promoter (Van de Rhee, et al., 1990, Plan Mol. Biol. 21:451-461; Payne, et al., 1988, Plant Mol. Biol. 11: 89-94; Pfitzner, et al., 1988, Mol. Gen. Genet. 211: 290-295). Activity of the promoters was demonstrated to rise after 24-48 hours of salicylate induction and was close for induction effected by TMV infection. Heat shock promoters have been investigated in depth in different plants. Their activity was often several times higher than that of the 35S promoter. Induction of the HS promoters usually happened when ambient temperature rose to 35-45 °C (Ainley and Key, 1990, Plant Mol. Biol. 14: 949-967). A negative feature of the Heat shock promoters is their expression in seedlings and their activation also by physical stimulus other than heat.

Another useful promoter is the maize glutatione-S-transferase gene promoter (GSTII-27) which can be activated by herbicide Safeners R-25788 (WO 94/03619). Chemically controlled expression of a promoter has been reported in the patent US 5,880,333.

Tn10 tet repressor system (Gatz and Quail, 1988, Proc. Natl. Acad. Sci. USA, 85: 1394-1397) is activated by the chemical agent tetracycline, but its mechanism is opposite to a standard induction. This system requires two genes. First, the recovering gene under the promoter containing the tet operator. The second gene is positioned under a constitutive or organ and/or development responsive promoter gene encoding for the repressor protein binding tet operator DNA signal sequence. The system stays under repression until externally applied tetracycline activates the recovering gene.

The present invention is related to vectors, plasmids comprising one or more Transgenic Inserts (TIs) which may be assembled to form the complex of DNA constructs or Recoverable Block of Function (RBF) systems of the present invention and the application of said Recoverable Block of Function (RBF) systems for providing an increased security level in the control of segregation and gene escape, when transferred into Sexually Reproducing Multicellular plants and non-human animals. The vectors, plasmids and DNA cassettes can be transferred into cells of a Sexually Reproducing plant forming transformed transgenic plant and ultimately transgenic plants. The level of control of segregation and/or escape into the environment is increased by the blocking construct, which can be recovered and not only used, but also reused by the farmer or another ultimate consumer provided with appropriate instruction.

In the practical use, transgenic plants carrying the Recoverable Block of Function (RBF) system can be grown in the open field and do not differ from non-transgenic plants. Crop plants carrying Recoverable Block of Function (RBF) system require an external treatment to recover the blocked function usually at a particular stage of development.

The method is described in detail only for plant application, but is equally well applicable in non-human animals.

The above analysis enables the comparison of the candidate constructs and mechanisms on the range, reliability and convenience of the various Recoverable Block of Function (RBF) systems in controlling gene segregation and escape. The superiority of the Recoverable Block of Function (RBF) systems of the present invention rests in their ability to eliminate the Blocking Construct (BC) action with simultaneous action of the Transgene of interest (TGI). Occasionally, the block may fail to operate following crossing-over or mutations of the Blocking Construct (BC) or through certain silencing mechanisms. Crossing-over can separate the Blocking Construct (BC) and the controlled Transgene of Interest (TGI) in case the genotype of the host plants is heterozygous for those genes. The closer together the Blocking Construct (BC) and the controlled Transgenes of Interest (TGIs) are, the smaller is the possibility of separation of the constructs. Positioning of the Blocking Construct (BC) in the intron of the Transgene of Interest (TGI) substantially decreases the possibility of separation or mutation of the Blocking Construct (BC) without damage to the Transgene of Interest (TGI), because of coinciding of signal and coding sequences of both of the genes. Constitutive expression of the Blocking Construct (BC) can lead to a continuous pressure to break of the block. Therefore, it is preferable that the Blocking Construct (BC) is expressed under organ- or development-specific promoters. A Segregating Recoverable Block of Function (S-RBF) system may increase the possibility of the separation or separate repression of the Blocking Construct (BC) and the Transgene of Interest (TGI), because of several hybridizations with other plants, whereas two different Blocking Constructs (BCs) on both sides of the Transgenes of Interest (TGIs), i.e. Double Recoverable Block of Function (RBF) system drastically decreases the possibility of failure of the blocking. Use of these precautionary means eliminates the possibility of functional failure in the Blocking Constructs (BCs).

The Multiple or Triple Recoverable Block of Function (M-RBF or T-RBF) system combines all the best traits of Segregating, Double and Reversed Recoverable Block of Function (RBF) systems. It confers stronger negative selection than the Segregating (Single and Reversed) Recoverable Block of Function (SR-RBF) system. Triple Recoverable Block of Function (T-RBF) does not require special treatments besides intraline hybridization or crossing, like the Segregating Recoverable Block of Function (S-RBF) system. It prevents any transgene construct flow into environment, like the Segregating Reversed Recoverable Block of Function (SR-RBF) system. It is protected in a double manner and is therefore highly secured, like the Double Recoverable Block of Function (D-RBF) system. It is quite easy to construct because different nucleotide sequences needed for the Transgenic Inserts (TIs) used in assembling the Recoverable Block of Function (RBF) system are placed in three different Transgenic Inserts (TIs) which are placed in different non-allelic chromosomes. It enables the use of ready-made recipient Sexually Reproducing plant lines for cloning varied Transgenes of Interest (TGI).

To summarize the reviewed models, the most convenient, reliable and secured Recoverable Block of Function (RBF) system available at present is the Triple Recoverable Block of Function (T-RBF) system. As blocking genes, genes with different functions and expressed under different promoters may be used. They should be placed on both sides of the Transgene or Transgenes of Interest (TGI).

The construction and use of different Recoverable Block of Function (RBF) systems are described in the following examples, which are included for illustrative purposes only and should not be used to limit the claimed scope of the protection.

### EXAMPLE 1

### Continuous compensation of a metabolite eliminated by constitutively expressing the Blocking Construct (BC) of L-asparaginase/L-glutaminase enzyme;

### Recovery with externally applied L-asparagine/L-glutamine

The schematic positions of the genes are shown in Figure 1A. The Recoverable Block of Function (RBF) system is an example of how the system works at the metabolite level - in this case amino acid or nitrogen metabolism level. It is the most controlled Recoverable Block of Function (RBF) system. A block of the nitrogen metabolism leads to lethal effects resulting from the lack of amino acids, especially during seed germination. The L-asparaginase is active in sink tissues rich in asparagine. Expression of the enzyme in physiologically active tissues can inhibit the normal nitrogen metabolism. The Blocking Construct (BC) is placed close to the gene of interest or between genes of interest. The L-asparaginase from *Lupinus angustifolius* (Dickson, et al., 1992, Plant Mol. Biol. 20: 333-336), *Erwinia chrysanthemi* (Filpula, et al., 1988, Nucl. Acid Res. 16: 10385), *Arabidopsis thaliana* (Casado, et al., 1995, Plant Physiol. 108: 1321-1322) or other source can be expressed under NOS, OCT, 35S or other constitutive promoters. Constitutive expression of the enzyme makes the host plant continuously dependent on external asparagine supply. Such a tight control of the transgene can be required when the gene of interest confers real threat to humans or the environment. The molecular constructs are described in Figure 1A.

### EXAMPLE 2

### Antisense mRNA of ent-kaurene synthase A temporally expressed in germinating seeds of turnip rape

### Recovery with externally applied gibberellins

The schematic positions of the genes are shown in Figure 1B. The Recoverable Block of Function (RBF) system works at RNA silencing level with the recovery of the block working on hormone level. A mutation in a single copy gene encoding for *Ent-kaurene synthase A* leads to dwarf phenotype of *Arabidopsis thaliana.* Expression of the antisense RNA of the enzyme leads to the same phenotype alteration due to a block of the gibberellin synthesis in the turnip rape plant. This is a temporal block of function because the enzyme is situated under the SH-EP (EP cysteine endopeptidase) promoter from *Vigna mungo.* The expression of the antisense *ent-kaurene synthase A* starts during late embryogenesis and rises at the stage of seed germination. The host plant carrying the Recoverable Block of Function (RBF) develops a dwarf phenotype and is incapable of reproduction under natural conditions. Recovering of this block of function comprises external application of gibberellins. It has been shown that normal plants can be recovered from the dwarf phenotype of *Arabidopsis* by external application of gibberellins (Xu, et al., 1997, Plant Physiol. (Rockville) 114: 1471-1476; Phillips and Huttly, 1994, Plant Mol. Biol. 24: 603-615). In practice, seeds of the transgenic crop plants should be coated with a gibberellin containing mix in order to obtain plants with a normal phenotype. The molecular construct is shown in Figure 1B.

### EXAMPLE 3

### Recoverable Block of Function (RBF) with barnase controlled by SH-EP endopeptidase promoter;

### Recovery by barstar gene controlled by HS heat shock promoter

The schematic positions of the genes are shown in Figure 2. The Recoverable Block of Function (RBF) system works at the protein interaction level. It consists of *barnase* and *barstar* genes originating from *Bacillus amyloliquefaciens* (Hartley, 1989, Trends Biochem. Sci. 14: 450-454). *Barnase* is the best known lethal gene since Mariani, et al. (1990, Nature 347: 737-741) used it for engineering fertility control in transgenic plants. When expressed in the plant Barnase blocks the expression of all the genes through destroying RNA molecules. Acting as Blocking Construct (BC) the *barnase* gene was expressed under cysteine endopeptidase promoter (SH-EPp) cloned from *Vigna mungo* and, previously named sulfhydryl-endopeptidase promoter (Akasofu et al., 1990, Nucl. Acid Res. 18: 1892; Yamauchi et al., 1996, Plant Mol. Biol. 30: 321-329). The Recovering Construct (RC) comprises *barstar* gene placed under heat shock promoter (HSp) of soybean (Ainley and Key, 1990, Plant Mol. Biol. 14: 949-967). The synthetic *barnase* was placed under the SH-EP promoter and allowed to express during embryogenesis and seed germination in transformed tobacco plants. The schematic structure of the construct carrying four genes is shown in Figure 2. The DNA construct contains also *uidA* (GUS) as representative of the gene of interest and *hpt* gene as a selectable marker. The *uidA* gene was cloned under 35S promoter of CaMV and the *hpt* gene under NOS promoter of *Agrobacterium.* All four genes were situated in one T-DNA of pBIN19 (pGPTV-HPT) based vector (Bevan M., 1984, Nucl. Acid Res. 12: 871-8717; Frish et al., 1995, Plant Mol. Biol. 27: 405-409).

To explore expression of the HSp and SH-EPp, tobacco plants were transformed with the *uidA* gene driven under the promoters. Investigations of the transgenic tobacco plants showed that HS promoter expressed leaky GUS enzyme sometimes at the low level in generative organs. It demonstrated a slight peak of expression on the third day of germination and faded during the next few days without outside stimulation. It was activated in response to a high temperature in the range of +37-45 °C applied for 0.5-3 hours. For the HS promoter an activation treatment of one hour at +40 °C was used. The GUS expression under the HS promoter rose during the first two days and continued for at least 3-4 days after the heat shock. The SH-EP promoter expressed the GUS enzyme in the embryo and in the seedlings. The peak of GUS activity under SH-EPp was achieved at middle or late stages of embryo development and it reappeared on the third to fifth day of germination. There was no expression of SH-EP promoter in other organs of tobacco plants.

Several transgenic tobacco plants were recovered after transformation with the DNA construct containing the *uidA* gene of interest, *hpt* selectable marker gene and the Recoverable Block of Function (RBF) with *barnase* and *barstar* genes. The transgenic tobacco plants carrying the Recoverable Block of Function (RBF) construct demonstrated a normal phenotype. They grew, flowered and produced seedpods after self-pollination quite normally. The seeds from the plants were of normal size. However, they did not germinate. Heat shock application during germination does not recover the blocked germination function of the transgenic seeds. As predicted, the embryo development was arrested by the expression of Barnase at the time of pod maturation. Therefore, embryos of the seeds were, actually, dead.

All of the inflorescences were cut off from the tobacco plants, after which tobacco plants were allowed to form inflorescences again. The plants with new flowers and green seedpods were exposed to heat shock +40 °C for 1 hour every second day. Matured seedpods were collected from the tobacco plants. The seeds were germinated and heat shock was applied at least one time for one hour +40 °C on the second or third day of germination. The heat-treated seeds formed normal seedlings, which grew in normal tobacco plants. The seeds started to germinate also without heat shock. Most of them, however, could not expand their cotyledons. They exhibited an etiolated phenotype and died. Part of the seedlings, however, expanded cotyledons and overcame the block of development. Most evidently, the successfully germinated seeds have accumulated a substantial quantity of Barstar protein to inhibit action of Barnase expressed during seed germination. Only repeated heat shock treatment of seedpods-carrying plants removed the block of germination function of seeds. A one time heat shock treatment of 2 hours at +40 °C applied at different stages of embryogenesis from flowering to seedpod browning did not remove the blocked germination. Continuous heat shock treatments applied to tobacco plants with seedpods and flowers also had no success. The seedpods suffered from drought and seeds could not developed properly. While non-transgenic seed controls germinated after the continuous heat shock, the Recoverable Block of Function (RBF) carrying seeds did not germinate. The continuous heat shock may have dried the seedpods, and the HS promoter has not produced enough Barnase in time before the seedpods dried. While part of the F1 germinated transgenic seedlings was not positive in the GUS assay in respect of the transgene segregation, all of the seeds of the same transgenic line not treated with heat shock did not germinate. It can be explained by the fact that GUS expression driven by SH-EP promoter was registered at low level also in other parts of the seedpod. It means that Barnase could block mRNA synthesis also in the embryos, which did not carry the Recoverable Block of Function (RBF).

The action of the blocking and recovering genes was confirmed by Northern expression analysis (Figure 3). The *barnase* mRNA expression under a SH-EP promoter had a peak at the middle embryogenesis stage (whitish embryos) at the level 0.1 pg/1 *µ*g of total RNA and decreased to 0.03 pg/1 µg of total RNA in the late embryogenesis (yellowish embryos). The second peak of the expression started on the third day of seed germination, achieved 0.04 pg/1 µg of total RNA at the fourth day and disappeared on the fifth day of germination. The HS promoter demonstrated a leaky expression of *barstar* mRNA at two development stages, i.e. embryo and seed germination at a level of 0.05-0.2 pg/1 µg of total RNA. The high temperature treatment activated the *barstar* expression under HS promoter up to 1-3 pg/1 µg of total RNA. Especially, the HS promoter activity rose after several subsequent one time high temperature treatments for one to two days. Although, Northern analysis showed that HS promoter had an high leaky activity, *barstar* expression is not enough to inhibit *barnase* action without high temperature treatment, especially at the stage of embryogenesis. In general, if it is taken in consideration that protein expression often follows after the mRNA expression with delays of some hours up to one day, the results of Northern and GUS analysis of expression of SH-EP and HS promoters were comparable and the Recoverable Block of Function (RBF) construct worked as predicted.

In practice, recovering of the Recoverable Block of Function (RBF) system comprises application of a one hour heat shock treatment at +40 °C once at two subsequent days during seedpod development and on least one time during germination. Any other temperature treatments used could not remove the blocked seed germination. The Recoverable Block of Function (RBF) reliably controls the segregation and escape of the Transgene of Interest (TGI) in this case *uidA*. If the transgenic plants occasionally would be heated in the same way in nature, it would probably not be repeated in several subsequent growth seasons. The transgenic plants producing seed can be grown in greenhouse conditions, where heat shock is easy to perform. In the open field the transgenic plant carrying the Recoverable Block of Function (RBF) system produces seeds, which do not germinate. Pollen carrying the Recoverable Block of Function (RBF) can pollinate outside with related plants. The seeds produced after the pollination, however, do not germinate. Therefore, the Recoverable Block of Function (RBF) prevents transgene spread in natural environment.

### EXAMPLE 4

### Segregating Recoverable Block of Function (S-RBF) with Transgene of Interest (TGI) cry9Aa gene and Blocking Construct (BC) barnase gene in one insert, and Recovering Construct (RC) barstar gene in the other Transgenic Insert (TI).

The schematic positions of the genes are shown in Figure 4. The Recoverable Block of Function (RBF) system includes as a Blocking Construct (BC) the *barnase* gene driven under SH-EP endopeptidase promoter (SH-EPp) cloned from *Vigna mungo.* The *cry9Aa* gene was used in the construct as a Transgene of Interest (TGI). The plant transformation vector pVK112 was constructed on the basis of the pGPTV-HPT vector (Becker, D. et al. 1992. New plant binary vectors with selectable markers located proximal to the left T-DNA border. Plant Mol. Biol. 20, 1195-1197). The Recovering Construct (RC), *barstar* gene under control of 35Sp or HSp promoter was cloned in the pGPTV-KAN (Becker, D. et al., 1992. Plant Mol. Biol. 20, 1195-1197) and transformed into tobacco plants. Plants expressing *barstar* mRNA in expected manner were used for a second transformation by a pGPTV-HPT vector carrying a Blocking Construct (BC) and the insecticidal cry9Aa gene of interest (Kuvshinov V., et al., 2001, Plant Sci. 160: 341-353).

Plants carrying two inserts including HSp-barstar Recovering Construct (RC) exhibited the same traits as plants with one insert Recoverable Block of Function (RBF) described in the Example 3. Transgenic tobaccos with Recovering Construct (RC) regulated by 35Sp, exhibit a normal phenotype. Lethal genotype in seeds containing an insert with Blocking Construct (BC) and *cry9Aa* gene alone occurs during hybridization with wild type tobaccos after segregation according to Figure 8. Living seeds contain both (BC and RC) constructs or only one Recovering Construct (RC) or do not contain the transgene inserts. The negative selection against transgenic insertion containing Blocking Construct (BC) and cry9Aa gene is 50 % in each progeny.

### EXAMPLE 5

### Segregating Recoverable Block of Function (S-RBF), when the blocking gene, barnase, is located under an organ specific promoter in the intron of the Transgene of Interest (TGI) and regulated by organ specific (SH-EP) promoter.

### Recovering Construct (RC) is the barstar gene situated in a different non-allelic chromosome under the constitutive (35Sp) or heat shock inducible (HSp) promoter.

The schematic positions of the genes are shown in Figures 5a and 5b. The Recoverable Block of Function (RBF) operates at a protein-protein interaction level. The blocking gene *barnase* was situated in the intron of the gene of interest *uidA* (GUS) to prevent separation of the Blocking Construct (BC) and the Transgene of Interest (TGI) during crossing-over. The *barnase* gene is regulated by a SH-EP promoter from *Vigna mungo.* The recovering gene, *barstar,* is situated in a different non-allelic chromosome under the same promoter.

The tobacco plants carrying Recovering Construct (RC) with *barstar* gene driven under HSp or 35Sp (described in Example 4) were re-transformed by transgenic construct including GUS gene with intron-introduced *barnase* coding sequence (Figure 5). The transgenic plant did not differ from the non-transgenic one because of the continuous lack of expression of Barnase. Recovering of the transgenic plants is provided when the homozygous (BBRR) transgenic plants cross with each other and the homozygous genotype is thus supported in the progeny. When the transgenic plant crosses with an outside plant, the hybrid plant of the first generation also exhibits normal phenotype but carries the heterozygous (BbRr) genotype. Segregating Recoverable Block of Function (S-RBF) becomes effective from the second hybrid generation, when the Blocking and Recovering Constructs (BCs and RCs) will segregate into different host cells. Every second hybrid embryo containing a Blocking Construct (BC) will carry the Bbrr genotype leading to death of the plant during embryogenesis or germination, the stage depending on the plant species. Hereby, Blocking Construct (BC) effects strong negative selection during hybridization with outside plants. This negative selection will eliminate the transgene from the population during several generations. The number of the plants carrying the negative selection factor (X) will be reduced with the number of generations (n) in the following proportion: X=Y.2⁻ⁿ, where Y is the starting number of the plants carrying negative selection in a big population. For example, if the starting number of the hybrid plants would be Y = 1000, after n = 10 generations the number of plants X carrying Recoverable Block of Function (RBF) drops below 1, X = 1000. 2-10 or X < 1. Because the Blocking Construct (BC) is situated inside the Transgene of Interest, (TGI) crossing-over cannot separate the Transgene of Interest (TGI) and the Blocking Construct (BC) into allelic chromosomes. A construct with the *barstar* gene regulated by heat shock promoter combines the traits of segregating and inducible Recoverable Block of Function (RBF) systems. In order to survive and reproduce, the transgenic plants require heat shock treatment as described in Example 3.

The sequences of the intron and the polyadenylation signal of the Transgene of Interest (*uidA*) contemplated with the promoter, UTR, coding sequence and polyadenylation signal of the blocking gene *(barnase).* The signal sequences of the intron used in the construct: TA proportion 55-70%, 5' exon/intron boundary site AG/GUAUGU (SEQ ID NO:5:), branchpoint sequence (UA)CUAAC (SEQ ID NO:6:) located 20-50 nt upstream from the 3' intron/exon boundary site GCAG/G (SEQ ID NO:7:). U-rich sequences stimulate splicing regardless of their position within an intron. Single or double U-to-G substitutions strongly inhibit splicing. High content of AU and especially U is required in the first and last 50 nts of the intron. Exons are about 15 % richer in GC content than introns.

Termination of the transcription by RNA polymerase II requires the presence of a functional polyadenylation signal in transcription unit near the upstream element (NUE) AAUAAA (SEQ ID NO:8:), however, plants have many modifications of this site. The far upstream element (FUE) is even more unconserved. Common traits of FUEs are U- or UG-rich sequences situated 10-40 nts before NUE. The most often occurring is UUUGUA (SEQ ID NO:9:) but also UGUGUUUUUU (SEQ ID NO:10:) or UGUUGUG (SEQ ID NO:11:). The cleavage site is also not highly conserved. Usually it consists of Y A nucleotides and is positioned 10-30 nts after the NUE site.

In the promoter region, there are two distinct boxes: TATA in consensus sequence TCACTATATATAG (SEQ ID NO:12:) located 15-60 nucleotides from the start of the transcription and CAAT (SEQ ID NO:13:) or AGGA (SEQ ID NO:14:) box located 30-100 nucleotides upstream the TATA box. Translation starts 40-80 nt downstream from the start of the translation from the AUG codon in preferred sequence AACAA TGGCT (SEQ ID NO:12:) (nucleotides in bold are highly conserved). As an example, the sequence of *barnase* gene situated in the intron of *uidA* gene under the SH-EP promoter is shown in Figure 5a.

The transgenic tobacco plant containing the intron-inserted Blocking Construct (BC) as well as the control plants containing the GUS gene under the salicylate acid promoter both expressed GUS gene and were gave positive results in the GUS-assay. However, control plants containing truncated GUS-gene did not show any GUS-activity. This result suggest that the intron of the GUS-gene was correctly spliced.

### Example 6.

### Double Blocking Construct (BC) and one Recovering Construct (RC) placed into one transgenic insert with gene of interest (GUS) and separately in different non-allelic chromosomes.

The schematical positions of the genes are shown in the Figure 7. The Recoverable Block of Function (RBF) system includes as a first Blocking Construct (BC) the *barnase* gene driven under SH-EP endopeptidase promoter (SH-EPp) cloned from *Vigna mungo* and a second Blocking Construct (BC) comprising the same *barnase* gene driven under the *Cruciferin* promoter (CRUp) from *Brassica napus.* The transgenic insert contains only one Recovering Construct (RC) comprising *barnase* gene driven under Heat Shock promoter (HSp). The transgene insert carried also GUS gene as Transgene of Interest (TGI) and hygromycin phosphate transferase *(hpt)* as selectable marker.

The construct was developed on the vector used in the Example 3. The second Blocking Construct (BC) *barnase* under CRUp was introduced into the another side of Transgene of Interest (GUS) between GUS and *hpt* genes composing vector pVK30 (Figure 7A). Preliminary studies of CRUp expression with GUS gene have shown that the promoter expresses in the stage of embryo development in the same manner as SH-EPp. The construct from pVK30 was transformed into tobacco plants. GUS-positive transgenic tobacco plants were collected further investigations. In the experiments the double Recoverable Block of Function (D-RBF) acted as in the single Recoverable Block of Function (RBF) described in Example 3. The only difference was that possibility of inactivation of Double Recoverable Block of Function (D-RBF) in the result of mutations or promoter silencing is estimated approximately in 10⁶ times less than in the case of single Recoverable Block of Function (RBF).

The second approach was performed with the final construct in pVK30. The recovering *barstar* gene has been removed from the construct comprising pVK31 (Figure 7B). The double Blocking Construct (BC) without *barstar* gene was transformed into tobacco plants carrying the *barstar* gene (expressed under 35Sp or HSp promoters) that were described in the Examples 4 and 5. Transformed regenerated shoots were selected on hygromycin selection. Expression of *uidA* gene was detected in GUS assay. The both Blocking Constructs (BCs) expressed during embryo maturation. *Barstar* gene expressed constitutively under 35S promoter and was heat shock-inducible under HSp promoter.

One-insert and two-insert Recoverable Block of Function (RBF) constructs with a HSp-regulated barstar gene acts in the same manner as described in Example 3. In order to successfully germinate seeds, transgenic tobacco plants should be heat shock-treated at the stage of seedpod maturation.

In the case of 35Sp-regulated barstar gene, the Blocking Constructs (BCs) kill the embryos after separation of the Transgene of Interest (TGI) containing insert from the Recovering Construct (RC) containing insert as shown in Figure 8.

### EXAMPLE 7

### Triple Recoverable Block of Function (RBF)

### Two Recoverable Block of Function (RBF) systems: barnase/barstar and NPK15/tet repressor GUS gene as Transgene of Interest placed between barnase and NPK15 blocking systems

The schematic positions of the genes are shown in Figure 9. The first Recoverable Block of Function (RBF) consists of a blocking gene *NPK15* or optionally oncogene encoding Indole Acetamide Synthase (IAMS) as Van Onchelen et al., 1986, FEBS lett. 198, 357-360 (B₁) driven under a chimeric SH-EPp promoter with an inserted tet operon sequence, and a recovering gene (R₁) encoding Tet10 repressor protein and driven under 35S promoter from CaMV. The second Recoverable Block of Function (RBF) consists of a *barnase* blocking gene (B₂) driven under a CRUp promoter, and a recovering gene (R₂) encoding for *barstar* gene and driven under the 35Sp promoter. The transgenic plant carries three different transgenic constructs situated in different non-allelic chromosomes. The first construct contains a Transgene of interest (I) placed between two different Blocking Constructs (B₁ and B₂). The second construct contains the first Recovering Construct (R₁) linked to the second Blocking Construct (B₂). The third construct contains the second Recovering Construct (R₂) linked to the first Blocking Construct (B₁). The Blocking Constructs acts mainly at the stage of embryogenesis and germination. Figure 10 depicts recipient transgenic plant line and plant transformation vector with double Blocking Construct for cloning different Transgene of Interest (TGI). The segregation of the constructs during hybridization and interbreeding is shown and described in more detail in Figure 11.

## Claims

1. A method for providing an increased security level for controlling transgene segregation in a Sexually Reproducing plant and/or for providing automatic prevention of transgene escape, **characterized in that** the method comprises a molecular mechanism including the steps of
(a) constructing one or more Recoverable Block of Function (RBF) systems or complexes of DNA constructs for inserting into a recipient cell or cell-line in addition to a Transgenic Insert (TI) comprising one or more Transgenes of Interest (TGIs) encoding one or more desired gene products, one or more Blocking Constructs (BC), and providing at least one user-controlled means for recovering the blocked functions;
(i) said Blocking Construct (BC) having the capacity of blocking at least one molecular or physiological function essential for the survival or reproduction of the Sexually Reproducing plant, which Blocking Construct (BC) is located in the same chromosome as, and in the close proximity of the Transgenic Insert (TI) comprising one or more Transgenes of Interest (TGIs) preferably in an intron of a Transgene of Interest (TGI) or flanking each side of a Transgenic Insert (TI) comprising;
(ii) said means for recovering the blocked function comprising one or more user-controlled intervention optionally combined with one or more Recovering Constructs (RC), which are placed close to a Blocking Construct (BC) in a Transgenic Insert (TI) comprising one or more Transgenes of Interest (TGIs) and/or in non-allelic chromosomes; and
(b) recovering the blocked function in order to allow normal development during cultivation and reproduction of said Sexually Reproducing plant for production purposes by applying, at a susceptible stage of the developmental or reproductive cycle of said Sexually Reproducing plant, one or more user-controlled interventions;
with the proviso that when solely one Blocking Construct is used, said Blocking Construct (BC) or a part of it is placed in one intron of the only Transgene of Interest (TGI).

2. The method according to claim 1, **characterized in that** the Blocking Constructs (BCs) are flanking each side of a Transgenic Insert (TI) comprising one or more Transgenes of Interest (TGIs), when two or more Blocking Constructs (BCs) are used.

3. The method according to claim 1, **characterized in that** the Recoverable Block of Function (RBF) system is a Double Recoverable Block of Function (D-RBF) system comprising at least two Blocking Constructs (BCs), which are the same or different and which are recoverable by the same or different recovering mechanisms, flanking each side of the Transgenic Insert (TI) comprising one or more Transgenes of Interest (TGIs).

4. The method according to claim 1, **characterized in that** the Recoverable Block of Function (RBF) system is a Multiple, preferably a Triple Recoverable Block of Function (M-RBF, T-RBF) system comprising two, preferably three or more Transgenic Inserts (TIs) placed in three or more non-allelic chromosomes, in which the first Transgenic Insert (TI) comprises at least two different Blocking Constructs (BCs), the second Transgenic Insert (TI) comprises one or more first Recovering Constructs (RCs) linked to an optional Blocking Construct (BC) being the same or different from one of the Blocking Constructs (BCs) of the first Transgenic Insert (TI) and a third Transgenic Insert (TI) comprising one or more second Recovering Constructs (RCs) which are different from the first Recovering Constructs (RCs) and which are linked to a Blocking Construct (BC) corresponding to one of the first Transgenic Insert (TI) which is not the same as the optional Blocking Construct (BC) of the second Transgenic Insert (TI).

5. The method according to claim 1, **characterized in that** for obtaining a Multiple Recoverable Block of Function (M-RBF) system the recipient transgenic Sexually Reproducing plant is provided with two Transgenic Inserts (TIs) of a Reversed Segregating Recoverable Block of Function (RS-RBF) system in two non-allelic chromosomes without the Transgene of Interest (TGI) and a transformation vector comprising two Blocking Constructs (BCs) placed on each side of a cloning site for desired Transgenes of Interest (TGIs) and implying said vector to transform the recipient transgenic Sexually Reproducing plant line into a third non-allelic chromosome.

6. The method according to claim 1, **characterized in that** the Recoverable Block of Function (RBF) system is a Segregating Recoverable Block of Function (S-RBF) system comprising a Recovering Construct (RC) and a Blocking Construct (BC) placed in different non-allelic chromosomes, with the Blocking Construct (BC) in an intron of a Transgenic Insert (TI) comprising one or more Transgenes of Interest (TGIs).

7. The method according to claim 1, **characterized in that** an Induced Recoverable Block of Function (I-RBF) system comprises a Recovering Construct (RC) placed in the same chromosome as the Blocking Construct (BC) but expressed under a different promoter, with the Blocking Construct (BC) or part of it placed in an intron of a Transgenic Insert (TI) comprising one or more Transgenes of Interest (TGIs).

8. The method according to claim 1, **characterized in that** the expression of at least one nucleotide sequence in the Blocking Construct (BC) blocks in a constitutive, organ-specific or spatiotemporal manner a molecular or physiological function essential for the survival or reproduction of a transgenic Sexually plant.

9. The method according to claim 1, **characterized in that** the removal of the effect of the Blocking Construct (BC) is achieved by applying a user-controlled intervention, which at a susceptible stage in the development and/or reproductive cycle of the transgenic Sexually Reproducing plant upheaves or vacates the constitutive, organ-specifically or spatiotemporally achieved morphological or physiological block preventing hybridization and/or sexual reproduction of the transgenic Sexually Reproducing plant.

10. The method according to claim 9, **characterized in that** the susceptible stage essential for the survival and/or reproduction is selected from a group consisting of early or late seed germination, flowering, formation of dwarf phenotype, incapability of forming inflorescence flowers and/or fruits, incapability of germination, vegetation, rooting and photosynthesis.

11. The method according to claim 9, **characterized in that** the susceptible stage essential for the survival and/or reproduction is selected from a group consisting of early or late seed germination.

12. The method according to claim 1, **characterized in that** the removal of the effect of the blocked function comprises a user-controlled externally or internally regulatable molecular mechanism optionally applicable under confined conditions.

13. The method according to claim 12, **characterized in that** the user-controlled, externally and/or internally regulatable molecular mechanism for removal of the blocked effect is selected from a group consisting of
(a) adding at least one externally applicable substance;
(b) applying at least one external chemical or physical stimulus capable of activating the optional Recovering Construct (RC);
(c) repressing the promoter of the Blocking Construct (BC);
(d) silencing the mRNA expression of the nucleotide sequence of the Blocking Construct (BC) by antisense RNA technology;
(e) allowing the expression of a protein capable of inactivating the protein, which is encoded by the nucleotide sequence of the Blocking Construct (BC) by applying an externally activatable molecular mechanisms;
(f) allowing the expression of an enzyme producing a substance capable of inactivating the protein expressed by a nucleotide sequence of the Blocking Construct (BC),
(g) allowing the expression of an enzyme capable of inactivating a toxic substance produced by the enzyme;
(h) allowing the expression of an enzyme capable of producing a substance compensating the deficiency caused by action of the Blocking Construct (BC);
(i) allowing the expression of an enzyme capable of producing a substance altering the action caused by the Blocking Construct (BC);
(j) allowing specific nucleic acid and/or protein binding;
(k) applying proteolysis;
(l) allowing protein-protein interaction;
(m) expressing an enzyme capable of detoxifying a toxic substance one;
(n) expressing a substance capable of compensating or inactivating the toxic overproduced substance;
(o) expressing a substance capable of interacting with a toxic substance;
(p) providing an enzyme capable of converting a toxic or overproduced substance with wrong spatiotemporal specificity;
(q) compensating externally the deficiency caused by the Blocking Construct (BC);
(r) inducing externally a responsive recovering DNA, RNA, protein or metabolite;
(s) expressing constitutively the nucleotide sequence of the Recovering Construct (RC);
(t) expressing spatiotemporally the nucleotide sequence of the Recovering Construct (RC); and
(u) allowing intraline crossing when the Recoverable Block of Function (RBF) system is a Segregating Double or a Multiple Recoverable Block of Function (RBF) system.

14. The method according to claim 1, **characterized in that** the user-controlled, externally regulatable molecular mechanism for recovering the blocked function comprises allowing intraline crossing supporting homozygous conditions.

15. A complex of DNA constructs or a Recoverable Block of Function (RBF) system for providing an increased security level in the control transgene segregation in a Sexually Reproducing plant and/or for providing an automatic prevention of introgression **characterized in that** said complex of DNA constructs comprises
(a) a Transgenic Insert (TI) comprising at least one Transgene of Interest (TGI) encoding one or more desired gene products;
(b) one or more Blocking Constructs (BC), which are recoverable by user-controlled means for recovering, said Blocking Construct (BC) including at least one nucleotide sequence having the capacity of blocking at least one molecular or physiological function essential for the survival or reproduction of the Sexually Reproducing plant and being located in the same chromosome as and in the close proximity of the Transgenic Insert (TI) comprising one or more Transgenes of Interest (TGIs) preferably in an intron of a Transgene of Interest (TGI) or flanking each side of a Transgenic Insert (TI) comprising one or more Transgenes of Interest (TGIs);
(c) one or more optional Recovering Constructs (RC) which are placed close to one of the Blocking Constructs (BCs) in an Transgenic Insert (TI) comprising one or more Transgenes of Interest (TGIs) placed in one or more non-allelic chromosomes; with the proviso that when solely one Blocking Construct (BC) is used, said Blocking Construct (BC) or part of it is placed in an intron of the only Transgene of Interest (TGI).

16. The complex of DNA constructs according to claim 15, **characterized in that** the Blocking Constructs (BCs) are flanking on each side of the Transgenic Insert (TI) comprising one or more Transgenes of Interest (TGIs), when two or more Blocking Constructs (BCs) are used forming a self-controlling and self supporting entity.

17. The complex of DNA constructs according to claim 15, **characterized in that** the Recoverable Block of Function (RBF) system is a Double Recoverable Block of Function (D-RBF) system comprising Blocking Constructs (BCs), which are at least two and the same or different and situated at both sides of a Transgenic Insert (TI) comprising one or more Transgenes of Interest (TGIs) and recoverable by the same or different recovering mechanisms.

18. The complex of DNA constructs according to claim 15, **characterized in that** the Recoverable Block of Function (RBF) system is a Multiple Recoverable Block of Function (M-RBF) system comprising two, preferably three or more Transgenic Inserts (TIs) placed in three or more non-allelic chromosomes, in which the first Transgenic Insert (TI) comprises at least two different Blocking Constructs (BCs), the second Transgenic Insert (TI) comprises one or more first Recovering Constructs (RCs) linked to an optional Blocking Construct (BC) corresponding to one of the Blocking Constructs (BCs) of the first Transgenic Insert (TI) and the third Transgenic Insert (TI) comprising one or more second Recovering Constructs (RCs) which are the same or different from the first Recovering Constructs (RCs) and which are linked to a Blocking Construct (BC) being the same as one of the first Transgenic Insert (TI) which is not the same as the optional Blocking Construct (BC) of the second Transgenic Insert (TI).

19. The complex of DNA constructs according to claim 15, **characterized in that** a Multiple Recoverable Block of Function (M-RBF) system the recipient transgenic Sexually Reproducing Multicellular Organism (SRMO) is provided with two Transgenic Inserts (TIs) of a Reversed Segregating Recoverable Block of Function (RS-RBF) system in two non-allelic chromosomes without the Transgene of Interest (TGI) and a transformation vector comprising to Blocking Constructs (BCs) placed on each side of a cloning site for desired Transgenes of Interest (TGIs) and implying said vector to transform the recipient transgenic Sexually Reproducing plant line into a third non-allelic chromosome.

20. The complex of DNA constructs according to claim 15, **characterized in that** the Recoverable Block of Function (RBF) system is an Induced Recoverable Block of Function (I-RBF) system comprising a Recovering Construct (RC) placed in the same chromosome as the Blocking Construct (BC) with the Blocking Construct (BC) in an intron of the Transgenic Insert (TI) comprising one or more Transgenes of Interest (TGIs).

21. The complex of DNA constructs according to claim 15, **characterized in that** the Recoverable Block of Function (RBF) system is a Segregating Recoverable Block of Function (S-RBF) system comprising a Recovering Construct (RC) and a Blocking Construct (BC) placed in different non-allelic chromosomes with the Blocking Construct (BC) or part of it placed in an intron of the Transgenic Insert (TI) comprising one or more Transgenes of Interest (TGIs).

22. The complex of DNA constructs according to claim 15, **characterized in that** an inducible Recovering Construct (RC) is activatable in response to a user-controlled intervention, which recovers the function essential for the survival and sexual reproduction of transgenic Sexually Reproducing Multicellular Organism (SRMO) and which function is blocked by a Blocking Construct (BC) expressed under the same or a different type of promoter.

23. The complex of DNA constructs according to claim 15, **characterized in that** the two nucleotide sequences of the Blocking Construct (BC) forming a Double Recoverable Block of Function (D-RBF) system are recoverable by the same or different means for recovering.

24. The complex of DNA constructs according to claim 15, **characterized in that** an inducible Recovering Construct (RC) is activatable in response to a user-controlled intervention, which recovers the function blocked by the Blocking Construct (BC) expressed under the same or a different promoter.

25. The complex of DNA constructs according to claim 15, **characterized in that** the Blocking Construct (BC) comprises at least one nucleotide sequence, the constitutive, organ specific or spatiotemporal expression of which blocks an essential molecular or physiological function at DNA, mRNA, protein or metabolite level and/or causes a phenotypical, physiological or morphological alteration preventing free hybridization and/or reproduction of an out-crossing, transgenic Sexually Reproducing plant under natural, uncontrolled conditions leading to its extinction.

26. The complex of DNA constructs according to claim 15, **characterized in that** the function blocked by the Blocking Construct (BC) of the Recoverable Block of Function (RBF) system, is recovered by applying a user-controlled externally or internally regulatable molecular mechanism optionally applicable under confined conditions.

27. The complex of DNA constructs according to claim 26, **characterized in that** the user-controlled intervention for recovering the blocked function is selected from a group consisting of
(a) adding at least one externally applicable substance;
(b) applying at least one external chemical or physical stimulus capable of activating the optional Recovering Construct (RC);
(c) repressing the promoter of the Blocking Construct (BC);
(d) silencing the blocking gene mRNA expression by antisense RNA technique;
(e) allowing the expression of a protein capable of inactivating the Blocking Construct (BC) encoding protein by an externally activatable molecular mechanisms;
(f) allowing the expression of an enzyme capable of producing a substance inactivating the protein expressed by the Blocking Construct (BC);
(g) allowing the expression of an enzyme capable of inactivating a toxic substance produced by the enzyme;
(h) allowing the expression of an enzyme capable of producing a substance compensating the deficiency caused by the action of the Blocking Construct (BC);
(i) allowing the expression of an enzyme capable of producing a substance compensating the alteration caused by the action of the Blocking Construct (BC);
(j) allowing specific nucleic acid and/or protein binding;
(k) applying proteolysis;
(l) allowing protein-protein interaction;
(m) expressing an enzyme capable of detoxifying a toxic substance one;
(n) expressing a substance capable of compensating or inactivating the toxic overproduced substance;
(o) expressing a substance capable of interacting with a toxic substance;
(p) providing an enzyme producing a toxic or overproduced substance;
(q) compensating externally the deficiency caused by the Blocking Construct (BC);
(r) inducing externally a responsive recovering DNA, RNA, protein or metabolite;
(s) expressing constitutively the nucleotide sequence of the Recovering Construct (RC);
(t) expressing spatiotemporally the nucleotide sequence of the Recovering Construct (RC); and
(u) allowing intraline crossing when the Recoverable Block of Function (RBF) system is a multiple Recoverable Block of Function (RBF) system.

28. The complex of DNA constructs according to claim 15, **characterized in that** the user-controlled externally regulatable molecular mechanism for recovering the blocked function comprises allowing intraline crossing.

29. The complex of DNA constructs according to claim 15, **characterized in that** the Blocking Construct (BC) comprises a nucleotide sequence expressing in the host organism in a constitutive, organ-specific or spatiotemporal manner and the Recovering Construct (RC), comprises a nucleotide sequence capable of being recovered by a user-controlled intervention regulating a treatment-responsive nucleotide sequence or its gene product or by constitutive expression of the nucleotide sequence in the Recovering Construct (RC) and/or its gene product.

30. The complex of DNA constructs according to claim 29, **characterized in that** the Blocking Construct (BC) comprises a nucleotide sequence which expresses an enzyme in an organ-specific, developmental or spatiotemporal manner, an antisense mRNA of an enzyme essential for the survival or sexual reproduction of the host organism.

31. The complex of DNA constructs according to claim 30, **characterized in that** the Blocking Construct (BC) comprises a synthetic nucleotide sequence adapted to host organism preference and/or to intron preference of the Transgene of Interest (TGI) encoding the desired product in a host organism.

32. The complex of DNA constructs according to claim 31, **characterized in that** the synthetic nucleotide sequence encodes the enzyme *barnase,* having the SEQ ID NO :1:, and has a nucleotide sequence adapted to plant preference and/or adapted to an intron of a plant gene said sequence being SEQ ID NO :3.

33. The complex of DNA constructs according to claim 15, **characterized in that** the Recovering Construct (RC) comprises a repressor peptide/protein binding the operon sequence, which controls the expression of the blocking nucleotide sequence.

34. The complex of DNA constructs according to claim 33, **characterized in that** the repressor peptide/protein is a Tn10 *tet* repressor protein and the operon sequence is a *tet-*operator DNA sequence.

35. The complex of DNA constructs according to claim 15, **characterized in that** the Recovering Construct (RC) comprises a nucleotide sequence expressing an antisense RNA of the blocking nucleotide sequence and being capable of activating a silencing mechanism of the Blocking Construct (BC) expression compensating the deficiency resulted by the action of the Blocking Construct (BC).

36. The complex of DNA constructs according to claim 15, **characterized in that** the Recovering Construct (RC) comprises a nucleotide sequence expressing a protein inactivating the protein expressed by Blocking Construct (BC).

37. The complex of DNA constructs according to claim 36, **characterized in that** the nucleotide sequence of the Recovering Construct (RC) expresses Barstar protein inactivating the Barnase nuclease by binding to it.

38. The complex of DNA constructs according to claim 15, **characterized in that** the Blocking Construct (BC) comprises a synthetic nucleotide sequence which adapted to be inserted into the intron of a Transgene of Interest (TGI) by providing intron nucleotide sequence preference.

39. The complex of DNA constructs according to claim 15, **characterized in that** the synthetic nucleotide sequence which is adapted for insertion is a nucleotide sequence substantially identical to the *barnase* gene sequence and having the SEQ ID NO :3:.

40. The complex of DNA constructs according to claim 38, **characterized in that** the synthetic sequences of *barnase* and *barstar* genes which are adapted for plant expression comprise SEQ ID NO: 1 and SEQ ID NO :2, respectively.

41. The complex of DNA constructs according to claim 15, **characterized in** having a synthetic polycloning site SEQ ID NO:4.

## Patentansprüche

1. Verfahren zur Bereitstellung eines erhöhten Sicherheits-Niveaus zum Kontrollieren von Transgen-Segregation in einer sich sexuell fortpflanzenden Pflanze und/oder zur Bereitstellung von automatischer Verhinderung des Transgen-Auskommens, **dadurch gekennzeichnet, dass** das Verfahren einen molekularen Mechanismus umfasst, der die Schritte beinhaltet
(a) das Konstruieren eines oder mehrerer Wiedererlangbare-Blockierung-von-Funktion(WBF)-Systeme oder -Komplexe von DNA-Konstrukten zum Einführen in eine Empfänger-Zelle oder -Zelllinie, zusätzlich zu einem Transgenen Insert (TI), das ein oder mehrere Transgene von Interesse (TGIs), die ein oder mehrere erwünschte Genprodukte codieren, ein oder mehrere Blockier-Konstrukte (BK) umfasst, und das Bereitstellen von mindestens einem Anwender-gesteuerten Mittel zum Wiedererlangen der blockierten Funktionen;
(i) wobei das Blockier-Konstrukt (BK) die Kapazität besitzt, mindestens eine molekulare oder physiologische Funktion, die für das Überleben oder die Fortpflanzung der sich sexuell fortpflanzenden Pflanze essentiell ist, zu blockieren, wobei das Blockier-Konstrukt (BK) im gleichen Chromosom wie und in enger Nachbarschaft zu dem Transgenen Insert (TI), das ein oder mehrere Transgene von Interesse (TGls) umfasst, lokalisiert ist, vorzugsweise in einem Intron eines Transgens von Interesse (TGI), oder jede Seite eines Transgenen Inserts (TI), das ein oder mehrere Transgene von Interesse (TGls) umfasst, flankiert;
(ii) wobei das Mittel zum Wiedererlangen der blockierten Funktion ein oder mehrere Anwender-gesteuerte Eingriffe umfasst, gegebenenfalls kombiniert mit einem oder mehreren Wiedererlangungs-Konstrukten (WK), die nahe zu einen Blockier-Konstrukt (BK) in einem Transgenen Insert (TI), das ein oder mehrere Transgene von Interesse (TGls) umfasst, und/oder in nicht-allelen Chromosomen angeordnet sind;
und
(b) das Wiedererlangen der blockierten Funktion, um eine normale Entwicklung während der Kultivierung und der Fortpflanzung der sich sexuell fortpflanzenden Pflanze für Produktionszwecke zu ermöglichen durch das Anwenden einer oder mehrerer Anwender-gesteuerter Eingriffe in einem empfänglichen Stadium des Entwicklungs- oder Reproduktions-Zyklus der sich sexuell vermehrenden Pflanze;
mit der Maßgabe, dass, wenn nur ein Blockier-Konstrukt verwendet wird, das Blockier-Konstrukt (BK) oder ein Teil davon in einem Intron des einzigen Transgens von Interesse (TGI) angeordnet ist.

2. Verfahren gemäß Anspruch 1, **dadurch gekennzeichnet, dass** die Blockier-Konstrukte (BKs) jede Seite eines Transgenen Inserts (TI), das ein oder mehrere Transgene von Interesse (TGls) umfasst, flankieren, wenn zwei oder mehrere Blockier-Konstrukte (BKs) verwendet werden.

3. Verfahren gemäß Anspruch 1, **dadurch gekennzeichnet, dass** das Wiedererlangbare-Blockierung-von-Funktion(WBF)-System ein Doppel-Wiedererlangbare-Blockierung-von-Funktion(D-WBF)-System ist, das mindestens zwei Blockier-Konstrukte (BKs), die gleich oder unterschiedlich sind und die durch die gleichen oder unterschiedlichen Wiedererlangungs-Mechanismen wiedererlangbar sind, umfasst und die jede Seite des Transgenen Inserts (TI), das ein oder mehrere Transgene von Interesse (TGIs) umfasst, flankieren.

4. Verfahren gemäß Anspruch 1, **dadurch gekennzeichnet, dass** das Wiedererlangbare-Blockierung-von-Funktion(WBF)-System ein Multiple-, vorzugsweise ein Triple-Wiedererlangbare-Blockierung-von-Funktion(M-WBF, T-WBF)-System ist, das zwei, vorzugsweise drei oder mehre Transgene Inserts (TIs) umfasst, die in drei oder mehreren nicht-allelen Chromosomen angeordnet sind, in denen das erste Transgene Insert (TI) mindestens zwei unterschiedliche Blockier-Konstrukte (BKs) umfasst, das zweite Transgene Insert (TI) ein oder mehrere erste Wiedererlangungs-Konstrukte (WKs) umfasst, die an ein fakultatives Blockier-Konstrukt (BK), das gleich oder unterschiedlich zu einem der Blockier-Konstrukte (BKs) des ersten Transgenen Inserts (TI) ist, verknüpft sind, und ein drittes Transgenes Insert (TI), das ein oder mehrere zweite Wiedererlangungs-Konstrukte (WKs) umfasst, die unterschiedlich zu den ersten Wiedererlangungs-Konstrukten (WKs) sind und die mit einem Blockier-Konstrukt (BK) entsprechend eine der ersten Transgenen Inserts (TI), das nicht das gleiche wie das fakultative Blockier-Konstrukt (BK) des zweiten Transgenen Inserts (TI) ist, verknüpft sind.

5. Verfahren gemäß Anspruch 1, **dadurch gekennzeichnet, dass** zum Erhalten eines Multiple-Wiedererlangbare-Blockierung-von-Funktion(M-WBF)-Systems die empfangende transgene, sich sexuell fortpfanzende Pflanze bereitgestellt wird mit zwei Transgenen Inserts (TIs) eines Reversen-Segregierende-Wiedererlangbare-Blockierung-von-Funktion-(WS-WBF)-Systems in zwei nicht-allelen Chromosomen ohne das Transgen von Interesse (TGI) und einem Transformationsvektor, der zwei Blockier-Konstrukte (BKs) umfasst, die auf jeder Seite einer Clonierungsstelle für gewünschte Transgene von Interesse (TGIs) angeordnet sind, und impliziert, dass der Vektor die empfangende transgene, sich sexuell fortpflanzende Pflanzenlinie in ein drittes nicht-alleles Chromosom transformiert.

6. Verfahren gemäß Anspruch 1, **dadurch gekennzeichnet, dass** das Wiedererlangbare-Blockierung-von-Funktion-(WBF)-System ein Segregierende-Wiedererlangbare-Blockierung-von-Funktion-(S-WBF)-System ist, das ein Wiedererlangungs-Konstrukt (WK) und ein Blockier-Konstrukt (BK) umfasst, die in unterschiedlichen nicht-allelen Chromosomen mit dem Blockier-Konstrukt (BK) in einem Intron eines Transgenen Inserts (TI), das ein oder mehrere Transgene von Interesse (TGIs) umfasst, angeordnet sind.

7. Verfahren gemäß Anspruch 1, **dadurch gekennzeichnet, dass** ein Induzierte-Wiedererlangbare-Blockierung-von-Funktion-(I-WBF)-System ein Wiedererlangungs-Konstrukt (WK) umfasst, das in dem gleichen Chromosom wie das Blockier-Konstrukt (BK) angeordnet ist, aber unter einem unterschiedlichen Promotor exprimiert wird, wobei das Blockier-Konstrukt (BK) oder ein Teil davon in einem Intron eines Transgenen Inserts (TI), das ein oder mehrere Transgene von Interesse (TGls) umfasst, angeordnet ist.

8. Verfahren gemäß Anspruch 1, **dadurch gekennzeichnet, dass** die Expression von mindestens einer Nucleotidsequenz in dem Blockier-Konstrukt (BK) eine molekulare oder physiologische Funktion, die essentiell für das Überleben oder die Fortpflanzung einer transgenen, sexuellen Pflanze ist, auf eine konstitutive, Organ-spezifische oder räumlich-zeitliche Art und Weise blockiert.

9. Verfahren gemäß Anspruch 1, **dadurch gekennzeichnet, dass** die Beseitigung des Effekts des Blockier-Konstrukts (BK) durch das Anwenden eines Anwender-gesteuerten Eingriffs, der in einem empfänglichen Stadium im Entwicklungs- und/oder Reproduktions-Zyklus der transgenen, sich sexuell vermehrenden Pflanze die konstitutive, organspezifische oder räumlich-zeitlich erlangte morphologische oder physiologische Blockierung emporhebt oder aufgibt und dabei die Hybridisierung und/oder sexuelle Reproduktion der transgenen, sich sexuell fortpflanzenden Pflanze verhindert.

10. Verfahren gemäß Anspruch 9, **dadurch gekennzeichnet, dass** das empfängliche Stadium, das für das Überleben und/oder die Fortpflanzung essentiell ist, ausgewählt ist aus der Gruppe, bestehend aus frühe oder späte Samenkeimung, Blüte, Ausbildung eines Zwerg-Phänotyps, Unfähigkeit infloreszente Blüten und/oder Früchte auszubilden, Unfähigkeit der Keimung, Wachstum, Wurzelbildung und Photosynthese.

11. Verfahren gemäß Anspruch 9, **dadurch gekennzeichnet, dass** das empfängliche Stadium, das für das Überleben und/oder die Reproduktion essentiell ist, ausgewählt ist aus der Gruppe bestehend aus früher oder später Samenkeimung.

12. Verfahren gemäß Anspruch 1, **dadurch gekennzeichnet, dass** die Beseitigung des Effektes der blockierten Funktion einen Anwender-gesteuerten extern- oder intern-regulierbaren molekularen Mechanismus umfasst, der gegebenenfalls unter eingeschränkten Bedingungen anwendbar ist.

13. Verfahren gemäß Anspruch 12, **dadurch gekennzeichnet, dass** der Anwender-gesteuerte, extern- und/oder intern-regulierbare molekulare Mechanismus zur Beseitigung des blockierten Effekts ausgewählt ist aus der Gruppe bestehend aus
(a) dem Hinzufügen von mindestens einer extern anwendbaren Substanz;
(b) dem Anwenden mindestens eines externen chemischen oder physikalischen Stimulus, der befähigt ist, das fakultative Wiedererlangungs-Konstrukt (WK) zu aktivieren;
(c) dem Hemmen des Promoters des Blockier-Konstrukts (BK);
(d) dem Eindämmen der mRNA-Expression der Nucleotidsequenz des Blockier-Konstrukts (BK) durch Antisense-RNA-Technologie;
(e) dem Erlauben der Expression eines Proteins, das befähigt ist, das Protein zu inaktivieren, das durch die Nucleotidsequenz des Blockier-Konstrukts (BK) codiert wird, durch Anwenden eines extern-aktivierbaren molekularen Mechanismus;
(f) dem Erlauben der Expression eines Enzyms, das eine Substanz produziert, die befähigt ist, das Protein, das durch die Nucleotidsequenz des Blockier-Konstrukts (BK) exprimiert wird, zu inaktivieren;
(g) dem Erlauben der Expression eines Enzyms, das befähigt ist, eine toxische Substanz, die durch das Enzym produziert wird, zu inaktivieren;
(h) dem Erlauben der Expression eines Enzyms, das befähigt ist, eine Substanz zu produzieren, die den Mangel, der durch die Aktivität des Blockier-Konstrukts (BK) verursacht wird, kompensiert;
(i) dem Erlauben der Expression eines Enzyms, das befähigt ist, eine Substanz zu produzieren, die die Aktivität, die durch das Blockier-Konstrukt (BK) verursacht wird, verändert;
(j) dem Erlauben von spezifischer Nucleinsäure- und/oder Proteinbindung;
(k) dem Anwenden von Proteolyse;
(l) dem Erlauben von Protein-Protein-Interaktion;
(m) dem Exprimieren eines Enzyms, das befähigt ist, eine toxische Substanz eins zu entgiften;
(n) dem Exprimieren einer Substanz, die befähigt ist, die toxische, über-produzierte Substanz zu kompensieren oder zu inaktivieren;
(o) dem Exprimieren einer Substanz, die befähigt ist, mit einer toxischen Substanz zu interagieren;
(p) dem Bereitstellen eines Enzyms, das befähigt ist eine toxische oder überproduzierte Substanz mit falscher räumlich-zeitlicher Spezifität umzuwandeln;
(q) dem externen Kompensieren des Mangels, der durch das Blockier-Konstrukt (BK) verursacht wird;
(r) dem externen Induzieren einer/eines reagierenden Wiedererlangungs-DNA, -RNA, -Proteins oder -Metabolits;
(s) dem konstitutiven Exprimieren der Nucleotidsequenz des Wiedererlangungs-Konstrukts (WK);
(t) dem räumlich-zeitlichen Exprimieren der Nucleotidsequenz des Wiedererlangungs-Konstrukts (WK); und
(u) dem Erlauben von Intra-Linie-Kreuzung ("intraline crossing"), wenn das Wiedererlangbare-Blockierung-von-Funktion-(WBF)-System ein Segregierende-Doppel- oder ein Multiple-Wiedererlangbare-Blockierung-von-Funktion-(WBF)-System ist.

14. Verfahren gemäß Anspruch 1, **dadurch gekennzeichnet, dass** der Anwender-gesteuerte, extern-regulierbare molekulare Mechanismus zur Wiedererlangung der blockierten Funktion das Erlauben von Intra-Linie-Kreuzung, die homozygote Zustände unterstützt, umfasst.

15. Komplex von DNA-Konstrukten oder einem Wiedererlangbare-Blockierung-von-Funktion-(WBF)-System zum Bereitstellen eines erhöhten Sicherheits-Niveaus in der Kontrolle von Transgen-Segregation in einer sich sexuell fortpflanzenden Pflanze und/oder zur Bereitstellung einer automatischen Verhinderung von Introgression, **dadurch gekennzeichnet, dass** der Komplex von DNA-Konstrukten umfasst
(a) ein Transgenes Insert (Tl), das mindestens ein Transgen von Interesse (TGI) umfasst, das ein oder mehrere erwünschte Genprodukte codiert;
(b) ein oder mehrere Blockier-Konstrukte (BK), die durch Anwender-gesteuerte Mittel zum Wiedererlangen wiedererlangbar sind, wobei das Blockier-Konstrukt (BK) mindestens eine Nucleotidsequenz, die die Fähigkeit zur Blockierung mindestens einer molekularen oder physiologischen Funktion, die für das Überleben oder die Fortpflanzung der sich sexuell fortpflanzenden Pflanze essentiell ist, beinhaltet und im gleichen Chromosom wie und in enger Nachbarschaft zu das/dem Transgene(n) Insert (TI), das ein oder mehrere Transgene von Interesse (TGIs) umfasst, vorzugsweise in einem Intron eines Transgens von Interesse (TGI), lokalisiert ist oder jede Seite eines Transgenen Inserts (TI), das ein oder mehrere Transgene von Interesse (TGIs) umfasst, flankiert;
(c) ein oder mehrere fakultative Wiedererlangungs-Konstrukte (WK), die nahe zu einem der Blockier-Konstrukte (BKs) in einem Transgenen Insert (TI) angeordnet sind, das ein oder mehrere Transgene von Interesse (TGIs), die in einem oder mehreren nicht-allelen Chromosomen angeordnet sind, umfasst;
mit der Maßgabe, dass wenn nur ein Blockier-Konstrukt (BK) verwendet wird, dass das Blockier-Konstrukt (BK) oder ein Teil davon in einem Intron des einzigen Transgens von Interesse (TGI) angeordnet ist.

16. Der Komplex von DNA-Konstrukten gemäß Anspruch 15, **dadurch gekennzeichnet, dass**, die Blockier-Konstrukte (BKs) jede Seite des Transgenen Inserts (TI), das ein oder mehrere Transgene von Interesse (TGls) umfasst, flankieren, und, wenn zwei oder mehrere Blockier-Konstrukte (BKs) verwendet werden, eine selbst-gesteuerte und selbst-unterstützende Einheit bilden.

17. Komplex von DNA-Konstrukten gemäß Anspruch 15, **dadurch gekennzeichnet, dass** das Wiedererlangbare-Blockierung-von-Funktion-(WBF)-System ein Doppel-Wiedererlangbare-Blockierung-von-Funktion-(D-WBF)-System ist, das Blockier-Konstrukte (BKs) umfasst, die mindestens zwei und gleich oder unterschiedliche sind und zu beiden Seiten eines Transgenen Inserts (TI), das ein oder mehrere Transgene von Interesse (TGIs) umfasst, angeordnet sind und die durch den gleichen oder unterschiedlichen Wiedererlangungs-Mechanismus wiedererlangbar sind.

18. Komplex vom DNA-Konstrukten gemäß Anspruch 15, **dadurch gekennzeichnet, dass** das Wiedererlangbare-Blockierung-von-Funktion(WBF)-System ein Multiple-Wiedererlangbare-Blockierung-vom-Funktion(M-WBF)-System ist, das zwei, vorzugsweise drei oder mehr Transgene Inserts (TI) umfasst, die in drei oder mehreren nicht-allelen Chromosomen angeordnet sind, in denen das erste Transgene Insert (TI) mindestens zwei unterschiedliche Blockier-Konstrukte (BKs) umfasst, das zweite Transgene Insert (TI), ein oder mehrere erste Wiedererlangungs-Konstrukte (WKs) umfasst, die mit einem fakultativen Blockier-Konstrukt (BK) entsprechend einem der Blockier-Konstrukte (BKs) des ersten Transgenen Inserts (TI) verknüpft sind, und ein drittes Transgenes Insert (Tl), das ein oder mehrere zweite Wiedererlangungs-Konstrukte (WKs) umfasst, die gleich oder unterschiedlich zu den ersten Wiedererlangungs-Konstrukten (WKs) sind, und die mit einem Blockier-Konstrukt (BK) verknüpft sind, das das gleiche wie eines des ersten Transgenen Inserts (TI) ist, das nicht das gleiche wie das fakulatative Blockier-Konstrukt (BK) des zweiten Transgenen Inserts (TI) ist.

19. Komplex von DNA-Konstrukten gemäß Anspruch 15, **dadurch gekennzeichnet, dass** ein Multiple-Wiederlangbare-Blockierung-von-Funktion(M-WBF)-System des empfangenden transgenen, sich sexuell fortpflanzenden multizellulären Organismus (SFMO) mit zwei Transgenen Inserts (Tls) eines Revers-Segregierende-Wiederlangbare-Blockierung-von-Funktion-(US-WBF)-Systems in zwei nicht-allelen Chromosomen ohne das Transgen von Interesse (TGI) und einem Transformationsvector, der zwei Blockier-Konstrukte (BKs) umfasst, die auf jeder Seite einer Clonierungsstelle für gewünschte Transgene von Interesse (TGls) angeordnet sind, bereit gestellt wird, und impliziert, dass der Vektor die empfangende, transgene, sich sexuell fortpflanzende Pflanzen-Linie in ein drittes nicht-alleles Chromosom transformiert.

20. Komplex von DNA-Konstrukten gemäß Anspruch 15, **dadurch gekennzeichnet, dass** das Wiederlangbare-Blockierung-von-Funktion(WBF)-System ein Induzierte-Wiedererlangbare-Blockierung-von-Funktion(I-WBF)-System ist, das ein Wiedererlangungs-Konstrukt (WK) umfasst, das im gleichen Chromosom wie das Blockier-Konstrukt (BK) mit dem Blockier-Konstrukt (BK) in einem Intron des Transgenen Inserts (TI), das ein oder mehrere Transgene von Interesse (TGI) umfasst, angeordnet ist.

21. Komplex von DNA-Konstrukten gemäß Anspruch 15, **dadurch gekennzeichnet, dass** das Wiedererlangbare-Blockierung-von-Funktion(WBF)-System ein Segregierende-Wiedererlangbare-Blockierung-von-Funktion(S-WBF)-System ist, das ein Wiedererlangungs-Konstrukt (WK) und ein Blockier-Konstrukt (BK) umfasst, die in unterschiedlichen nicht-allelen Chromosomen mit dem Blockier-Konstrukt (BK) oder einem Teil davon, das in einem Intron des Transgenen Inserts (TI), das ein oder mehrere Transgene von Interesse (TGIs) umfasst, angeordnet sind.

22. Komplex von DNA-Konstrukten gemäß dem Anspruch 15, **dadurch gekennzeichnet, dass** ein induzierbares Wiedertangungs-Konstrukt (WK) aktivierbar ist in Reaktion auf einen Anwender-gesteuerten Eingriff, der die Funktion, die essentiell für das Überleben und die sexuelle Fortpflanzung des transgenen, sich sexuell fortpflanzenden multizellulären Organismus (SFMO) ist, wiedererlangt und dessen Funktion durch ein Blockier-Konstrukt (BK), das unter demselben oder einem unterschiedlichen Promoter-Typ exprimiert wird, blockiert ist.

23. Komplex von DNA-Konstrukten gemäß Anspruch 15, **dadurch gekennzeichnet, dass** die zwei Nucleotidsequenzen des Blockier-Konstrukts (BK), das ein Doppel-Wiedererlangbare-Blockierung-von-Funktion(D-WBF)-System ausbildet, durch die gleichen oder unterschiedlichen Mittel zur Wiedererlangung wiedererlangbar sind.

24. Komplex von DNA-Konstrukten gemäß Anspruch 15, **dadurch gekennzeichnet, dass** ein induzierbares Wiedererlangbares-Konstrukt (WK) aktivierbar ist in Reaktion auf einen Anwender-gesteuerten Eingriff, der die Funktion wiedererlangt, die durch das Blockier-Konstrukt (BK), das unter dem gleichen oder einem unterschiedlichen Promoter exprimiert ist, blockiert ist.

25. Komplex von DNA-Konstrukten gemäß Anspruch 15, **dadurch gekennzeichnet, dass** das Blockier-Konstrukt (BK) mindestens eine Nucleotidsequenz umfasst, deren konstitutive, organisch-spezifische oder räumlich-zeitliche Expression eine essentielle molekulare oder physiologische Funktion auf DNA-, mRNA, Protein- oder Metabolit-Ebene blockiert, und/oder eine phänotypische, physiologische oder morphologische Veränderung verursacht, die freie Hybridisierung und/oder Fortpflanzung einer sich auskreuzenden, transgenen, sich sexuell fortpflanzenden Pflanze unter natürlichen, nicht-gesteuerten Bedingungen, die zu deren Auslöschung führen, verhindert.

26. Komplex von DNA-Konstrukten gemäß Anspruch 15, **dadurch gekennzeichnet, dass** die Funktion, die durch das Blockier-Konstrukt (BK) des Wiederlangbare-Blockierung-von-Funktion(WBF)-Systems blockiert ist, durch Anwendung eines Anwender-gesteuerten extern- oder intern-regulierbaren molekularen Mechanismus, der wahlweise unter eingeschränkten Bedingungen anwendbar ist, wiedererlangt wird.

27. Komplex von DNA-Konstrukten gemäß Anspruch 27, **dadurch gekennzeichnet, dass** der Anwender-gesteuerte Eingriff zum Wiedererlangen der blockierten Funktion ausgewählt ist aus der Gruppe bestehend aus:
(a) dem Hinzufügen von mindestens einer extern anwendbaren Substanz;
(b) dem Anwenden mindestens eines externen chemischen oder physikalischen Stimulus, der befähigt ist, das fakultative Wiedererlangungs-Konstrukt (WK) zu aktivieren;
(c) dem Hemmen des Promoters des Blockier-Konstrukts (BK);
(d) dem Eindämmen der Expression der mRNA des blockierenden Gens durch Antisense-RNA-Technik;
(e) dem Erlauben der Expression eines Proteins, das befähigt ist, das Blockier-Konstrukt (BK)-codierte Protein durch einen extern-aktivierbaren molekularen Mechanismus zu aktivieren;
(f) dem Erlauben der Expression eines Enzymes, das befähigt ist, eine Substanz zu produzieren, die das Protein, das durch das Blockier-Konstrukt (BK) exprimiert wird, inaktiviert;
(g) dem Erlauben der Expression eines Enzyms, das befähigt ist, eine toxische Substanz, die durch das Enzym produziert wird, zu inaktivieren;
(h) dem Erlauben der Expression eines Enzyms, das befähigt ist, eine Substanz zu produzieren, die den Mangel, der durch die Aktivität des Blockier-Konstrukts (BK) verursacht wurde, kompensiert;
(i) dem Erlauben der Expression eines Enzyms, dass befähigt ist, eine Substanz zu produzieren, die die Änderung, die durch die Aktivität des Blockier-Konstrukts (BK) verursacht wurde, kompensiert.
(j) dem Erlauben von spezifischer Nucleinsäure- und/oder Proteinbindung;
(k) dem Anwenden von Proteolyse;
(l) dem Erlauben von Protein-Protein-Interaktion;
(m) dem Exprimieren eines Enzyms, das befähigt ist, eine toxische Substanz eins zu entgiften;
(n) dem Exprimieren einer Substanz, die befähigt ist, die toxische, über-produzierte Substanz zu kompensieren oder zu inaktivieren;
(o) dem Exprimieren einer Substanz, die befähigt ist, mit einer toxischen Substanz zu interagieren;
(p) dem Bereitstellen eines Enzyms, das eine toxische oder über-produzierte Substanz produziert;
(q) dem externen Kompensieren des Mangels, der durch das Blockier-Konstrukt (BK) verursacht wird;
(r) dem externen Induzieren einer/eines reagierenden Wiedererlangungs-DNA, -RNA, -Proteins oder -Metabolits;
(s) dem konstitutiven Exprimieren der Nucleotidsequenz des Wiedererlangungs-Konstrukts (WK);
(t) dem räumlich-zeitlichen Exprimieren der Nucleotidsequenz des Wiedererlangungs-Konstrukts (WK); und
(u) dem Erlauben von Intra-Linie-Kreuzung, wenn das Wiedererlangbare-Blockierung-von-Funktion-(WBF)-System ein Segregierende-Doppel- oder ein Multiple-Wiedererlangbare-Blockierung-von-Funktion(WBF)-System ist.

28. Komplex von DNA-Konstrukten gemäß Anspruch 15, **dadurch gekennzeichnet, dass** der Anwender-gesteuerte, extern-regulierbare molekulare Mechanismus zur Wiedererlangung der blockierten Funktion das Erlauben von Intra-Linie-Kreuzung umfasst.

29. Komplex von DNA-Konstrukten gemäß Anspruch 15, **dadurch gekennzeichnet, dass** das Blockier-Konstrukt (BK) eine Nucleotidsequenz umfasst, die in dem Wirts-Organismus auf eine konstitutive, organ-spezifische oder räumlich-zeitliche Art und Weise exprimiert wird und das Wiederlangungs-Konstrukt (WK) eine Nucleotidsequenz umfasst, die befähigt ist, durch einen Anwender-gesteuerten Eingriff, der eine auf Behandlung reagierende Nucleotidsequenz oder deren Genprodukt reguliert, oder durch konstitutive Expression der Nucleotidsequenz in dem Wiedererlangungs-Konstrukt (WK) und/oder deren Genprodukt wiedererlangt zu werden.

30. Komplex von DNA-Konstrukten gemäß Anspruch 29, **dadurch gekennzeichnet, dass** das Blockier-Konstrukt (BK) eine Nucleotidsequenz umfasst, die ein Enzym auf eine organ-spezifische, entwicklungsgemäße oder räumlich-zeitliche Art und Weise exprimiert, eine Antisense-mRNA eines Enzyms, das für das Überleben oder die sexuelle Fortpflanzung des Wirts-Organismus essentiell ist.

31. Komplex von DNA-Konstrukten gemäß Anspruch 30, **dadurch gekennzeichnet, dass** das Blockier-Konstrukt (BK) eine synthetische Nucleotidsequenz umfasst, die angepasst ist an die Wirts-Organismus-Präferenz und/oder die Intron-Präferenz des Transgens von Interesse (Tl), das das gewünschte Produkt in einem Wirts-Organismus codiert.

32. Komplex von DNA-Konstrukten gemäß Anspruch 31, **dadurch gekennzeichnet, dass** die synthetische Nucleotidsequenz das Enyzm *Barnase* codiert, das die SEQ ID NO: 1 hat und die Nucleotidsequenz hat, die an Pflanzen-Präferenz angepasst ist und/oder die an ein Intron eines Pflanzengens angepasst ist, wobei die Sequenz SEQ ID NO: 3 ist.

33. Komplex von DNA-Konstrukten gemäß Anspruch 15, **dadurch gekennzeichnet, dass** das Wiedererlangungs-Konstrukt (WK) ein Repressor-Peptid/-Protein umfasst, das an die Operon-Sequenz, die die Expression der blockierenden Nucleotidsequenz steuert, bindet.

34. Komplex von DNA-Konstrukten gemäß Anspruch 33, **dadurch gekennzeichnet, dass** das Repressor-Peptid/-Protein ein Tn10 tet-Repressor-Protein ist und dass die Operon-Sequenz eine tet-Operator-DNA-Sequenz ist.

35. Komplex von DNA-Konstrukten gemäß Anspruch 15, **dadurch gekennzeichnet, dass** das Wiedererlangungs-Konstrukt (WK) eine Nucleotidsequenz umfasst, die eine Antisense-RNA der blockierenden Nucleotidsequenz exprimiert und die befähigt ist, einen Eindämmungs-Mechanismus der Blockier-Konstrukt (BK)-Expression zu aktivieren, was den Mangel, der sich aus der Aktivität des Blockier-Konstrukts (BK) ergibt, kompensiert.

36. Komplex von DNA-Konstrukten gemäß Anspruch 15, **dadurch gekennzeichnet, dass** das Wiedererlangungs-Konstrukt (WK) eine Nucleotidsequenz umfasst, die ein Protein exprimiert, das das Protein, das durch das Blockier-Konstrukt (BK) exprimiert wird, inaktiviert.

37. Komplex von DNA-Konstrukten gemäß Anspruch 36, **dadurch gekennzeichnet, dass** die Nucleotidsequenz des Wiederlangungs-Konstrukts (WK) Barstar-Protein exprimiert, das die Barnase-Nuclease inaktiviert, indem es daran bindet.

38. Komplex von DNA-Konstrukten gemäß Anspruch 15, **dadurch gekennzeichnet, dass** das Blockier-Konstrukt (BK) eine synthetische Nucleotidsequenz umfasst, die durch Bereitstellen von Intron-Nucleotidsequenz-Präferenz angepasst ist, um in das Intron eines Transgens von Interesse (TI) eingefügt zu werden.

39. Komplex von DNA-Konstrukten gemäß Anspruch 15, **dadurch gekennzeichnet, dass** die synthethische Nucleotidsequenz, die zur Einfügung angepasst ist, eine Nucleotidsequenz ist, die im wesentlichen zu der *Barnase*-Gensequenz identisch ist und die SEQ ID NO: 3 hat.

40. Komplex von DNA-Konstrukten gemäß Anspruch 38, **dadurch gekennzeichnet, dass** die synthetischen Sequenzen der *Barnase*- und *Barstar*-Gene, die für Pflanzen-Expression angepasst sind, SEQ ID NO: 1 bzw. SEQ ID NO: 2 umfassen.

41. Komplex von DNA-Konstrukten gemäß Anspruch 15, **dadurch gekennzeichnet, dass** er eine synthetische Polyclonierungsstelle SEQ ID NO: 4 aufweist.

## Revendications

1. Procédé pour obtenir un niveau de sécurité augmenté pour contrôler la ségrégation de transgène dans une plante se reproduisant sexuellement et/ou pour obtenir la prévention automatique de l'échappement de transgène, **caractérisé en ce que** le procédé comprend un mécanisme moléculaire comprenant les étapes de
(a) construction d'un ou plusieurs systèmes de bloc de fonction récupérable (RBF) ou de complexes de constructions d'ADN pour insertion dans une cellule ou lignée de cellules receveuse en plus d'un insert transgénique (TI) comprenant un ou plusieurs transgènes cibles (TGI) codant pour un ou plusieurs produits génétiques souhaités, une ou plusieurs constructions bloquantes (BC), et fourniture d'au moins un moyen contrôlé par l'utilisateur pour récupérer les fonctions bloquées;
(i) la dite construction bloquante (BC) ayant la capacité de bloquer au moins une fonction moléculaire ou physiologique essentielle pour la survie ou la reproduction de la plante se reproduisant sexuellement, ladite construction bloquante (BC) étant située dans le même chromosome que, et à proximité étroite de l'insert transgénique (TI) comprenant un ou plusieurs transgènes cibles (TGI) de préférence dans un intron d'un transgène cible (TGI) ou flanquant chaque côté d'un insert transgénique (TI) inclus;
(ii) lesdits moyens pour récupérer la fonction bloquée comprenant une ou plusieurs interventions contrôlées par l'utilisateur facultativement combinées avec une ou plusieurs constructions de récupération (RC) qui sont placées à proximité d'une construction bloquante (BC) dans un insert transgénique (TI) comprenant un ou plusieurs transgènes cibles (TGI) et/ou dans des chromosomes non alléliques; et
(b) récupération de la fonction bloquée afin de permettre un développement normal durant la culture et la reproduction de ladite plante se reproduisant sexuellement à des fins de production par application, à un stade sensible dans le cycle de développement ou de reproduction de ladite plante se reproduisant sexuellement, d'une ou plusieurs interventions contrôlées par l'utilisateur; à condition que lorsque seulement une construction bloquante est utilisée, ladite construction bloquante (BC) ou une partie de celle -ci soit placée dans un intron de l'unique transgène cible (TGI).

2. Procédé selon la revendication 1, **caractérisé en ce que** les constructions bloquantes (BC) flanquent chaque côté d'un insert transgénique (TI) comprenant un ou plusieurs transgènes cibles (TGI), lorsque deux constructions bloquantes (BC) ou plus sont utilisées.

3. Procédé selon la revendication 1, **caractérisé en ce que** le système de bloc de fonction récupérable (RBF) est un système à bloc de fonction récupérable double (D-RBF) comprenant au moins deux constructions bloquantes (BC), qui sont identiques ou différentes et qui sont récupérables par des mécanismes de récupération identiques ou différents, flanquant chaque côté de l'insert transgénique (TI) comprenant un ou plusieurs transgènes cibles (TGI).

4. Procédé selon la revendication 1, **caractérisé en ce que** le système de bloc de fonction récupérable (RBF) est un système à bloc de fonction récupérable multiple, de préférence triple (M-RBF, T -RBF) comprenant deux, de préférence trois inserts transgéniques (TI) ou plus placés dans trois chromosomes n on alléliques ou plus, dans lequel le premier insert transgénique (TI) comprend au moins deux constructions bloquantes (BC) différentes, le deuxième insert transgénique (TI) comprend une ou plusieurs premières constructions de récupération (RC) liées à une construction bloquante (BC) facultative étant identique à ou différente de l'une des constructions bloquantes (BC) du premier insert transgénique (TI) et un troisième insert transgénique (TI) comprenant une ou plusieurs deuxièmes constructions de récupération (RC) qui sont différentes des premières constructions de récupération (RC) et qui sont liées à une construction bloquante (BC) correspondant à l'une d u premier insert transgénique (TI) qui n'est pas identique à la construction bloquante facultative (BC) du deuxième insert transgénique (TI).

5. Procédé selon la revendication 1, **caractérisé en ce que** pour obtenir un système de bloc de fonction récupérable multiple (M -RBF) la plante se reproduisant sexuellement transgénique receveuse est pourvue de deux inserts transgéniques (TI) d'un système de bloc de fonction récupération à ségrégation inverse (RS-RBF) dans deux chromosomes non alléliques sans le transgène cible (TGI) et un vecteur de transformation comprenant deux constructions bloquantes (BC) placées sur chaque côté d'un site de clonage pour des transgènes cibles (TGI) souhaités et conduisant ledit vecteur à transformer la lignée de plante se reproduisant sexuellement transgénique receveuse dans un troisième chromosome non allélique.

6. Procédé selon la revendication 1, **caractérisé en ce que** le système de bloc de fonction récupérable (RBF) est un système de bloc de fonction récupérable à ségrégation (S-RBF) comprenant une construction de récupération (RC) et une construction bloquante (BC) placées dans des chromosomes non alléliques différents, avec la construction bloquante (BC) dans un intron d'un insert transgénique (TI) comprenant un ou plusieurs transgènes cibles (TGI).

7. Procédé selon la revendication 1, **caractérisé en ce qu'**un système de bloc de fonction récupérable induit (I-RBF) comprend une construction de récupération (RC) placée dans le même chromosome que la construction bloquante (BC) mais exprimée sous l'effet d'un promoteur différent, la construction bloquante (BC) ou une partie de cel le-ci étant placée dans un intron d'un insert transgénique (TI) comprenant un ou plusieurs transgènes cibles (TGI).

8. Procédé selon la revendication 1, **caractérisé en ce que** l'expression d'au moins une séquence de nucléotides dans la construction bloquan te (BC) bloque d'une manière constitutive, spécifique d'un organe ou spatiotemporelle une fonction moléculaire ou physiologique essentielle pour la survie ou la reproduction d'une plante sexuellement transgénique.

9. Procédé selon la revendication 1, **caractérisé en ce que** l'élimination de l'effet de la construction bloquante (BC) est effectuée en appliquant une intervention contrôlée par l'utilisateur, qui à un stade sensible dans le cycle de développement et/ou de reproduction de la plante se reproduisant sexuellement transgénique désorganise ou vide le bloc morphologique ou physiologique obtenu de manière spécifique d'un organe ou spatiotemporelle en prévenant l'hybridation et/ou la reproduction sexuelle de la plante se reproduisant sexuellement transgénique.

10. Procédé selon la revendication 9, **caractérisé en ce que** le stade sensible essentiel pour la survie et/ou la reproduction est choisi dans le groupe constitué de la germination de graine précoce ou tardive, la floraison, la formation de phénotype nain, l'incapacité de formation de fleurs à inflorescence et/ou de fruits, l'incapacité de germination, de végétation, d'enracinement et de photosynthèse.

11. Procédé selon la revendication 9, **caractérisé en ce que** le stade sensible essentiel pour la survie et/ou la reproduction est choisi dans le groupe constitué de la germination de graine précoce ou tardive.

12. Procédé selon la revendication 1, **caractérisé en ce que** l'élimination de l'effet de la fonction bloquée comprend un mécanisme moléculaire régulable de manière externe ou interne contrôlé par l'utilisateur facultativement applicable dans des conditions confinées.

13. Procédé selon la revendication 12, **caractérisé en ce que** le mécanisme moléculaire régulable de manière externe et/ou interne contrôlé par l'utilisateur pour l'élimination de l'effet bloqué est choisi dans le groupe constitué de
(a) l'ajout d'au moins une substance applicable de manière externe;
(b) l'application d'au moins un stimulus chimique ou physique externe capable d'activer la construction de récupération (RC) facultative;
(c) la répression du promoteur de la construction bloquante (BC) ;
(d) l'extinction de l'expression d'ARNm de la séquence de nucléotides de la construction bloquante (BC) par la technologie d'ARN antisens;
(e) l'autorisation de l'expression d'une protéine capable d'inactiver la protéine, qui est codée par la séquence de nucléotides de la construction bloquante (BC) par application d'un mécanisme moléculaire activable de manière externe;
(f) l'autorisation de l'expression d'une enzyme produisant une substance capable d'inactiver la protéine exprimée par une séquence de nucléotides de la construction bloquante (BC) ;
(g) l'autorisation de l'expression d'une enzyme capable d'inactiver une substance toxique produite par l'enzyme;
(h) l'autorisation de l'expression d'une enzyme capable de produire une substance compensant la déficience causée par l'action de la construction bloquante (BC);
(i) l'autorisation de l'expression d'une enzyme capable de produire une substance altérant l'action causée par la construction bloquante (BC);
(j) l'autorisation d'une liaison d'un acide nucléique et/ou d'une protéine spécifique;
(k) l'application d'une protéolyse;
(l) l'autorisation d'une interaction protéine-protéine;
(m) l'expression d'une enzyme capable de détoxifier une substance toxique;
(n) l'expression d'une substance capable de compenser ou d'inactiver la substance toxique surproduite;
(o) l'expression d'une substance capable d'interagir avec une substance toxique;
(p) la fourniture d'une enzyme capable de convertir une substance toxique ou surproduite avec une spécificité spatiotemporelle erronée;
(q) la compensation externe de la déficience causée par la construction bloquante (BC);
(r) l'induction externe d'un ADN, ARN, protéine ou métabolite de récupération réactif;
(s) l'expression constitutive de la séquence de nucléotides de la construction de récupération (RC);
(t) l'expression spatiotemporelle de la séquence de nucléotides de la construction de récupération (RC); et
(u) l'autorisation d'un croisement intralignée lorsque le système de bloc de fonction récupérable (RBF) est un système de bloc de fonction récupérable (RBF) double ou multiple à ségrégation.

14. Procédé selon la revendication 1, **caractérisé en ce que** le mécanisme moléculaire régulable de manière externe contrôlé par l'utilisateur pour récupérer la fonction bloquée comprend l'autorisation d'un croisement intralignée favorisant des conditions d'homozygotie.

15. Complexe de constructions d'ADN ou d'un système de bloc de fonction récupérable (RBF) pour obtenir un niveau de sécurité augmenté dans le contrôle de la ségrégation de transgène dans une plante se reproduisant sexuellement et/ou pour obtenir la prévention automatique de l'introgression **caractérisé en ce que** ledit complexe de constructions d'ADN comprend
(a) un insert transgénique (TI) comprenant au moins un transgène cible (TGI) codant pour un ou plusieurs produits génétiques souhaités;
(b) une ou plusieurs constructions bloquantes (BC), qui sont récupérables par des moyens contrôlés par l'utilisateur pour récupération, ladite construction bloquante (BC) comprenant au moins une séquence de nucléotides ayant la capacité de bloquer au moins une fonction moléculaire ou physiologique essentielle pour la survie ou la reproduction de la plante se reproduisant sexuellement et étant localisée dans le même chromosome que et à proximité étroite de l'insert transgénique (TI) comprenant un ou plusieurs transgènes cibles (TGI) de préférence dans un intron d'un transgène cible (TGI) ou flanquant chaque côté d'un insert transgénique (TI) comprenant un ou plusieurs transgènes cibles (TGI);
(c) une ou plusieurs constructions de récupération (RC) facultatives qui sont placées à proximité de l'une des constructions bloquant es (BC) dans un insert transgénique (TI) comprenant un ou plusieurs transgènes cibles (TGI) placé dans un ou plusieurs chromosomes non alléliques; à condition que lorsque seulement une construction bloquante (BC) est utilisée, ladite construction bloquante (BC) ou une partie de celle -ci soit placée dans un intron de l'unique transgène cible (TGI).

16. Complexe de constructions d'ADN selon la revendication 15, **caractérisé en ce que** les constructions bloquantes (BC) flanquent chaque côté de l'insert transgénique (TI) comprenant un ou plusieurs transgènes cibles (TGI), lorsque deux constructions bloquantes (BC) ou plus sont utilisées de manière à former une entité autocontrôlée et autosoutenue.

17. Complexe de constructions d'ADN selon la revendication 15, **caractérisé en ce que** le système de bloc de fonction récupérable (RBF) est un système de bloc de fonction récupérable double (D-RBF) comprenant des constructions bloquantes (BC), qui sont au moins deux et identiques ou différentes et situées sur les deux côtés d'un insert transgénique (TI) comprenant un ou plusieurs transgènes cibles (TGI) et récupérables par des mécanismes de récupération identiques ou différents.

18. Complexe de constructions d'ADN selon la revendication 15, **caractérisé en ce que** le système de bloc de fonction récupérable (RBF) est un système de bloc de fonction récupérable multiple (M-RBF) comprenant deux, de préférence trois inserts transgéniques (TI) ou plus placés dans trois chromosomes non alléliques ou plus, dans lequel le premier insert transgénique (TI) comprend au moins deux constructions bloquantes (BC) différentes, le deuxième insert transgénique (TI) comprend une ou plusieurs premières constructions de récupération (RC) liées à une construction bloquante (BC) facultative correspondant à l'une des constructions bloquantes (BC) du premier insert transgénique (TI) et le troisième insert transgénique (TI) comprenant une ou plusieurs deuxièmes constructions de récupération (RC) qui sont identiques ou différentes des premières constructions de récupération (RC) et qui sont liées à une construction bloquante (BC) étant identique à l'une du premier insert transgénique (TI) qui n'est pas le même que la construction bloquante (BC) facultative du deuxième insert transgénique (TI).

19. Complexe de constructions d'ADN selon la revendication 15, **caractérisé en ce que** pour obtenir un système de bloc de fonction récupérable multiple (M-RBF) l'organisme pluricellulaire se reproduisant sexuellement (SRMO) transgénique receveur est pourvu de deux inserts transgéniques (TI) d'un système de bloc de fonction récupérable à ségrégation inverse (RS -RBF) dans deux chromosomes non alléliques sans le transgène cible (TGI) et un vecteur de transformation comprenant deux constructions bloquantes (BC) placées sur chaque côté d'un site de clonage pour des transgènes cibles souhaités (TGI) et conduisant ledit vecteur à transformer la lignée de plante se reproduisant sexuellement transgénique receveuse dans un troisième chromosome non allélique.

20. Complexe de cons tructions d'ADN selon là revendication 15, **caractérisé en ce que** le système de bloc de fonction récupérable (RBF) est un système de bloc de fonction récupérable induit (I-RBF) comprenant une construction de récupération (RC) placée dans le même chromosome que la construction bloquante (BC), la construction bloquante (BC) ou une partie de celle -ci étant placée dans un intron d'un insert transgénique (TI) comprenant un ou plusieurs transgènes cibles (TGI) .

21. Complexe de constructions d'ADN selon la revendi cation 15, **caractérisé en ce que** le système de bloc de fonction récupérable (RBF) est un système de bloc de fonction récupérable à ségrégation (S-RBF) comprenant une construction de récupération (RC) et une construction bloquante (BC) placées dans des chromosomes non alléliques différents, la construction bloquante (BC) ou une partie de celle-ci étant placée dans un intron de l'insert transgénique (TI) comprenant un ou plusieurs transgènes cibles (TGI).

22. Complexe de constructions d'ADN selon la revendication 15, **caractérisé en ce qu'**une construction de récupération (RC) inductible est activable en réponse à une intervention contrôlée par l'utilisateur, qui récupère la fonction essentielle pour la survie et la reproduction sexuelle d'un organisme pluricellulaire se reproduisant sexuellement (SRMO) transgénique et cette fonction est bloquée par une construction bloquante (BC) exprimée sous le contrôle d'un type identique ou différent de promoteur.

23. Complexe de constructions d'ADN selon la revendication 15, **caractérisé en ce que** les deux séquences de nucléotides de la construction bloquante (BC) formant un système de bloc de fonction récupérable double (D-RBF) sont récupérables par des moyens de récupération identiques ou différents.

24. Complexe de cons tructions d'ADN selon la revendication 15, **caractérisé en ce qu'**une construction de récupération (RC) inductible est activable en réponse à une intervention contrôlée par l'utilisateur, qui récupère la fonction bloquée par la construction bloquante (BC) exprimée sous le contrôle d'un promoteur identique ou différent.

25. Complexe de constructions d'ADN selon la revendication 15, **caractérisé en ce que** la construction bloquante (BC) comprend au moins une séquence de nucléotides, dont l'expression constitutive, spécifique d'un organe ou spatiotemporelle bloque une fonction moléculaire ou physiologique essentielle au niveau d'un ADN, un ARNm, une protéine ou un métabolite et/ou cause une altération phénotypique, physiologique ou morphologique empêchant l'hybridation et/ou la reproduction libre d'une plante se reproduisant sexuellement croisée dans des conditions naturelles non contrôlées conduisant à son extinction.

26. Complexe de constructions d'ADN selon la revendication 15, **caractérisé en ce que** la fonction bloquée par la construction bloquante (BC) du système de bloc de fonction récupérable (RBF) est récupérée par application d'un mécanisme moléculaire régulable de manière externe ou interne contrôlé par l'utilisateur facultativement applicable dans des conditions confinées.

27. Complexe de constructions d'ADN selon la revendication 26, **caractérisé en ce que** l'intervention contrôlée par l'utilisateur pour récupérer la fonction bloquée est choisie dans le groupe constitué du
(a) l'ajout d'au moins une substance applicable de manière externe;
(b) l'application d'au moins un stimulus chimique ou physique externe capable d'activer la construction de récupération (RC) facultative;
(c) la répression du promoteur de la construction bloquante (BC) ;
(d) l'extinction de l'expression d'ARNm de gène bloquant par la technique d'ARN antisens;
(e) l'autorisation de l'expression d'une protéine capable d'inactiver la construction bloquante (BC) codant pour une protéine par des mécanismes moléculaires activables de manière externe;
(f) l'autorisation de l'expression d'une enzyme capable de produire une substance inactivant la protéine exprimée par la construction bloquante (BC);
(g) l'autorisation de l'expression d'une enzyme capable d'inactiver une substance toxique produite par l'enzyme;
(h) l'autorisation de l'expression d'une enzyme capable de produire une substance compensant la déficience causée par l'action de la construction bloquante (BC);
(i) l'autorisation de l'expression d'une enzyme capable de produire une substance compensant l'altération causée par l'action de la construction bloquante (BC);
(j) l'autorisation d'une liaison d'un acide nucléique et/ou d'une protéine spécifique;
(k) l'application d'une protéolyse;
(l) l'autorisation d'une interaction protéine-protéine;
(m) l'expression d'une enzyme capable de détoxifier une substance toxique;
(n) l'expression d'une substance capable de compenser ou d'inactiver la substance surproduite toxique;
(o) l'expression d'une substance capable d'interagir avec une substance toxique;
(p) la fourniture d'une enzyme produisant une substance toxique ou surproduite;
(q) la compensation externe de la déficience causée par la construction bloquante (BC);
(r) l'induction externe d'un ADN, ARN, protéine ou métabolite de récupération réactif;
(s) l'expression constitutive de la séquence de nucléotides de la construction de récupération (RC);
(t) l'expression spatiotemporelle de la séquence de nucléotides de la construction de récupération (RC); et
(u) l'autorisation d'un croisement intrali gnée lorsque le système de bloc de fonction récupérable (RBF) est un système de bloc de fonction récupérable (RBF) multiple.

28. Complexe de constructions d'ADN selon la revendication 15, **caractérisé en ce que** le mécanisme moléculaire régulable de manière externe contrôlé par l'utilisateur pour récupérer la fonction bloquée comprend un croisement intralignée.

29. Complexe de constructions d'ADN selon la revendication 15, **caractérisé en ce que** la construction bloquante (BC) comprend une séquence de nucléotides exprimée dans l'organisme hôte d'une manière constitutive, spécifique d'un organe ou spatiotemporelle et la construction de récupération (RC) comprend une séquence de nucléotides pouvant être récupérée par une intervention contrôlée par l'utilisateur régulant une séquence de nucléotides réactive à un traitement ou son produit génétique ou par expression constitutive de la séquence de nucléotides dans la construction de récupération (RC) et/ou son produit génétique.

30. Complexe de constructions d'ADN selon la revendication 29, **caractérisé en ce que** la construction bloquante (BC) comprend une séquence de nucléotides qui exprime une enzyme d'une manière spécifique d'un organe, liée au développement ou spatiotemporelle, un ARNm antisens d'une enzyme essentielle pour la survie ou la reproduction sexuelle de l'organisme hôte.

31. Complexe de constructions d'ADN selon la revendication 30, **caractérisé en ce que** la construction bloquante (BC) comprend une séquence de nucléotides synthétique adaptée pour une préférence pour l'organisme hôte et/ou une préférence pour un intron du transgène cible (TGI) codant pour le produit souhaité dans un organisme hôte.

32. Complexe de constructions d'ADN selon la revendication 31, **caractérisé en ce que** la séquence de nucléotides synthétique code pour l'enzyme *barnase*, ayant SEQ ID n° 1, et a une séquence de nucléotides adaptée pour une préférence pour une plante et/ou adaptée pour un intron d'un gène de plante, ladite séquence étant SEQ ID n° 3.

33. Complexe de constructions d'ADN selon la revendication 15, **caractérisé en ce que** la construction de récupération (RC) comprend un peptide/protéine répresseur se liant à la séquence d'opéron qui contrôle l'expression de la séquence de nucléotides bloquante.

34. Complexe de constructions d'ADN selon la revendication 33, **caractérisé en ce que** le peptide/protéine répresseur est une protéine de répresseur Tn10 *tet* et la séquence d'opéron est une séquence d'ADN d'opérateur *tet*.

35. Complexe de constructions d'ADN selon la revendication 15, **caractérisé en ce que** la construction de récupération (RC) comprend une séquence de nucléotides exprimant un ARN antisens de la séquence de nucléotides bloquante et étant capable d'activer un mécanisme d'extinction de l'expression de la construction bloquante (BC) compensant la déficience résultant de l'action de la construction bloquante (BC).

36. Complexe de constructions d'ADN selon la revendication 15, **caractérisé en ce que** la construction de récupération (RC) comprend une séquence de nucléotides exprimant une protéine inactivant la protéine exprimée par la construction bloquante (BC).

37. Complexe de constructions d'ADN selon la revendication 36, **caractérisé en ce que** la séquence de nucléotides de la construction de récupération (RC) exprime la protéine Barstar inactivant la nucléase barnase en se liant à celle-ci.

38. Complexe de constructions d'ADN selon la revendication 15, **caractérisé en ce que** la construction bloquante (BC) comprend une séquence de nucléotides synthétique qui est adaptée pour être insérée dans l'intron d'un transgène cible (TGI) en présentant une préférence pour une séquence de nucléotides d'intron.

39. Complexe de constructions d'ADN selon la revendication 15, **caractérisé en ce que** la séquence de nucléotides synthétique qui est adaptée pour insertion est une séquence de nucléotides sensiblement identique à la séquence du gène *barnase* et ayant SEQ ID n° 3.

40. Complexe de constructions d'ADN selon la revendication 38, **caractérisé en ce que** les séquences synthétiques des gènes *barnase* et *barstar* qui sont adaptées pour l'expression dans une plante comprennent SEQ ID n° 1 et SEQ ID n° 2, respectivement.

41. Complexe de constructions d'ADN selon la revendication 15, **caractérisé en ce qu'**il comprend un site de polyclonage synthétique SEQ ID n° 4.


### SEQUENCE LISTING

<110> Unicrop Ltd
<120> Molecular Control of Transgene Segregation and Escape by Recoverable Block of Function
<130> A0420PC
<140>
   <141>
<150> US 09/617,543
   <151> 2000-07-14
<160> 15
<170> PatentIn Ver. 2.1
<210> 1
   <211> 357
   <212> DNA
   <213> Artificial Sequence
<220>
   <223> Description of Artificial Sequence: -
<220>
   <223> Description: Bacillus amyloliquefaciens
<220>
   <223> Plant adapted coding sequence of barnase gene
<220>
   <223> Fragment type: Full coding sequence
<400> 1
   cgcggatcca tggcacaagt tatcaacacc tttgatggag ttgctgacta ccttcagacc 60
   taccataagc ttccagataa ctacatcacc aagtctgagg ctcaggctct tggatgggtt 120
   gcttctaagg gaaaccttgc tgatgtcgct ccaggaaagt ctatcggagg tgatatcttc 180
   tctaacaggg agggaaagct tccaggaaag tctggaagga cctggaggga ggctgatatc 240
   aactacacct ctggattcag gaactctgat aggatccttt actcttccga ctggcttatc 300
   tacaagacca ctgaccacta ccagaccttc accaagatcc ggtgagagct cgagcgc    357
<210> 2
   <211> 299
   <212> DNA
   <213> Artificial Sequence
<220>
   <223> Description of Artificial Sequence: -
<220>
   <223> Description: Bacillus amyloliquefaciens
<220>
   <223> Plant adapted synthetic coding sequence of barstar gene
<220>
   <223> Fragment type: Full coding sequence
<400> 2
   cgcggatcct gatcatgaag aaggctgtta tcaacggtga gcaaattagg tctatctctg 60
   atcttcacca gacccttaag aaggagcttg ctcttccaga gtactacgga gagaaccttg 120
   atgctctatg ggattgcctt accggatggg tggagtaccc acttgttttg gagtggaggc 180
   agtttgagca gtctaagcag cttactgaga atggagctga gagtgttctt caggttttcc 240
   gggaggctaa ggctgaggga tgcgatatca ccatcattct ttcttgagag ctcgagcgc  299
<210> 3
   <211> 856
   <212> DNA
   <213> Artificial Sequence
<220>
   <223> Description of Artificial Sequence: -
<220>
   <223> Description: Vigna mungo (SH-EP promoter), Bacillus amyloliquefaciens (barnase gene), Escherichia coli (uidA gene)
<220>
   <221> promoter
   <222> (1)..(352)
   <223> Modified SH-EP promoter
<220>
   <221> misc_feature
   <222> (353)..(404)
   <223> 5' end exon of modified uidA gene
<220>
   <221> misc_feature
   <222> (443)..(778)
   <223> Synthetic coding sequence of barnase gene
<220>
   <221> intron
   <222> (405)..(830)
   <223> Intron of uidA gene
<220>
   <221> misc_feature
   <222> (831)..(856)
   <223> Exon of modified uidA gene
<400> 3
   cagcatgcaa gagaaagatg attcttgaag catacgataa cagattgaat gtgacaaaaa 60
   gtttgtgtct cagcttcagg gtcggcacct aatacaaaag gaaaatttgt caggtttcct 120
   tccgtagttt cattcactat tattgaatcc tttggctacc attcttgaga aacacaaaca 180
   cttcttatat ctgttctaca caattctctg agtgcgtgcc acagtttggt atcttcatga 240
   ttgctcattg ttcatgccca taaggaacat gtaacttcct catttattta ttattgcttt 300
   tgttttcttc tcactagttt acaaacgttt ccctatataa accctccttt gttcactgct 360
   ttcctccctg ttgtggcttc tctccgaagt tcatcccggt ccacctgcaa aataagtaat 420
   aagataaagt aaaaaagtta gtatggctca agttattaat acttttgatg gagttgctga 480
   ttatcttcaa acttatcata aacttccaga taattatatt actaaatctg aagctcaagc 540
   tcttggatgg gttgcttcta aaggaaatct tgctgatgtt gctccaggaa aatctattgg 600
   aggagatatt ttttcaaata gagaaggaaa acttccagga aaatctggaa gaacatggag 660
   agaagctgat attaattata cttctggatt tagaaattca gatagaatcc tttattcatc 720
   tgattggctt atttataaaa ctacagatca ttatcaaact tttacaaaaa ttagataaat 780
   atttgtgttt tttgtatgtt gtgatcatta ataaataaat aaatacatac ctcttctgca 840
   gcaggaaggc agccga                                                 856
<210> 4
   <211> 249
   <212> DNA
   <213> Artificial Sequence
<220>
   <223> Description of Artificial Sequence: -
<220>
   <223> Description: Synthetic polycloning site for plasmid DNA
<400> 4
   aagcttacta gtggtaccac cggtacgcgt gggcccgcat gctgatcaat gcatgtcgac 60
   catatggccg gcgctagcca attggacgtc cttaagatcg atccgcggct gcaggcgcgc 120
   ggatcccgta cgccatggcg gccgggcgcc gagctcgtgc actctagacc cgggggcgcg 180
   ccctcgagtg tacatcatga gcggccgcag atctggccgg cctttaatta atccggacct 240
   agggaattc 249
<210> 5
   <211> 8
   <212> RNA
   <213> Artificial Sequence
<220>
   <223> Description of Artificial Sequence: -
<220>
   <223> 5' exon/intron boundary site
<400> 5
   agguaugu 8
<210> 6
   <211> 7
   <212> RNA
   <213> Artificial Sequence
<220>
   <223> Description of Artificial Sequence: -
<220>
   <223> Branchpoint sequence, located 20-50 nt upstream from boundary site
<400> 6
   uacuaac 7
<210> 7
   <211> 5
   <212> RNA
   <213> Artificial Sequence
<220>
   <223> Description of Artificial Sequence:
<220>
   <223> 3' intron/exon boundary site
<400> 7
   gcagg 5
<210> 8
   <211> 6
   <212> RNA
   <213> Artificial Sequence
<220>
   <223> Description of Artificial Sequence: -
<220>
   <223> Near upstream element (NUE) of polyadenylation site
<400> 8
   aauaaa 6
<210> 9
   <211> 6
   <212> RNA
   <213> Artificial Sequence
<220>
   <223> Description of Artificial Sequence: -
<220>
   <223> Far upstream element (FUE) of polyadenylation site
<400> 9
   uuugua 6
<210> 10
   <211> 10
   <212> RNA
   <213> Artificial Sequence
<220>
   <223> Description of Artificial Sequence: -
<220>
   <223> Upstream FUE element
<400> 10
   uguguuuuuu 10
<210> 11
   <211> 7
   <212> RNA
   <213> Artificial Sequence
<220>
   <223> Description of Artificial Sequence: -
<220>
   <223> Upstream FUE element
<400> 11
   uguugug 7
<210> 12
   <211> 13
   <212> DNA
   <213> Artificial Sequence
<220>
   <223> Description of Artificial Sequence: -
<220>
   <223> Promoter region located 15-60 nucleotides from the start of the transcription with TATA in consensus sequence
<400> 12
   tcactatata tag 13
<210> 13
   <211> 4
   <212> DNA
   <213> Artificial Sequence
<220>
   <223> Description of Artificial Sequence: -
<220>
   <223> Box located 30-100 nucleotides upstream from the TATA box
<400> 13
   caat 4
<210> 14
   <211> 4
   <212> DNA
   <213> Artificial Sequence
<220>
   <223> Description of Artificial Sequence: -
<220>
   <223> Box located 30-100 nucelotides upstream from the TATA box
<400> 14
   agga 4
<210> 15
   <211> 10
   <212> DNA
   <213> Artificial Sequence
<220>
   <223> Description of Artificial Sequence: -
<220>
   <223> Sequence 40-80 nucleotides downstream from the AUG codon starting
<400> 15
   aacaatggct 10

### SEQUENCE LISTING

<110> Unicrop Ltd
<120> Molecular Control of Transgene Segregation and Its Escape by a Recoverable Block of Function (RBF) System
<130> A0420PC-
<140>
   <141>
<150> US 09/617,543
   <151> 2000-07-14
<150> PCT/FI01/00670
   <151> 2001-07-16
<160> 15
<170> PatentIn Ver. 2.1
<210> 1
   <211> 357
   <212> DNA
   <213> Artificial Sequence
<220>
   <223> Description of Artificial Sequence: -
<220>
   <223> Description: Bacillus amyloliquefaciens
<220>
   <223> Plant adapted coding sequence of barnase gene
<220>
   <223> Fragment type: Full coding sequence
<400> 1
   cgcggatcca tggcacaagt tatcaacacc tttgatggag ttgctgacta ccttcagacc 60
   taccataagc ttccagataa ctacatcacc aagtctgagg ctcaggctct tggatgggtt 120
   gcttctaagg gaaaccttgc tgatgtcgct ccaggaaagt ctatcggagg tgatatcttc 180
   tctaacaggg agggaaagct tccaggaaag tctggaagga cctggaggga ggctgatatc 240
   aactacacct ctggattcag gaactctgat aggatccttt actcttccga ctggcttatc 300
   tacaagacca ctgaccacta ccagaccttc accaagatcc ggtgagagct cgagcgc    357
<210> 2
   <211> 299
   <212> DNA
   <213> Artificial Sequence
<220>
   <223> Description of Artificial Sequence: -
<220>
   <223> Description: Bacillus amyloliquefaciens
<220>
   <223> Plant adapted synthetic coding sequence of barstar gene
<220>
   <223> Fragment type: Full coding sequence
<400> 2
   cgcggatcct gatcatgaag aaggctgtta tcaacggtga gcaaattagg tctatctctg 60
   atcttcacca gacccttaag aaggagcttg ctcttccaga gtactacgga gagaaccttg 120
   atgctctatg ggattgcctt accggatggg tggagtaccc acttgttttg gagtggaggc 180
   agtttgagca gtctaagcag cttactgaga atggagctga gagtgttctt caggttttcc 240
   gggaggctaa ggctgaggga tgcgatatca ccatcattct ttcttgagag ctcgagcgc  299
<210> 3
   <211> 856
   <212> DNA
   <213> Artificial Sequence
<220>
   <223> Description of Artificial Sequence: -
<220>
   <223> Description: Vigna mungo (SH-EP promoter), Bacillus amyloliquefaciens (barnase gene), Escherichia coli (uidA gene)
<220>
   <221> promoter
   <222> (1)..(352)
   <223> Modified SH-EP promoter
<220>
   <221> misc_feature
   <222> (353)..(404)
   <223> 5' end exon of modified uidA gene
<220>
   <221> misc_feature
   <222> (443)..(778)
   <223> Synthetic coding sequence of barnase gene
<220>
   <221> intron
   <222> (405)..(830)
   <223> Intron of uidA gene
<220>
   <221> misc_feature
   <222> (831)..(856)
   <223> Exon of modified uidA gene
<400> 3
   cagcatgcaa gagaaagatg attcttgaag catacgataa cagattgaat gtgacaaaaa 60
   gtttgtgtct cagcttcagg gtcggcacct aatacaaaag gaaaatttgt caggtttcct 120
   tccgtagttt cattcactat tattgaatcc tttggctacc attcttgaga aacacaaaca 180
   cttcttatat ctgttctaca caattctctg agtgcgtgcc acagtttggt atcttcatga 240
   ttgctcattg ttcatgccca taaggaacat gtaacttcct catttattta ttattgcttt 300
   tgttttcttc tcactagttt acaaacgttt ccctatataa accctccttt gttcactgct 360
   ttcctccctg ttgtggcttc tctccgaagt tcatcccggt ccacctgcaa aataagtaat 420
   aagataaagt aaaaaagtta gtatggctca agttattaat acttttgatg gagttgctga 480
   ttatcttcaa acttatcata aacttccaga taattatatt actaaatctg aagctcaagc 540
   tcttggatgg gttgcttcta aaggaaatct tgctgatgtt gctccaggaa aatctattgg 600
   aggagatatt ttttcaaata gagaaggaaa acttccagga aaatctggaa gaacatggag 660
   agaagctgat attaattata cttctggatt tagaaattca gatagaatcc tttattcatc 720
   tgattggctt atttataaaa ctacagatca ttatcaaact tttacaaaaa ttagataaat 780
   atttgtgttt tttgtatgtt gtgatcatta ataaataaat aaatacatac ctcttctgca 840
   gcaggaaggc agccga                                                 856
<210> 4
   <211> 249
   <212> DNA
   <213> Artificial Sequence
<220>
   <223> Description of Artificial Sequence: -
<220>
   <223> Description: Synthetic polycloning site for plasmid DNA
<400> 4
   aagcttacta gtggtaccac cggtacgcgt gggcccgcat gctgatcaat gcatgtcgac 60
<210> 5
   <211> 8
   <212> RNA
   <213> Artificial Sequence
<220>
   <223> Description of Artificial Sequence: -
<220>
   <223> 5' exon/intron boundary site
<400> 5
   agguaugu 8
<210> 6
   <211> 7
   <212> RNA
   <213> Artificial Sequence
<220>
   <223> Description of Artificial Sequence: -
<220>
   <223> Branchpoint sequence, located 20-50 nt upstream from boundary site
<400> 6
   uacuaac 7
<210> 7
   <211> 5
   <212> RNA
   <213> Artificial Sequence
<220>
   <223> Description of Artificial Sequence: -
<220>
   <223> 3' intron/exon boundary site
<400> 7
   gcagg 5
<210> 8
   <211> 6
   <212> RNA
   <213> Artificial Sequence
<220>
   <223> Description of Artificial Sequence: -
<220>
   <223> Near upstream element (NUE) of polyadenylation site
<400> 8
   aauaaa 6
<210> 9
   <211> 6
   <212> RNA
   <213> Artificial Sequence
<220>
   <223> Description of Artificial Sequence: -
<220>
   <223> Far upstream element (FUE) of polyadenylation site
<400> 9
   uuugua 6
<210> 10
   <211> 10
   <212> RNA
   <213> Artificial Sequence
<220>
   <223> Description of Artificial Sequence: -
<220>
   <223> Upstream FUE element
<400> 10
   uguguuuuuu 10
<210> 11
   <211> 7
   <212> RNA
   <213> Artificial Sequence
<220>
   <223> Description of Artificial Sequence: -
<220>
   <223> Upstream FUE element
<400> 11
   uguugug 7
<210> 12
   <211> 13
   <212> DNA
   <213> Artificial Sequence
<220>
   <223> Description of Artificial Sequence: -
<220>
   <223> Promoter region located 15-60 nucleotides from the start of the transcription with TATA in consensus sequence
<400> 12
   tcactatata tag 13
<210> 13
   <211> 4
   <212> DNA
   <213> Artificial Sequence
<220>
   <223> Description of Artificial Sequence: -
<220>
   <223> Box located 30-100 nucleotides upstream from the TATA box
<400> 13
   caat 4
<210> 14
   <211> 4
   <212> DNA
   <213> Artificial Sequence
<220>
   <223> Description of Artificial Sequence: -
<220>
   <223> Box located 30-100 nucelotides upstream from the TATA box
<400> 14
   agga 4
<210> 15
   <211> 10
   <212> DNA
   <213> Artificial Sequence
<220>
   <223> Description of Artificial Sequence: -
<220>
   <223> Sequence 40-80 nucleotides downstream from the AUG codon starting
<400> 15
   aacaatggct 10